# EUROPEAN PATENT APPLICATION

(11) **EP 4 840 958 A1**
(43) Date of publication of application: **18.11.2026**
(21) Application number: 26178673.5
(22) Date of filing: 13.05.2026
(51) Int. Cl.: C12N 15/113

(54) **SIRNA DUPLEX FOR TARGETED REGULATION OF LPA GENE EXPRESSION AND USE THEREOF IN PREVENTING AND TREATING CARDIOVASCULAR AND CEREBROVASCULAR DISEASES**

(30) Priority: 14.05.2025 CN 202510616122; 27.04.2026 CN 202610569478
(71) Applicant: Beijing Youcare Kechuang Pharmaceutical Technology Co., Ltd., Daxing District, Beijing 100176 (CN)
(72) Inventor: YANG, Shuo, Beijing, 100176 (CN); HUANG, Zeao, Beijing, 100176 (CN); SONG, Gengshen, Beijing, 100176 (CN); LIU, Wei, Beijing, 100176 (CN); YU, Fei, Beijing, 100176 (CN); WU, Yucheng, Beijing, 100176 (CN); FU, Rui, Beijing, 100176 (CN)
(74) Representative: Dehns

(57) **Abstract**

The present disclosure provides an siRNA duplex for targeted regulation of LPA gene expression and the use thereof in the prevention and treatment of cardiovascular and cerebrovascular diseases. The present disclosure also relates to corresponding conjugates and pharmaceutical compositions. Results from cell-based and animal experiments demonstrate that the oligonucleotide duplexes of the present disclosure can significantly inhibit LPA gene expression, and thus may be used for the manufacture of medicaments for preventing or treating atherosclerosis and other related diseases.

## Description

### SPECIFICATION

### CROSS-REFERENCE TO RELATED APPLICATION

The present application claims the priority of Chinese patent application 2025106161221, filed on May 14, 2025 and the priority of Chinese patent application 2026105694789, filed on April 27, 2026. The contents of the above patent applications are incorporated herein by reference in their entirety.

### TECHNICAL FIELD

The present disclosure belongs to the field of biomedicine, and specifically relates to an siRNA duplex for targeted regulation of LPA gene expression and the use thereof in the prevention and treatment of cardiovascular and cerebrovascular diseases.

### BACKGROUND

Nucleic acid drugs, particularly oligonucleotide drugs, have been widely used due to their simple synthesis and high biological activity. Oligonucleotide drugs typically include antisense oligonucleotides (ASOs), small interfering RNAs (siRNAs), microRNAs (miRNAs), and aptamers.

Oligonucleotides are short DNA or RNA molecules or oligomers that can bind to their complementary oligonucleotides, DNA, or RNA in a sequence-specific manner to form duplexes or, although less commonly, hybrids of a higher order under certain circumstances. Oligonucleotides can bind sequence-specifically to complementary RNA strands and, upon hybridization, can induce RNase H-mediated cleavage of the target RNA. In natural oligonucleotides, nucleotides are linked via phosphodiester bonds. Under physiological conditions, oligonucleotides are particularly sensitive to nucleases. Therefore, when preparing oligonucleotide drugs, natural, unstructured, and unmodified oligonucleotide are readily and rapidly degraded *in vivo,* resulting in very limited biological activity and consequently poor druggability. Modification of oligonucleotides is an effective approach to enhance their activity, which can improve their resistance to nucleases and their affinity for RNA, and better facilitate endocytosis and tissue targeting, thereby effectively regulating the expression of target genes.

The basic structure of a nucleotide can typically be divided into four components: a base, a ribose, a phosphate backbone, and terminal groups. Modifications of these four components may include, for example, the following:
1) Base modifications: These mainly include three types: purine modifications, pyrimidine modifications, and base substitutions. Purine modifications include N6-methyladenosine, N1-methyladenosine, and 7-methylguanosine modifications. Pyrimidine modifications include 3-methyluridine, 5-methyluridine, 5-methylcytidine, N4-acetylcytidine, pseudouridine, thiouridine, propynyluridine, and dihydrouridine modifications.
2) Ribose modifications: These mainly include modifications and substitutions of functional groups at specific positions on the ribose ring. Ribose modifications include, but are not limited to, 2'-modifications, 4'-modifications, 5'-modifications, and isomeric modifications. The most common 2'-modifications of ribose are 2'-OMe (2'-O-methyl) and 2'-F (2'-fluoro) modifications. Compared to natural oligonucleotides, oligonucleotides incorporating both 2'-OMe and 2'-F modifications exhibit higher Tm values, greater serum stability, and improved biological activity.
3) Phosphate backbone modifications: The primary modification patterns include, but are not limited to, phosphorothioate modifications; methylphosphonate, selenophosphate, boranophosphate, and dithiophosphate modifications; substitution of the bridging oxygen atom in the phosphodiester linkage with a sulfur atom; and complete substitution of the phosphodiester linkage between nucleosides with a non-phosphorus-containing group, such as substitution of the phosphorus atom with a carbon, sulfur, or nitrogen atom, thereby forming structures such as guanidinium linkages, S-methylthiourea linkages, or nitrate linkages.
4) Terminal modifications: These include, but are not limited to, covalent attachment of specific groups to the 5' end and/or 3' end of the sense strand, and phosphorylation modification at the 5' end of the antisense strand.

Lipoprotein(a) (Lp(a)) is a special type of lipoprotein particle discovered using immunological methods. It plays a role in the occurrence and development of cardiovascular diseases and is a significant risk factor for such diseases. Lp(a) consists of an LDL-like particle containing lipid components such as cholesterol and oxidized phospholipids (OxPL), together with apolipoprotein(a) (Apo(a)) and apolipoprotein B-100, wherein apolipoprotein B-100 is covalently linked to Apo(a). Apo(a) is a highly polymorphic glycoprotein encoded by the LPA gene.

Currently, Lp(a) is believed to promote the formation of atherosclerotic plaques through various mechanisms. Epidemiological and genetic studies have shown that elevated Lp(a) levels are an independent risk factor for cardiovascular diseases. Even when LDL-C levels are controlled within the optimal range specified by current guidelines, the cardiovascular risk associated with Lp(a) persists. Therefore, inhibition of LPA gene expression can reduce the risk of atherosclerosis and related cardiovascular events. Alternative and combination therapies for patients with lipoprotein(a)-related diseases are needed in clinical practice.

### BRIEF SUMMARY OF THE INVENTION

To address the technical problem in the prior art of lacking a more effective siRNA duplex for targeted regulation of LPA gene expression, the present disclosure provides an siRNA duplex for targeted regulation of LPA gene expression and the use thereof in the prevention and treatment of cardiovascular and cerebrovascular diseases. In the present disclosure, by modifying the siRNA core sequences, multiple siRNA derivatives that exhibit significant inhibitory effects on LPA gene expression are identified, thereby providing corresponding siRNA conjugates.

The technical solutions of the present disclosure include, but are not limited to, the following:
In one aspect, the present disclosure provides an siRNA duplex comprising an oligonucleotide duplex formed by pairing of a sense strand and an antisense strand.

In another aspect, the present disclosure provides a conjugate for reducing the expression of LPA, comprising the siRNA duplex described above and a conjugate moiety attached thereto.

In another aspect, the present disclosure provides a nucleic acid-protein complex comprising a duplex region of the siRNA duplex described above or an antisense strand of the duplex region, and a nuclease.

In another aspect, the present disclosure provides a recombinant vector comprising a nucleic acid molecule encoding the siRNA duplex described above.

In some embodiments, the vector backbone of the recombinant vector is selected from recombinant virus-derived circular RNA vectors, tRNA scaffolds, rRNA scaffolds, and chimeric tRNA/pre-miRNA vectors.

In another aspect, the present disclosure provides a recombinant cell that synthesizes and secretes the siRNA duplex described above.

In some embodiments, the recombinant cell is selected from *Rhodovulum sulfidophilum* and ribonuclease III-deficient *Corynebacterium glutamicum.*

In another aspect, the present disclosure provides a method of preparing an siRNA duplex, comprising culturing the recombinant cell described above or chemical synthesis.

In another aspect, the present disclosure provides a pharmaceutical composition comprising the siRNA duplex described above, the conjugate described above, or the nucleic acid-protein complex described above, and a pharmaceutically acceptable carrier.

In another aspect, the present disclosure provides a method of inhibiting LPA gene expression, comprising contacting the siRNA duplex described above, the conjugate described above, the nucleic acid-protein complex described above, or the pharmaceutical composition described above with a target cell.

In some embodiments, the method is for non-diagnostic or non-therapeutic purposes.

In some embodiments, the method is performed *in vivo* or *in vitro.*

In another aspect, the present disclosure provides the use of the siRNA duplex described above, the conjugate described above, the nucleic acid-protein complex described above, or the pharmaceutical composition described above in the manufacture of a medicament for treating a disease associated with LPA gene expression.

The disease associated with LPA gene expression is selected from diseases caused by overexpression of Apo(a) protein, pathogenic mutations in the LPA gene, abnormal metabolism of Apo(a) protein, and abnormal interactions between LPA or Apo(a) and another substance.

In some embodiments, the disease associated with LPA gene expression is selected from Berger's disease, peripheral arterial disease, coronary artery disease, metabolic syndrome, acute coronary syndrome, aortic stenosis, aortic regurgitation, aortic dissection, retinal artery occlusion, cerebrovascular disease, mesenteric ischemia, superior mesenteric artery occlusion, renal artery stenosis, stable or unstable angina, acute coronary syndrome, heterozygous or homozygous familial hypercholesterolemia, hyperapobetalipoproteinemia, cerebrovascular atherosclerosis, cerebrovascular disease and venous thrombosis, stroke, atherosclerosis, thrombosis, coronary heart disease or aortic stenosis, and/or any other disease associated with elevated levels of Lp(a) particles, as well as other unidentified related disorders, pathologies, or syndromes.

In another aspect, the present disclosure provides the siRNA duplex described above, the conjugate described above, the nucleic acid-protein complex described above, or the pharmaceutical composition described above for use in the treatment of a disease associated with LPA gene expression.

In another aspect, the present disclosure provides a method of treating a disease associated with LPA gene expression, comprising administering to a subject in need thereof an effective amount of the siRNA duplex described above, the conjugate described above, the nucleic acid-protein complex described above, or the pharmaceutical composition described above.

In another aspect, the present disclosure provides the use of the siRNA duplex described above, the conjugate described above, the nucleic acid-protein complex described above, or the pharmaceutical composition described above in the manufacture of a medicament for preventing or treating atherosclerosis and cardiovascular and cerebrovascular diseases.

In another aspect, the present disclosure provides the siRNA duplex described above, the conjugate described above, the nucleic acid-protein complex described above, or the pharmaceutical composition described above for use in the prevention or treatment of atherosclerosis and cardiovascular and cerebrovascular diseases.

In another aspect, the present disclosure provides a method of preventing or treating atherosclerosis and cardiovascular and cerebrovascular diseases, comprising administering to a subject in need thereof an effective amount of the siRNA duplex described above, the conjugate described above, the nucleic acid-protein complex described above, or the pharmaceutical composition described above.

The beneficial effects achieved by the present disclosure include, but are not limited to, the following:
(1) The siRNA core sequences, such as siRNA B01001, B01023, B01042, B01047, B02417, B06001, B01801, and B01012, all exhibit significant inhibitory effects on the LPA gene, with the highest inhibition rate exceeding 50%.
(2) The siRNA derivatives of the present disclosure can achieve an inhibition rate of up to 70% or more against the LPA gene. Furthermore, the derivatives are conjugated with a GalNAc conjugate moiety to form corresponding siRNA conjugates, which can be efficiently delivered to the liver of animals, significantly inhibiting LPA gene expression and significantly reducing serum Lp(a) levels.
(3) The sequences modified using alternating modifications and the modification templates of the present disclosure exhibit significantly enhanced inhibitory activity against LPA compared to siRNAs with minimal differences disclosed in the prior art, with an increase of up to 36.0%.

### BRIEF DESCRIPTION OF THE DRAWINGS

To more clearly illustrate the technical solutions of the embodiments of the present disclosure, the accompanying drawings used in the embodiments will be briefly described below. It is apparent that the drawings described below are solely for illustrative purposes of some examples of the present disclosure and are not intended to limit the present disclosure.

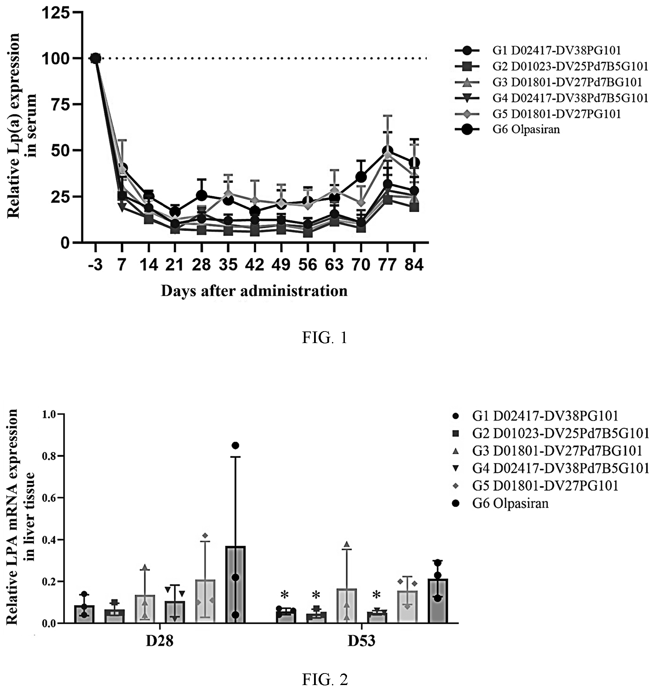

FIG. 1 shows the inhibitory effects of siRNA B01023, B02417, and B01801 on the expression levels of Lp(a) in the serum of cynomolgus monkeys after template modification and anti-off-target modification, followed by conjugation with a GalNAc conjugate moiety ((relative to pre-dose D-3) - 1 mg/kg).
FIG. 2 shows the inhibitory effects of siRNA B01023, B02417, and B01801 on the mRNA expression levels of the LPA gene in liver tissues of cynomolgus monkeys after template modification and anti-off-target modification, followed by conjugation with a GalNAc conjugate moiety ((relative to pre-dose D-9) - 1 mg/kg).

### DETAILED DESCRIPTION OF THE INVENTION

To facilitate understanding of the present disclosure, certain terms are first defined. In addition, it should be noted that whenever a value or a range of values for a parameter is recited, it is intended that intermediate values and ranges between the recited values are also intended to be encompassed within the scope of the present disclosure.

As used herein, the articles "a" and "an" refer to one or more than one *(i.e.,* at least one) of the grammatical object of the article. By way of example, "an element" means one element or more than one element, *e.g.,* a plurality of elements.

The term "including" as used herein means, and is used interchangeably with, the phrase "including, but not limited to".

The term "or" as used herein means, and is used interchangeably with, the term "and/or", unless the context clearly indicates otherwise.

As used herein, the term "about" or "approximately", as applied to one or more target values, refers to a value that is similar to the stated reference value. In certain embodiments, unless otherwise specified or clearly evident from the context, the term "approximately" or "about" refers to a value within 20%, 19%, 18%, 17%, 16%, 15%, 14%, 13%, 12%, 11%, 10%, 9%, 8%, 7%, 6%, 5%, 4%, 3%, 2%, 1%, or less of the stated reference value in either direction (greater than or less than) (unless such a range would exceed 100% of the possible value).

As used herein, "LPA" refers to the gene encoding apolipoprotein(a) or the protein expressed by the gene.

The term "LPA gene" may refer to a wild-type LPA gene or a mutant LPA gene containing sequence variations. Numerous sequence variations in the LPA gene have been identified and can be found in databases such as NCBI dbSNP and UniProt (see, for example, ncbi.nlm.nih.gov/snp).

"G", "C", "A", and "U" each generally represent nucleotides containing guanine, cytosine, adenine, and uracil as their bases, respectively. "T" and "dT" are used interchangeably herein and refer to a deoxyribonucleotide in which the nucleobase is thymine, such as deoxyribothymine, 2'-deoxythymidine, or thymidine. However, it should be understood that the term "ribonucleotide", "nucleotide", or "deoxyribonucleotide" may also refer to a modified nucleotide (as described in further detail below) or a surrogate replacement moiety. Those skilled in the art will readily appreciate that guanine, cytosine, adenine, and uracil may be replaced by other moieties without substantially altering the base pairing properties of an oligonucleotide (including a nucleotide comprising such a replacement moiety). For example, without limitation, a nucleotide containing inosine as its base may pair with a nucleotide containing adenine, cytosine, or uracil. Hence, nucleotides containing uracil, guanine, or adenine may be replaced in the nucleotide sequences of the present disclosure by a nucleotide containing, for example, inosine. Sequences containing such replacement moieties are applicable to, but are not limited to, double-stranded ribonucleic acids, double-stranded ribonucleic acid derivatives, double-stranded ribonucleic acid conjugates, pharmaceutical compositions, and methods of the present disclosure.

The terms "complementary", "fully complementary", and "substantially complementary", as used herein, may refer to base pairing between the sense and antisense strands of an siRNA, or between the antisense strand of an siRNA and a target sequence, as understood from the context in which they are used. In some embodiments of the present disclosure, a first nucleotide sequence may be considered complementary to a second nucleotide sequence if the two sequences exhibit at least 70%, 75%, 80%, 85%, 90%, 95%, 96%, 97%, 98%, or 99% sequence complementarity. In an exemplary embodiment, 18 out of 20 nucleobases in the first nucleotide sequence pair with a corresponding region of the second nucleotide sequence, thereby achieving 90% complementarity.

The terms "double-stranded ribonucleic acid", "double-stranded RNA (dsRNA) molecule", "dsRNA", and "RNA duplex" are used interchangeably and refer to a complex of ribonucleic acid molecules having a double-stranded structure, comprising two antiparallel, complementary or substantially complementary nucleic acid strands, which possess "sense" and "antisense" orientations relative to a target gene, such as the LPA gene. In some examples, double-stranded ribonucleic acid (dsRNA) triggers degradation of a target RNA, such as mRNA, through a post-transcriptional gene silencing mechanism (referred to herein as RNA interference or RNAi). As used herein, the term "siRNA duplex" in some cases also typically refers to the technical meaning as defined above.

As is well known in the art, the terms "siRNA duplex", "double-stranded RNAi agent", "RNAi agent", "small interfering ribonucleic acid", or "siRNA" refer to small RNAi molecules. These are a class of double-stranded RNA molecules, also known in the art as short interfering RNA or silencing RNA. siRNA typically comprises a sense strand (also referred to as a passenger strand) and an antisense strand (also referred to as a guide strand), each of which is 17 to 30 nucleotides in length, typically 19 to 25 nucleotides in length, wherein the antisense strand is complementary (e.g., at least 95% complementary, such as fully complementary) to a target nucleic acid (suitably a mature mRNA sequence), and the sense strand is complementary to the antisense strand, such that the sense and antisense strands form a duplex or duplex region. The sense and antisense strands of an siRNA may form a blunt-ended duplex, or may form a duplex containing a 3' overhang, which may be, for example, of 1, 2, or 3 nucleotides in length, similar to products generated by Dicer, which can form substrates for RISC *in vivo.* Effective extended forms of Dicer substrates have been described in US 8,349,809 and US 8,513,207, which are incorporated herein by reference. In some examples, both the sense strand and the antisense strand have a 3' overhang of two nucleotides in length. Accordingly, the duplex region may be, for example, 17, 18, 19, 20, 21, 22, 23, 24, or 25 nucleotides in length, such as 19, 20, 21, 22, or 23 nucleotides in length.

Moreover, as used herein, "siRNA" in some cases also refers to a "core sequence". As used herein, the term "core sequence" in some cases specifically refers to an siRNA duplex in which each nucleotide in the double-stranded ribonucleic acid is an unmodified nucleotide, and it also appears in the context as "motif" or "siRNA motif". Accordingly, in the present disclosure, the terms "siRNA", "core sequence", "motif", and "siRNA motif" are used interchangeably, and their meanings also include the corresponding nucleotide arrangement of the siRNA duplex to which they refer. In the present disclosure, those skilled in the art can clearly understand the precise technical meaning of these terms based on the context. Furthermore, the 5'-terminal nucleotide of the antisense strand of the motif may be linked to a 5'-phosphate group or a 5'-phosphate derivative, or may not be linked to a 5'-phosphate group or a 5'-phosphate derivative.

As used herein, the term "siRNA derivative" refers to a double-stranded ribonucleic acid comprising at least one modified nucleotide, and in some cases also appears as a "double-stranded ribonucleic acid derivative". In the present disclosure, the siRNA motif is modified using different modification patterns to prepare corresponding siRNA derivatives. For example, in some embodiments, the motif is modified using an alternating modification pattern, thereby obtaining an alternately modified siRNA derivative. In other embodiments, the motif is modified using a specific modification template, thereby obtaining an siRNA derivative with the specific modification template. In still other embodiments, the motif is modified using the anti-off-target modification pattern described herein, thereby obtaining an siRNA derivative with anti-off-target modifications. In some cases, multiple different modification patterns may be simultaneously applied to the same siRNA motif, thereby obtaining corresponding siRNA derivatives with multiple modification patterns.

As used herein, the term "siRNA conjugate" refers to a double-stranded ribonucleic acid conjugate or a conjugate of a double-stranded ribonucleic acid derivative obtained by attaching a conjugate moiety to a double-stranded ribonucleic acid or a double-stranded ribonucleic acid derivative. Preferably, the term "siRNA conjugate" refers to a conjugate of a double-stranded ribonucleic acid derivative.

As used herein, the term "siRNA" in some cases not only refers to the unmodified siRNA duplex (or siRNA motif) described above, but may also refer to the corresponding siRNA derivative and/or siRNA conjugate. For example, in contexts involving, but not limited to, therapeutic methods or therapeutic agents, "siRNA" broadly refers to at least one of an siRNA motif, an siRNA derivative, and/or an siRNA conjugate. Those skilled in the art can clearly understand the specific technical meaning based on the context.

The term "antisense strand" refers to the strand of a double-stranded ribonucleic acid *(e.g.,* the RNA duplex described herein) that includes a region substantially complementary to a target sequence. As used herein, the term "region of complementarity" refers to a region on the antisense strand that is substantially complementary to a sequence defined herein *(e.g.,* a target sequence). When the region of complementarity is not fully complementary to the target sequence, mismatches may occur in the internal or terminal regions of the molecule. Typically, mismatches are most tolerated in the terminal regions, for example, within 5, 4, 3, 2, or 1 nucleotides from the 5' and/or 3' ends.

As used herein, the term "sense strand" refers to the strand of a double-stranded ribonucleic acid that includes a region substantially complementary to the antisense strand (as defined herein).

The term "alternating modification" refers to a modification pattern in which nucleotides are modified with 2'-O-methyl (2'-OMe) and 2'-fluoro (2'-F) alternately according to the nucleotide arrangement of a double-stranded ribonucleic acid. For example, for the antisense strand of an siRNA, odd-numbered positions *(i.e.,* positions 1, 3, 5, 7, 11, 13, 15, 17, 19, 21, and 23) are modified with 2'-O-methyl, while even-numbered positions *(i.e.,* positions 2, 4, 6, 8, 10, 12, 14, 16, 18, 20, and 22) are modified with 2'-fluoro. For the sense strand complementary to the antisense strand, the positions corresponding to those modified with 2'-O-methyl on the antisense strand are modified with 2'-fluoro, while the positions corresponding to those modified with 2'-fluoro on the antisense strand are modified with 2'-O-methyl on the sense strand.

For RNA interference (RNAi), inhibition of a target gene is achieved by loading the antisense strand of an siRNA into the AGO2 protein to form an RNA-induced silencing complex (RISC), which then cleaves the mRNA transcript of the gene. Loading of the RNA-induced silencing complex (RISC) requires phosphorylation at the 5' end of the antisense strand (5'-phosphate). 5' phosphorylation may occur naturally in cells through the action of cleavage and polyadenylation factor I subunit 1 (Clp1), or may be introduced through chemical synthesis. The term "natural 5' phosphorylation" or "simple direct 5' phosphorylation" refers to phosphorylation at the 5' end of the antisense strand of siRNA occurring in the intracellular environment, rather than being introduced through chemical synthesis.

As used herein, the term "conjugate moiety" refers to a GalNAc derivative attached to an oligonucleotide. In some cases, the conjugate moiety comprises a targeting moiety (also referred to as a ligand), and optionally a linker; for example, a GalNAc derivative attached to an oligonucleotide via a linker (*e.g.,* a divalent, trivalent, or tetravalent branched linker arm); for another example, a GalNAc derivative attached to an oligonucleotide via a monovalent linker arm. In most cases, the terms "ligand" and "conjugate moiety" have meanings well known in the art.

As used herein, the term "inhibiting" is used interchangeably with "reducing", "silencing", "downregulating", "suppressing", and other similar terms, and includes any level of inhibition. As used herein, the term "regulating" in some cases refers to "inhibiting", and those skilled in the art can clearly understand the intended meaning based on the context.

As used herein, the phrase "inhibiting expression of LPA" includes inhibition of expression of any LPA gene (*e.g.,* a mouse LPA gene, a rat LPA gene, a monkey LPA gene, or a human LPA gene) as well as variants (*e.g.*, naturally occurring variants) or mutants of the LPA gene. Thus, the LPA gene may be a wild-type LPA gene, a mutant LPA gene, or a transgenic LPA gene in the context of genetically manipulated cells, cell populations, or organisms.

"Inhibiting LPA gene expression" includes any level of inhibition of the LPA gene, *e.g.,* at least partial inhibition of LPA gene expression, such as inhibition of at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%.

LPA gene expression may be assessed based on the level of any variable associated with LPA gene expression, such as LPA mRNA levels or LPA protein levels. The inhibition may be assessed by a reduction in the absolute or relative level of one or more of these variables compared to a control level. The control level may be any type of control level utilized in the art, *e.g.,* a pre-dose baseline level or a level determined from a similar subject, cell, or sample that is untreated or treated with a control (*e.g.,* a buffer-only control or an inert control).

As used herein, the term "regulating" in some cases may carry the same meaning as "inhibiting". Accordingly, "regulating LPA gene expression" may mean "inhibiting LPA gene expression." Those skilled in the art can clearly understand the specific technical meaning based on the context.

As used herein, a "patient" or "subject" is intended to include humans or non-human animals, preferably mammals, such as monkeys. More preferably, the subject or patient is a human.

As used herein, an "LPA-associated disease" is intended to include any disease associated with the LPA gene or protein. The disease may be caused, for example, by overproduction of the LPA protein, by mutations in the LPA gene, by abnormal cleavage of the LPA protein, or by abnormal interaction between LPA and other proteins or other endogenous or exogenous substances. Exemplary LPA-associated diseases include Berger's disease, peripheral arterial disease, coronary artery disease, metabolic syndrome, acute coronary syndrome, aortic stenosis, aortic regurgitation, aortic dissection, retinal artery occlusion, cerebrovascular disease, mesenteric ischemia, superior mesenteric artery occlusion, renal artery stenosis, stable or unstable angina, acute coronary syndrome, heterozygous or homozygous familial hypercholesterolemia, hyperapobetalipoproteinemia, cerebrovascular atherosclerosis, cerebrovascular disease and venous thrombosis, stroke, atherosclerosis, thrombosis, coronary heart disease or aortic stenosis, and/or any other disease associated with elevated levels of Lp(a) particles, as well as other unidentified related disorders, pathologies, or syndromes.

As used herein, a "therapeutically effective amount" is intended to include an amount of an RNAi agent that, when administered to a patient for treating an LPA-associated disease, is sufficient to treat the disease *(e.g.,* by attenuating, ameliorating, or maintaining the existing disease or one or more symptoms of the disease). The "therapeutically effective amount" may vary depending on the RNAi agent, how the agent is administered, the disease and its severity, medical history, age, weight, family history, genetic makeup, stage of pathological processes mediated by the expression of LPA, type of prior or concomitant therapy (if any), and other individual characteristics of the patient to be treated.

As used herein, a "prophylactically effective amount" is intended to include an amount of an RNAi agent that, when administered to a subject who has not yet experienced or exhibited symptoms of an LPA-associated disease, but who may be susceptible to the disease, is sufficient to prevent or ameliorate the disease or one or more symptoms of the disease. Amelioration of the disease includes slowing the progression of the disease or reducing the severity of later-onset disease. The "prophylactically effective amount" may vary depending on the RNAi agent, how the agent is administered, degree of risk of the disease, medical history, age, weight, family history, genetic makeup, type of prior or concomitant therapy (if any), and other individual characteristics of the patient to be treated.

The "therapeutically effective amount" or "prophylactically effective amount" also includes an amount of an RNAi agent that produces a desired local or systemic effect at a reasonable benefit/risk ratio applicable to any treatment. The RNAi agent used in the methods of the present disclosure may be administered in an amount sufficient to achieve a reasonable benefit/risk ratio applicable to such treatment.

As used herein, the term "sample" includes a collection of similar fluids, cells, or tissues isolated from a subject, as well as fluids, cells, or tissues present in the subject. Examples of biological fluids include blood, serum and serous fluid, plasma, cerebrospinal fluid, ocular fluid, lymph, urine, saliva, *etc.* Tissue samples may include samples from tissues, organs, or localized regions. For example, samples may be derived from specific organs, parts of organs, or fluids or cells within these organs. In certain examples, samples may be derived from the liver *(e.g.,* the entire liver or certain segments of the liver, or certain types of cells in the liver, such as hepatocytes). In a preferred example, a "sample derived from a subject" refers to blood or plasma drawn from the subject. In other examples, a "sample derived from a subject" refers to liver tissue (or subcomponents thereof) derived from the subject.

As used herein, unless otherwise specified, when referring to any nucleotide position on any strand of an siRNA motif, siRNA derivative, siRNA conjugate, siRNA duplex, *etc.,* the positions are indicated in the 5' to 3' direction.

In one aspect, the present disclosure provides an siRNA duplex comprising a sense strand and an antisense strand that form a reverse complementary duplex region, wherein the antisense strand comprises a fragment of at least 15, 16, 17, 18, or 19 consecutive nucleotides complementary to a target sequence of the nucleotide sequence as shown in SEQ ID NO: 184, SEQ ID NO: 76, SEQ ID NO: 132, SEQ ID NO: 419 (AUGGUAAUGGACAGAGUU), or SEQ ID NO: 420 (GACAGAGUUAUCAAGGCA), or a modified fragment thereof.

The target sequence is derived from *Homo sapiens* lipoprotein(a) (LPA), mRNA, with NCBI accession number NM_005577.4.

In some embodiments, the antisense strand comprises a fragment of at least 15, 16, 17, 18, or 19 consecutive nucleotides in any one of the sequences of SEQ ID NOs: 194, 214, 232, 237, 269, 325, 377, and 379, or a modified fragment thereof.

In some embodiments, the reverse complementary duplex region is 17 to 21 bp in length, such as 20 or 21 bp in length.

In some embodiments, the sense strand comprises a fragment of at least 15, 16, 17, 18, or 19 consecutive nucleotides in any one of the sequences of SEQ ID NOs: 1, 21, 39, 44, 76, 132, 184, and 186, or a modified fragment thereof.

In some embodiments, the sense strand and the antisense strand each independently comprise 19 to 23 nucleotides; preferably, the sense strand comprises 19 to 21 nucleotides, and the antisense strand comprises 21 to 23 nucleotides.

In some embodiments, the siRNA duplex comprises any one oligonucleotide duplex selected from the following pairings of a sense strand and an antisense strand, or a combination of at least two thereof, optionally wherein the sense strand and the antisense strand each independently comprise at least one modified nucleotide:
(1) the sense strand has the sequence of SEQ ID NO: 21 or a fragment thereof, or a modified sequence of the sequence or the fragment thereof; and the antisense strand has the sequence of SEQ ID NO: 214 or a fragment thereof, or a modified sequence of the sequence or the fragment thereof;
(2) the sense strand has the sequence of SEQ ID NO: 1 or a fragment thereof, or a modified sequence of the sequence or the fragment thereof; and the antisense strand has the sequence of SEQ ID NO: 194 or a fragment thereof, or a modified sequence of the sequence or the fragment thereof;
(3) the sense strand has the sequence of SEQ ID NO: 39 or a fragment thereof, or a modified sequence of the sequence or the fragment thereof; and the antisense strand has the sequence of SEQ ID NO: 232 or a fragment thereof, or a modified sequence of the sequence or the fragment thereof;
(4) the sense strand has the sequence of SEQ ID NO: 44 or a fragment thereof, or a modified sequence of the sequence or the fragment thereof; and the antisense strand has the sequence of SEQ ID NO: 237 or a fragment thereof, or a modified sequence of the sequence or the fragment thereof;
(5) the sense strand has the sequence of SEQ ID NO: 76 or a fragment thereof, or a modified sequence of the sequence or the fragment thereof; and the antisense strand has the sequence of SEQ ID NO: 269 or a fragment thereof, or a modified sequence of the sequence or the fragment thereof;
(6) the sense strand has the sequence of SEQ ID NO: 132 or a fragment thereof, or a modified sequence of the sequence or the fragment thereof; and the antisense strand has the sequence of SEQ ID NO: 325 or a fragment thereof, or a modified sequence of the sequence or the fragment thereof;
(7) the sense strand has the sequence of SEQ ID NO: 184 or a fragment thereof, or a modified sequence of the sequence or the fragment thereof; and the antisense strand has the sequence of SEQ ID NO: 377 or a fragment thereof, or a modified sequence of the sequence or the fragment thereof;
(8) the sense strand has the sequence of SEQ ID NO: 186 or a fragment thereof, or a modified sequence of the sequence or the fragment thereof; and the antisense strand has the sequence of SEQ ID NO: 379 or a fragment thereof, or a modified sequence of the sequence or the fragment thereof.

In some embodiments, the siRNA duplex is an RNAi agent for inhibiting LPA gene expression.

In some embodiments, the sense strand differs from any one of the sequences of SEQ ID NOs: 1, 21, 39, 44, 76, 132, 184, and 186 by 1 to 3 nucleotides.

In some embodiments, the antisense strand differs from any one of the sequences of SEQ ID NOs: 194, 214, 232, 237, 269, 325, 377, and 379 by 1 to 3 nucleotides.

In some embodiments, the number of nucleotides in the sense strand is either the same as or different from the number of nucleotides in the antisense strand.

In some embodiments, the sense strand comprises 19 nucleotides and the antisense strand comprises 19 nucleotides.

In some embodiments, the sense strand comprises 19 nucleotides and the antisense strand comprises 21 nucleotides.

In some embodiments, the sense strand comprises 20 nucleotides and the antisense strand comprises 20 nucleotides.

In some embodiments, the sense strand comprises 20 nucleotides and the antisense strand comprises 22 nucleotides.

In some embodiments, the sense strand comprises 21 nucleotides and the antisense strand comprises 21 nucleotides.

In some embodiments, the sense strand comprises 21 nucleotides and the antisense strand comprises 23 nucleotides.

In some embodiments, the sense strand comprises 23 nucleotides and the antisense strand comprises 23 nucleotides.

In some embodiments, the sense strand and the antisense strand each independently comprise at least one modified nucleotide.

In some embodiments, the at least one modified nucleotide is any one selected from the group consisting of a deoxy-nucleotide, a 3'-deoxy-thymidine (dT) nucleotide, a 2'-O-methyl modified nucleotide, a 2'-fluoro modified nucleotide, a 2'-deoxy-modified nucleotide, a locked nucleotide, an unlocked nucleotide, a conformationally restricted nucleotide, a constrained ethyl nucleotide, an abasic nucleotide, a 2'-amino-modified nucleotide, a 2'-O-allyl-modified nucleotide, a 2'-C-alkyl-modified nucleotide, a 2'-hydroxy-modified nucleotide, a 2'-methoxyethyl modified nucleotide, a 2'-O-alkyl-modified nucleotide, a morpholino nucleotide, a phosphoramidate, a nucleotide comprising a non-natural base, a tetrahydropyran modified nucleotide, a 1,5-anhydrohexitol modified nucleotide, a cyclohexenyl modified nucleotide, a nucleotide comprising a phosphorothioate group, a nucleotide comprising a methylphosphonate group, a nucleotide comprising a 5'-phosphate group, and a nucleotide comprising a 5'-phosphate mimic, or a combination of at least two thereof.

In some embodiments, at least one strand comprises a 3' overhang of at least one nucleotide.

In some embodiments, at least one strand comprises a 3' overhang of at least two nucleotides.

In some embodiments, all nucleotides on the sense and antisense strands are modified at the 2' position of the ribose of the nucleotide.

In some embodiments, the modification at the 2' position of the ribose of the nucleotide is any one selected from 2'-O-methyl modification, 2'-methoxyethyl modification, 2'-fluoro modification, 2'-benzyloxy modification, 2'-methylcarbonylamino modification, and 2'-pyridinylmethoxy modification, or a combination thereof.

In some embodiments, the modification at the 2' position of the ribose of each nucleotide is selected from a combination of 2'-O-methyl modification and 2'-fluoro modification.

In some embodiments, the modification at the 2' position of the ribose of each nucleotide is selected from an alternating combination of 2'-O-methyl modification and 2'-fluoro modification.

In some embodiments, the modification pattern at the 2' position of the ribose of each nucleotide is as follows: on the sense strand, all odd-numbered positions are modified with 2'-fluoro, and all even-numbered positions are modified with 2'-O-methyl; and on the antisense strand, all odd-numbered positions are modified with 2'-O-methyl, and all even-numbered positions are modified with 2'-fluoro.

In some embodiments, nucleotides are linked to one another via a 3',5'-phosphodiester bond.

In some embodiments, nucleotides are linked to one another via a 3',5'-phosphorothioate diester bond.

In some embodiments, the aforementioned oligonucleotides have alternating 2'-fluoro and 2'-O-methyl modifications or template modifications.

In some embodiments, the antisense strand of the siRNA derivative has any one of the following modification patterns A to F shown below:

| No. | **Nucleotide position (5'-3')** | | | | | | | | | | | |
|---|---|---|---|---|---|---|---|---|---|---|---|---|
| **A** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **B** | **1** | **2** | **3** | **4** | **5** | **6** | **7** | **8** | **9** | **10** | **11** | **12** |
| | 2'-OMe +PS +EVP | **2'-F +PS** | **2'-F** | **2'-F** | 2'-OMe | 2'-F | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **C** | **1** | **2** | **3** | **4** | **5** | **6** | **7** | **8** | **9** | **10** | **11** | **12** |
| | 2'-OMe +PS | **2'-F +PS** | 2'-OMe | **2'-F** | **2'-F** | **2'-F** | 2'-OMe | 2'-OMe | 2'-OMe | 2'-OMe | 2'-OMe | 2'-OMe |
| | +EVP | | | | | | | | | | | |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **D** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **E** | **1** | **2** | **3** | **4** | **5** | **6** | **7** | **8** | **9** | **10** | **11** | **12** |
| | 2'-OMe +PS +EVP | **2'-F +PS** | 2'-OMe | **2'-F** | **2'-F** | 2'-OMe | 2'-OMe | 2'-OMe | **2'-F** | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **F** | **1** | **2** | **3** | **4** | **5** | **6** | **7** | **8** | **9** | **10** | **11** | **12** |
| | 2'-OMe +PS +EVP | **2'-F +PS** | **2'-F** | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | 2'-F | 2'-OMe | **2'-F** | 2'-OMe | 2'-OMe | 2'-OMe | 2'-OMe | 2'-OMe +PS | 2'-OMe +PS | 2'-OMe | |

and/or, the sense strand adopts modification pattern a or b:

| No. | **Nucleotide position (5'-3')** | | | | | | | | | | |
|---|---|---|---|---|---|---|---|---|---|---|---|
| **a** | **1** | **2** | **3** | **4** | **5** | **6** | **7** | **8** | **9** | **10** | **11** |
| | 2'-OMe+PS | 2'-OMe+PS | 2'-OMe | 2'-OMe | 2'-OMe | 2'-OMe | **2'-F** | 2'-OMe | **2'-F** | **2'-F** | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | **2'-F** | 2'-OMe | 2'-OMe | 2'-OMe | 2'-OMe | |
| **b** | **1** | **2** | **3** | **4** | **5** | **6** | **7** | **8** | **9** | **10** | **11** |
| | 2'-OMe+PS | 2'-OMe+PS | 2'-OMe | 2'-OMe | 2'-OMe | 2'-OMe | **2'-F** | 2'-OMe | **2'-F** | 2'-OMe | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | **2'-F** | 2'-OMe | 2'-OMe | 2'-OMe | 2'-OMe | |

wherein 2'-OMe represents a 2'-O-methyl modification; 2'-F represents a 2'-fluoro modification; PS indicates that, starting from the 5' end, the nucleotide at the position is linked to the adjacent nucleotide on the right via a 3',5'-phosphorothioate bond; EVP represents a 5'-vinyl-(E)-phosphonate.

In some embodiments, the antisense strand is modified according to modification pattern A, and the sense strand is modified according to modification pattern a.

In some embodiments, the antisense strand is modified according to modification pattern B, and the sense strand is modified according to modification pattern a.

In some embodiments, the antisense strand is modified according to modification pattern C, and the sense strand is modified according to modification pattern a.

In some embodiments, the antisense strand is modified according to modification pattern D, and the sense strand is modified according to modification pattern a.

In some embodiments, the antisense strand is modified according to modification pattern E, and the sense strand is modified according to modification pattern a.

In some embodiments, the antisense strand is modified according to modification pattern F, and the sense strand is modified according to modification pattern a.

In some embodiments, the antisense strand is modified according to modification pattern A, and the sense strand is modified according to modification pattern b.

In some embodiments, the antisense strand is modified according to modification pattern B, and the sense strand is modified according to modification pattern b.

In some embodiments, the antisense strand is modified according to modification pattern C, and the sense strand is modified according to modification pattern b.

In some embodiments, the antisense strand is modified according to modification pattern D, and the sense strand is modified according to modification pattern b.

In some embodiments, the antisense strand is modified according to modification pattern E, and the sense strand is modified according to modification pattern b.

In some embodiments, the antisense strand is modified according to modification pattern F, and the sense strand is modified according to modification pattern b.

In some embodiments, the antisense strand of the siRNA derivative has any one of the following modification patterns A' to F' shown below:

| No. | **Nucleotide position (5'-3')** | | | | | | | | | | |
|---|---|---|---|---|---|---|---|---|---|---|---|
| | **1** | **2** | **3** | **4** | **5** | **6** | **7** | **8** | **9** | **10** | **11** |
| **A'** | 2'-OMe +PS +EVP | **2'-F +PS** | 2'-OMe | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe | 2'-OMe | 2'-OMe |
| | | | | | | | | | +PS | +PS | |
| **B'** | **1** | **2** | **3** | **4** | **5** | **6** | **7** | **8** | **9** | **10** | **11** |
| | 2'-OMe +PS +EVP | **2'-F +PS** | **2'-F** | **2'-F** | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **C'** | **1** | **2** | **3** | **4** | **5** | **6** | **7** | **8** | **9** | **10** | **11** |
| | 2'-OMe +PS +EVP | **2'-F +PS** | 2'-OMe | **2'-F** | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **D'** | **1** | **2** | **3** | **4** | **5** | **6** | **7** | **8** | **9** | **10** | **11** |
| | 2'-OMe +PS +EVP | **2'-F +PS** | 2'-OMe | 2'-OMe | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe+PS | 2'-OMe+PS | 2'-OMe |
| **E'** | **1** | **2** | **3** | **4** | **5** | **6** | **7** | **8** | **9** | **10** | **11** |
| | 2'-OMe +PS +EVP | **2'-F +PS** | 2'-OMe | **2'-F** | **2'-F** | 2'-OMe | 2'-OMe | 2'-OMe | **2'-F** | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **F'** | **1** | **2** | **3** | **4** | **5** | **6** | **7** | **8** | **9** | **10** | **11** |
| | 2'-OMe +PS +EVP | **2'-F +PS** | **2'-F** | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |

and/or, the sense strand adopts modification pattern a' or b':

| No. | **Nucleotide position (5'-3')** | | | | | | | | | |
|---|---|---|---|---|---|---|---|---|---|---|
| **a'** | **1** | **2** | **3** | **4** | **5** | **6** | **7** | **8** | **9** | **10** |
| | 2'-OMe+PS | 2'-OMe+PS | 2'-OMe | 2'-OMe | 2'-OMe | **2'-F** | 2'-OMe | **2'-F** | **2'-F** | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | **2'-F** | 2'-OMe | 2'-OMe | 2'-OMe | 2'-OMe |
| **b'** | **1** | **2** | **3** | **4** | **5** | **6** | **7** | **8** | **9** | **10** |
| | 2'-OMe+PS | 2'-OMe+PS | 2'-OMe | 2'-OMe | 2'-OMe | **2'-F** | 2'-OMe | **2'-F** | 2'-OMe | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | **2'-F** | 2'-OMe | 2'-OMe | 2'-OMe | 2'-OMe |

wherein 2'-OMe represents a 2'-O-methyl modification; 2'-F represents a 2'-fluoro modification; PS indicates that, starting from the 5' end, the nucleotide at the position is linked to the adjacent nucleotide on the right via a 3',5'-phosphorothioate bond; EVP represents a 5'-vinyl-(E)-phosphonate.

In some embodiments, the antisense strand is modified according to modification pattern A', and the sense strand is modified according to modification pattern a'.

In some embodiments, the antisense strand is modified according to modification pattern B', and the sense strand is modified according to modification pattern a'.

In some embodiments, the antisense strand is modified according to modification pattern C', and the sense strand is modified according to modification pattern a'.

In some embodiments, the antisense strand is modified according to modification pattern D', and the sense strand is modified according to modification pattern a'.

In some embodiments, the antisense strand is modified according to modification pattern E', and the sense strand is modified according to modification pattern a'.

In some embodiments, the antisense strand is modified according to modification pattern F', and the sense strand is modified according to modification pattern a'.

In some embodiments, the antisense strand is modified according to modification pattern A', and the sense strand is modified according to modification pattern b'.

In some embodiments, the antisense strand is modified according to modification pattern B', and the sense strand is modified according to modification pattern b'.

In some embodiments, the antisense strand is modified according to modification pattern C', and the sense strand is modified according to modification pattern b'.

In some embodiments, the antisense strand is modified according to modification pattern D', and the sense strand is modified according to modification pattern b'.

In some embodiments, the antisense strand is modified according to modification pattern E', and the sense strand is modified according to modification pattern b'.

In some embodiments, the antisense strand is modified according to modification pattern F', and the sense strand is modified according to modification pattern b'.

In some embodiments, nucleotides at positions 2 to 8 from the 5' end of the antisense strand are each independently a modified nucleotide, wherein the modified nucleotide is UNA or GNA; or, nucleotides at positions 2 to 8 from the 5' end of the antisense strand are each independently DNA, wherein the structures of UNA and GNA are as follows: 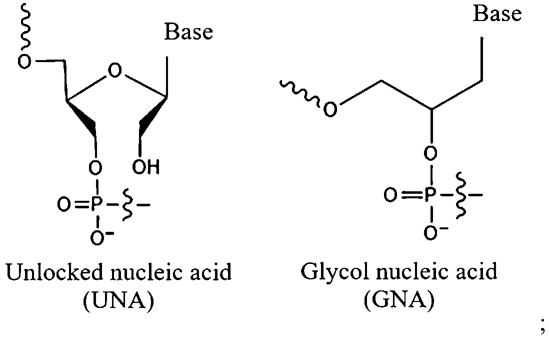 wherein the base is any one selected from adenine, guanine, cytosine, thymine, and uracil.

In some embodiments, the 5' carbon atom of the sugar moiety of the nucleotide at the 5' end of the modified antisense strand is phosphorylated, and the phosphorylation of the 5' carbon atom includes, but is not limited to, the following 5'-phosphorylation groups: 5'-vinylphosphonate group (5'-E-VP), 5'-methylphosphonate group (5'-MP), 5'-C-methylphosphonate group, 5'-phosphorothioate group (5'-PS), and 5'-phosphate group (5'-P). The structures of the modified nucleotides are as follows: 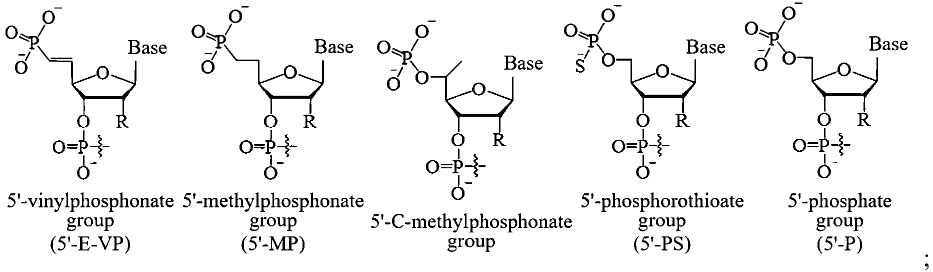
wherein R is hydrogen, hydroxyl, amino, C₁₋₄ alkyl, aryl, C₁₋₄ alkoxy, C₁₋₄ alkylcarbonylamino, or halogen;
the base is any one selected from adenine, guanine, cytosine, thymine, and uracil.

In some embodiments, there is a 3',5'-phosphorothioate diester bond between nucleotides at positions 1 to 2 from the 3' and/or 5' ends of the sense strand and/or the antisense strand of the siRNA derivative. For example, in some embodiments, a stereochemically pure 3',5'-phosphorothioate diester bond is formed. In some embodiments, one, two, or three 3',5'-phosphorothioate diester bonds may be present among nucleotides at positions 1 to 4 from the 5' end of the sense strand and/or the antisense strand, and one, two, or three 3',5'-phosphorothioate diester bonds may be present among nucleotides at positions 1 to 4 from the 3' end of the antisense strand.

In some embodiments, the siRNA duplex comprises any one oligonucleotide duplex selected from the following pairings of a sense strand and an antisense strand, or a combination of at least two thereof:
(1) the sense strand has the sequence of SEQ ID NO: 21 or a fragment thereof, or a modified sequence of the sequence or the fragment thereof; and the antisense strand has the sequence of SEQ ID NO: 214 or a fragment thereof, or a modified sequence of the sequence or the fragment thereof; wherein the antisense strand is modified according to modification pattern B, and the sense strand is modified according to modification pattern a;
(2) the sense strand has the sequence of SEQ ID NO: 1 or a fragment thereof, or a modified sequence of the sequence or the fragment thereof; and the antisense strand has the sequence of SEQ ID NO: 194 or a fragment thereof, or a modified sequence of the sequence or the fragment thereof; wherein the antisense strand is modified according to modification pattern A, and the sense strand is modified according to modification pattern a;
(3) the sense strand has the sequence of SEQ ID NO: 39 or a fragment thereof, or a modified sequence of the sequence or the fragment thereof; and the antisense strand has the sequence of SEQ ID NO: 232 or a fragment thereof, or a modified sequence of the sequence or the fragment thereof; wherein the antisense strand is modified according to modification pattern C', and the sense strand is modified according to modification pattern a';
(4) the sense strand has the sequence of SEQ ID NO: 44 or a fragment thereof, or a modified sequence of the sequence or the fragment thereof; and the antisense strand has the sequence of SEQ ID NO: 237 or a fragment thereof, or a modified sequence of the sequence or the fragment thereof; wherein the antisense strand is modified according to modification pattern C', and the sense strand is modified according to modification pattern b';
(5) the sense strand has the sequence of SEQ ID NO: 76 or a fragment thereof, or a modified sequence of the sequence or the fragment thereof; and the antisense strand has the sequence of SEQ ID NO: 269 or a fragment thereof, or a modified sequence of the sequence or the fragment thereof; wherein the antisense strand is modified according to modification pattern E, and the sense strand is modified according to modification pattern b;
(6) the sense strand has the sequence of SEQ ID NO: 132 or a fragment thereof, or a modified sequence of the sequence or the fragment thereof; and the antisense strand has the sequence of SEQ ID NO: 325 or a fragment thereof, or a modified sequence of the sequence or the fragment thereof; wherein the antisense strand is modified according to modification pattern B, and the sense strand is modified according to modification pattern a;
(7) the sense strand has the sequence of SEQ ID NO: 184 or a fragment thereof, or a modified sequence of the sequence or the fragment thereof; and the antisense strand has the sequence of SEQ ID NO: 377 or a fragment thereof, or a modified sequence of the sequence or the fragment thereof; wherein the antisense strand is modified according to modification pattern C, and the sense strand is modified according to modification pattern a;
(8) the sense strand has the sequence of SEQ ID NO: 186 or a fragment thereof, or a modified sequence of the sequence or the fragment thereof; and the antisense strand has the sequence of SEQ ID NO: 379 or a fragment thereof, or a modified sequence of the sequence or the fragment thereof; wherein the antisense strand is modified according to modification pattern E, and the sense strand is modified according to modification pattern b;
(9) the sense strand has the sequence of SEQ ID NO: 21 or a fragment thereof, or a modified sequence of the sequence or the fragment thereof; and the antisense strand has the sequence of SEQ ID NO: 214 or a fragment thereof, or a modified sequence of the sequence or the fragment thereof; wherein the antisense strand is modified according to modification pattern A, and the sense strand is modified according to modification pattern a; nucleotides at positions 5 and 7 of the antisense strand are replaced with DNA, and the nucleotide at the position of the sense strand that is complementary to position 7 of the antisense strand is also replaced with DNA;
(10) the sense strand has the sequence of SEQ ID NO: 184 or a fragment thereof, or a modified sequence of the sequence or the fragment thereof; and the antisense strand has the sequence of SEQ ID NO: 377 or a fragment thereof, or a modified sequence of the sequence or the fragment thereof; wherein the antisense strand is modified according to modification pattern C, and the sense strand is modified according to modification pattern a; nucleotides at positions 5 and 7 of the antisense strand are replaced with DNA, and the nucleotide at the position of the sense strand that is complementary to position 7 of the antisense strand is also replaced with DNA;
(11) the sense strand has the sequence of SEQ ID NO: 76 or a fragment thereof, or a modified sequence of the sequence or the fragment thereof; and the antisense strand has the sequence of SEQ ID NO: 269 or a fragment thereof, or a modified sequence of the sequence or the fragment thereof; wherein the antisense strand is modified according to modification pattern E, and the sense strand is modified according to modification pattern b; nucleotides at positions 5 and 7 of the antisense strand are replaced with DNA, and the nucleotide at the position of the sense strand that is complementary to position 7 of the antisense strand is also replaced with DNA.

The present disclosure provides an siRNA conjugate comprising an siRNA duplex described herein and a conjugate moiety linked to the siRNA duplex.

The double-stranded ribonucleic acid or double-stranded ribonucleic acid derivative of the present disclosure may optionally be linked to one or more conjugate moieties. The conjugate moiety may be attached to the sense strand, the antisense strand, or both strands at the 3' end, the 5' end, or both ends. For example, the conjugate moiety may be attached to the sense strand. In a preferred example, the conjugate moiety is attached to the 3' end of the sense strand. In one example, the conjugate moiety has an arbitrary GalNAc structure.

In some embodiments, the conjugate moiety is linked to the 3' end or 5' end of the sense strand.

In some embodiments, the conjugate moiety comprises one or more GalNAc derivatives attached using a bivalent or trivalent branched linker arm.

Typically, the conjugate moiety comprises at least one pharmaceutically acceptable targeting moiety, or further comprises a linker, wherein the siRNA, the linker, and the targeting moiety are sequentially linked. In some embodiments, the number of targeting moieties is 1 to 6. In some embodiments, the number of targeting moieties is 2 to 4. In some embodiments, the number of targeting moieties is 3. The conjugate moiety may be covalently or non-covalently linked to the siRNA molecule, wherein the linkage site may be located at the 3' end or 5' end of the sense strand, at the 5' end of the antisense strand, or within the internal sequence of the siRNA. In some embodiments, the linkage site is located at the 3' end of the sense strand of the siRNA.

In some embodiments, the pharmaceutically acceptable targeting moiety may be a conventional ligand in the field of siRNA delivery, such as various ligands described in WO2009082607A2, the contents of which are incorporated herein by reference in their entirety.

In some embodiments, each ligand is independently selected from a ligand capable of binding to a cell surface receptor. In some embodiments, at least one ligand is a ligand capable of binding to a hepatocyte surface receptor. In some embodiments, at least one ligand is a ligand capable of binding to a mammalian cell surface receptor. In some embodiments, at least one ligand is a ligand capable of binding to a human hepatocyte surface receptor. In some embodiments, at least one ligand is a ligand capable of binding to a hepatocyte surface asialoglycoprotein receptor (ASGPR). The types of these ligands are well known to those skilled in the art, and their general function is to specifically bind to receptors on the surface of target cells, thereby mediating the delivery of siRNA conjugated to the ligand into the target cells.

In some embodiments, the pharmaceutically acceptable targeting moiety in the siRNA conjugate may be galactose or N-acetylgalactosamine, wherein the galactose or N-acetylgalactosamine molecule may be monovalent, divalent, trivalent, or tetravalent. That is, when the siRNA molecule forms an siRNA conjugate with a conjugate moiety containing a galactose or N-acetylgalactosamine molecule as the targeting moiety, the molar ratio of the siRNA molecule to the galactose or N-acetylgalactosamine molecule in the siRNA conjugate is 1:1, 1:2, 1:3, or 1:4. In some embodiments, when the siRNA is conjugated with a conjugate moiety containing N-acetylgalactosamine, the N-acetylgalactosamine molecule is trivalent.

The targeting moiety may be linked to the siRNA molecule via a suitable linker, and those skilled in the art may select an appropriate linker based on the specific type of targeting moiety. The types of these linkers and targeting moieties, as well as the modes of attachment to siRNA may be found in WO2015006740A2, the contents of which are incorporated herein by reference in their entirety.

In some embodiments, the structure of the conjugate moiety is, for example, as follows: 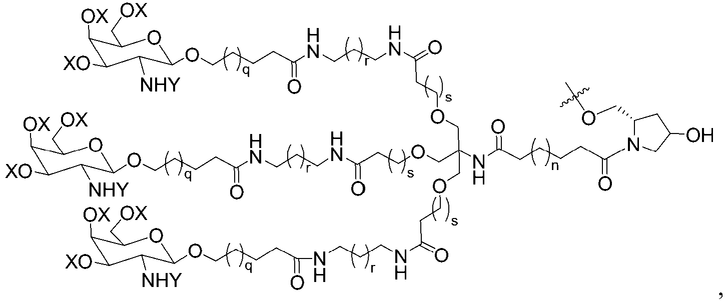 wherein X is a hydroxyl protecting group or H, the hydroxyl protecting group being selected from acetyl, benzoyl, and isobutyryl; Y is an amino protecting group or H, the amino protecting group being selected from formyl, acetyl, propionyl, n-butyryl, and isobutyryl; n is an integer from 0 to 20; q, r, and s are independently integers from 1 to 7.

In some embodiments, the structure of the conjugate moiety is, for example, as follows:

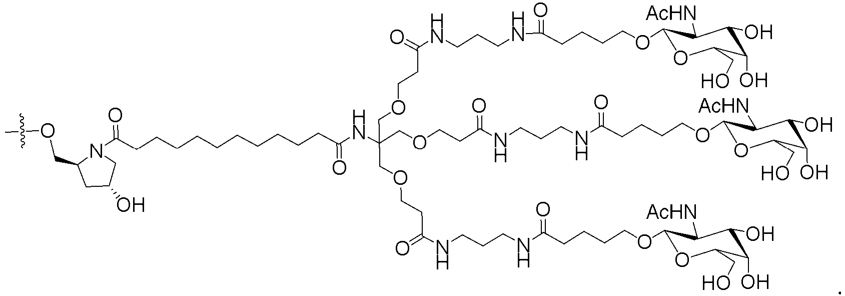

In some embodiments, the conjugate moiety is, for example, as follows: 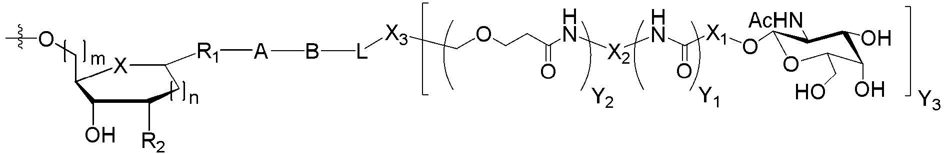
wherein X is oxygen, -N(Y)-, or sulfur;
Y is C₁₋₄ alkyl or C₆₋₁₀ aryl;
R₁ is oxygen or sulfur;
R₂ is hydrogen, -NH₂, C₁₋₄ alkyl, C₆₋₁₀ aryl, C₁₋₄ alkoxy, or halogen;
A is -(CH₂)ₐ-, -(CH₂CH₂O)_{b}-, -((CH₂)_{c}NHCO)_{d}-, or -((CH₂)_{c}CONH)_{d}-, wherein a is an integer from 1 to 15, b is an integer from 1 to 7, c is an integer from 1 to 7, and d is an integer from 1 to 5;
B is -(CH₂)ₑ-, wherein e is an integer from 0 to 7;
L is -CONH- or -NHCO-;
X₁ is -(CH₂)_{f}- or -(CH₂CH₂O)_{f}CH₂-, wherein f is an integer from 1 to 5;
X₂ is -(CH₂)_{g}-, wherein g is an integer from 1 to 6;
X₃ is oxygen or sulfur;
Y₁ is 0 or 1;
Y₂ is 0, 1, or 2;
when Y₃ is 1, X₄ is CH₂; when Y₃ is 2, X₄ is CH; when Y₃ is 3, X₃ is carbon;
m is an integer from 0 to 4;
n is an integer from 0 to 4.

In some embodiments, the conjugate moiety has, for example, any one of the following structures: 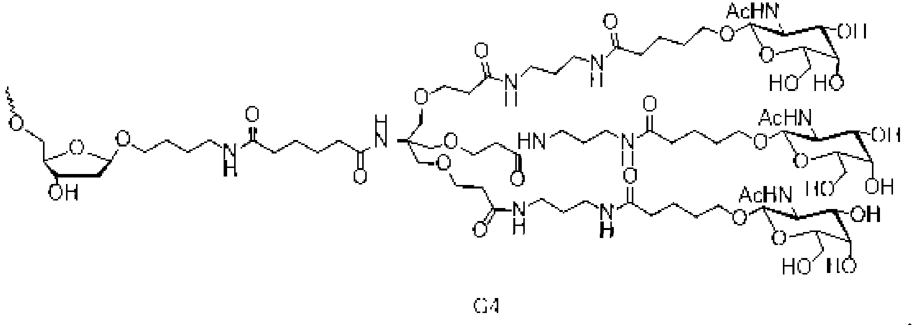 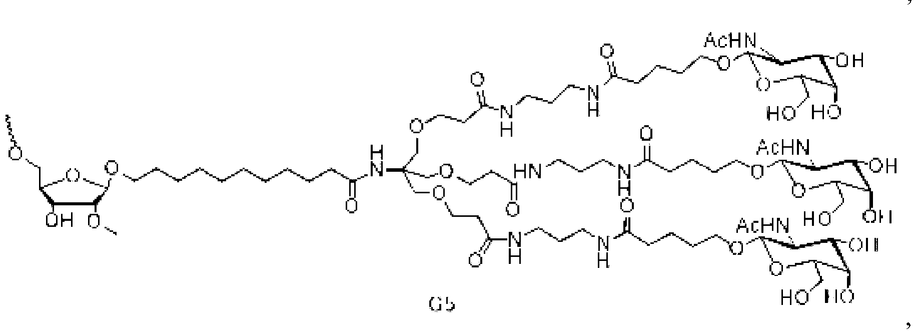 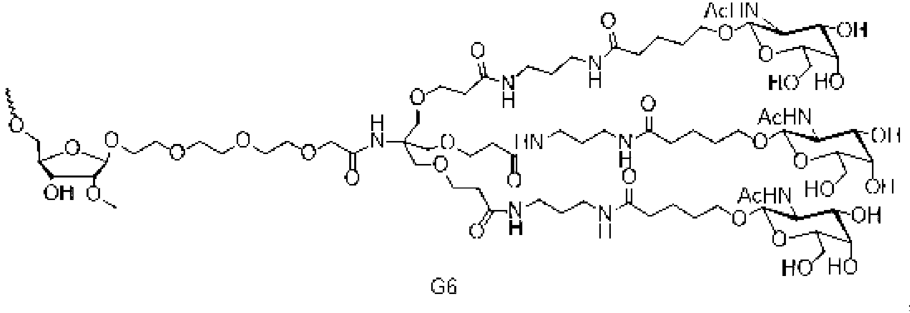 , or

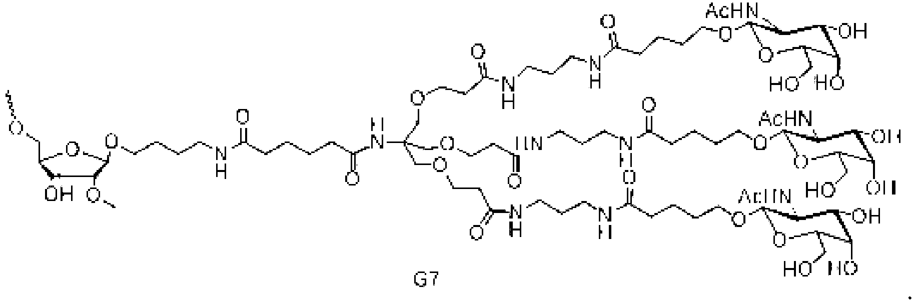

In some embodiments, the siRNA conjugate of the present disclosure may have any one of the following structures: 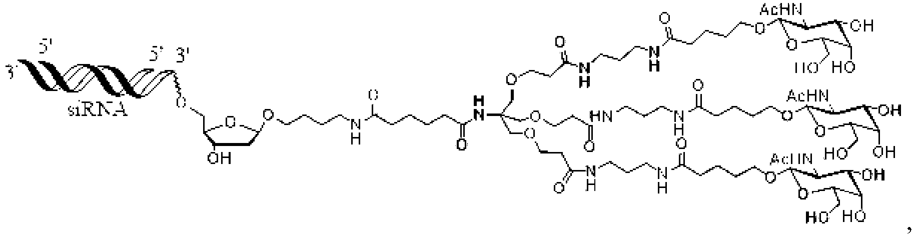 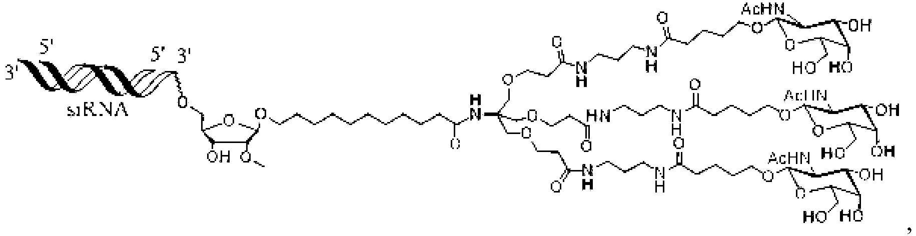 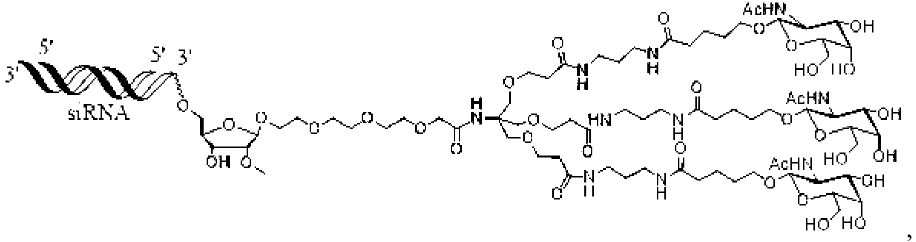 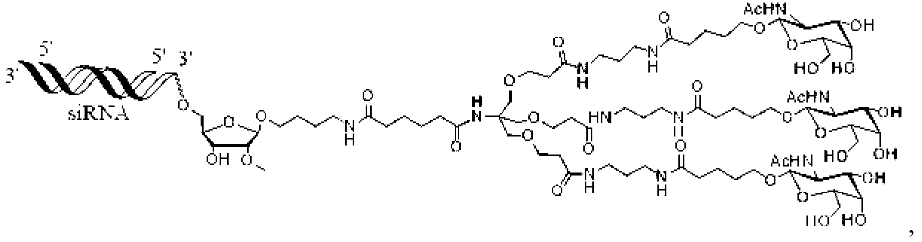 or

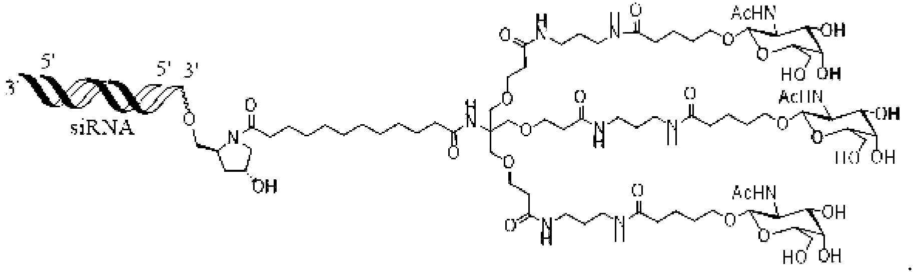

In some embodiments, the conjugate moiety has, for example, the following structure: 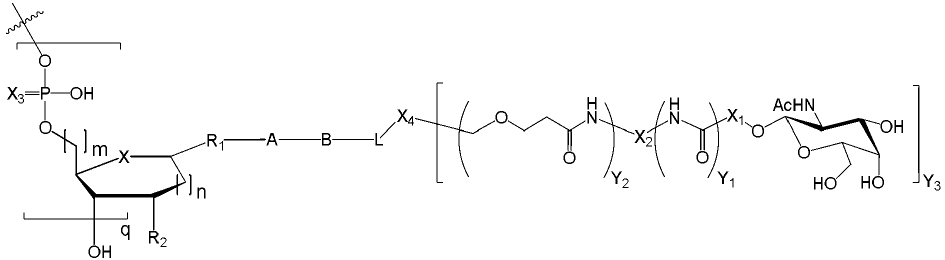
wherein X is oxygen, -N(Y)-, or sulfur;
Y is C₁₋₄ alkyl or C₆₋₁₀ aryl;
R₁ is oxygen or sulfur;
R₂ is hydrogen, -NH₂, C₁₋₄ alkyl, C₆₋₁₀ aryl, C₁₋₄ alkoxy, or halogen;
A is -(CH₂)ₐ-, -(CH₂CH₂O)_{b}-, -((CH₂)_{c}NHCO)_{d}-, or -((CH₂)_{c}CONH)_{d}-, wherein a is an integer from 1 to 15, b is an integer from 1 to 7, c is an integer from 1 to 7, and d is an integer from 1 to 5;
B is -(CH₂)ₑ-, wherein e is an integer from 0 to 7;
L is -CONH- or -NHCO-;
X₁ is -(CH₂)_{f}- or -(CH₂CH₂O)_{f}CH₂-, wherein f is an integer from 1 to 5;
X₂ is -(CH₂)_{g}-, wherein g is an integer from 1 to 6;
X₃ is oxygen or sulfur;
Y₁ is 0 or 1;
Y₂ is 0, 1, or 2;
when Y₃ is 1, X₄ is CH₂; when Y₃ is 2, X₄ is CH; when Y₃ is 3, X₃ is carbon;
m is an integer from 0 to 4;
n is an integer from 0 to 4;
q is an integer from 0 to 4.

In some embodiments, the conjugate moiety has, for example, any one of the following structures: 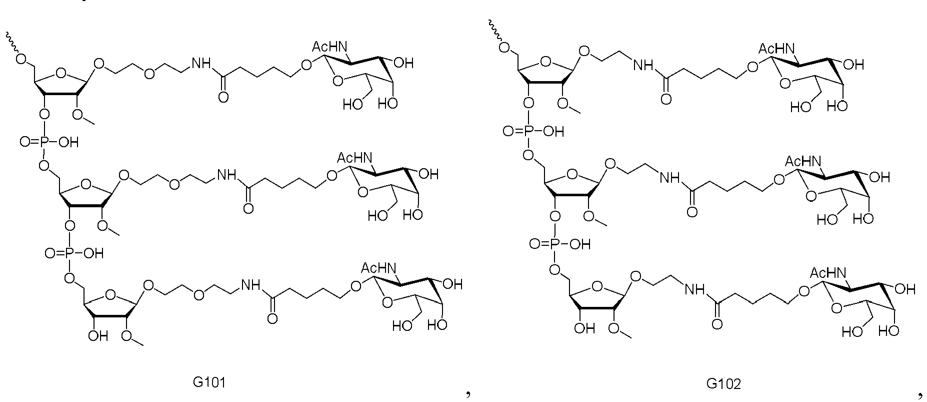 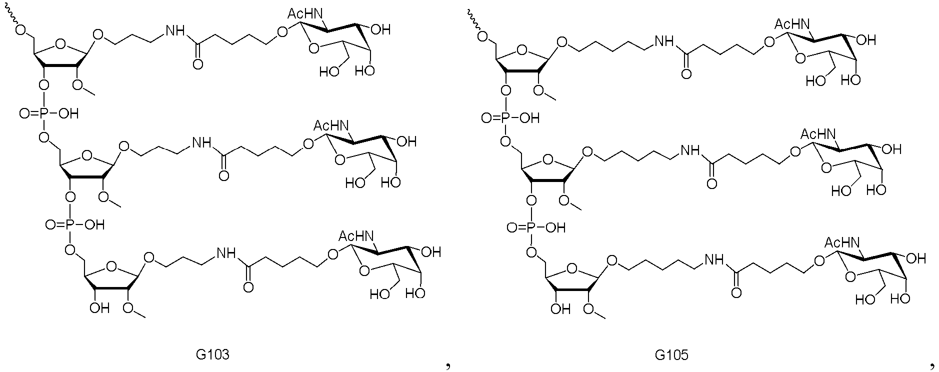 or

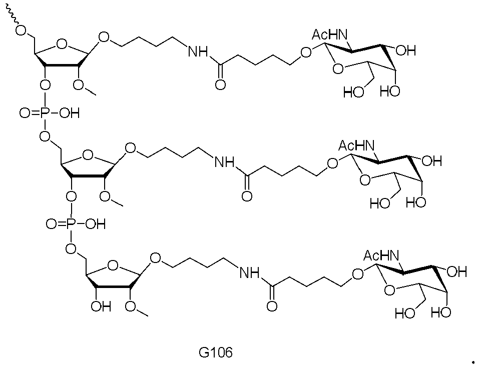

In some embodiments, the siRNA conjugate of the present disclosure has, for example, any one of the following structures:

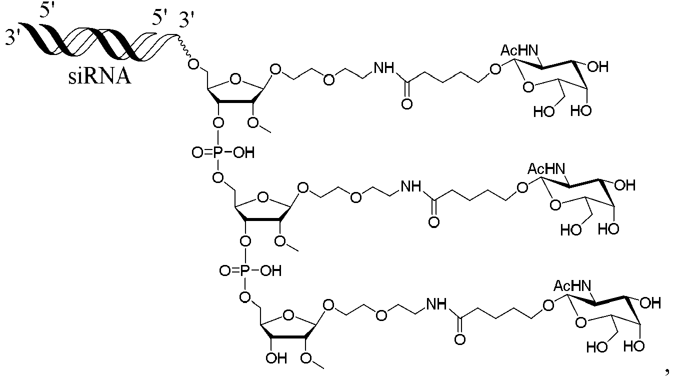

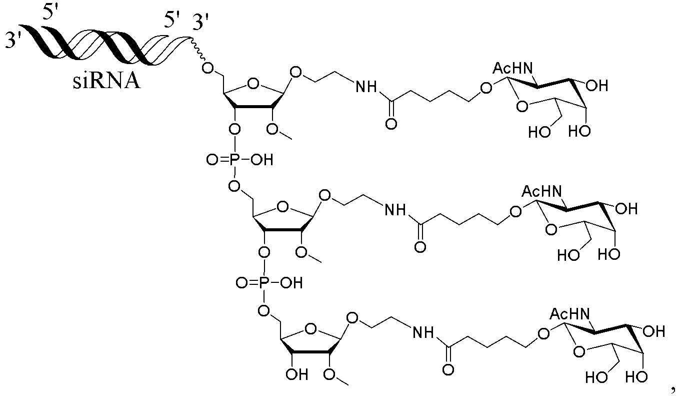

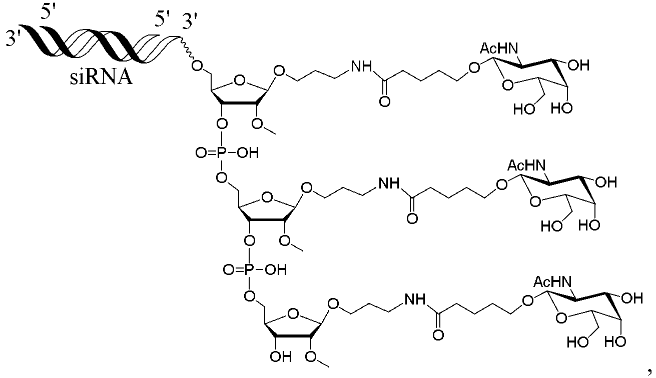

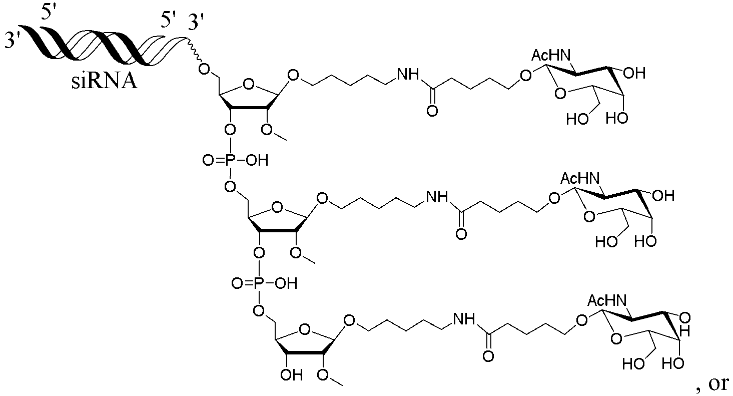

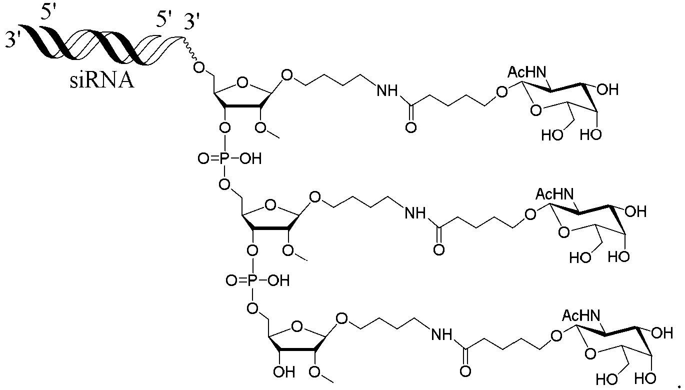

In some embodiments, the siRNA duplex comprises any one oligonucleotide duplex selected from the following pairings of a sense strand and an antisense strand, or a combination of at least two thereof:
(1) the sense strand has the sequence of SEQ ID NO: 21 or a fragment thereof, or a modified sequence of the sequence or the fragment thereof; and the antisense strand has the sequence of SEQ ID NO: 214 or a fragment thereof, or a modified sequence of the sequence or the fragment thereof; wherein the antisense strand is modified according to modification pattern B, and the sense strand is modified according to modification pattern a;
(2) the sense strand has the sequence of SEQ ID NO: 1 or a fragment thereof, or a modified sequence of the sequence or the fragment thereof; and the antisense strand has the sequence of SEQ ID NO: 194 or a fragment thereof, or a modified sequence of the sequence or the fragment thereof; wherein the antisense strand is modified according to modification pattern A, and the sense strand is modified according to modification pattern a;
(3) the sense strand has the sequence of SEQ ID NO: 39 or a fragment thereof, or a modified sequence of the sequence or the fragment thereof; and the antisense strand has the sequence of SEQ ID NO: 232 or a fragment thereof, or a modified sequence of the sequence or the fragment thereof; wherein the antisense strand is modified according to modification pattern C', and the sense strand is modified according to modification pattern a';
(4) the sense strand has the sequence of SEQ ID NO: 44 or a fragment thereof, or a modified sequence of the sequence or the fragment thereof; and the antisense strand has the sequence of SEQ ID NO: 237 or a fragment thereof, or a modified sequence of the sequence or the fragment thereof; wherein the antisense strand is modified according to modification pattern C', and the sense strand is modified according to modification pattern b';
(5) the sense strand has the sequence of SEQ ID NO: 76 or a fragment thereof, or a modified sequence of the sequence or the fragment thereof; and the antisense strand has the sequence of SEQ ID NO: 269 or a fragment thereof, or a modified sequence of the sequence or the fragment thereof; wherein the antisense strand is modified according to modification pattern E, and the sense strand is modified according to modification pattern b;
(6) the sense strand has the sequence of SEQ ID NO: 132 or a fragment thereof, or a modified sequence of the sequence or the fragment thereof; and the antisense strand has the sequence of SEQ ID NO: 325 or a fragment thereof, or a modified sequence of the sequence or the fragment thereof; wherein the antisense strand is modified according to modification pattern B, and the sense strand is modified according to modification pattern a;
(7) the sense strand has the sequence of SEQ ID NO: 184 or a fragment thereof, or a modified sequence of the sequence or the fragment thereof; and the antisense strand has the sequence of SEQ ID NO: 377 or a fragment thereof, or a modified sequence of the sequence or the fragment thereof; wherein the antisense strand is modified according to modification pattern C, and the sense strand is modified according to modification pattern a;
(8) the sense strand has the sequence of SEQ ID NO: 186 or a fragment thereof, or a modified sequence of the sequence or the fragment thereof; and the antisense strand has the sequence of SEQ ID NO: 379 or a fragment thereof, or a modified sequence of the sequence or the fragment thereof; wherein the antisense strand is modified according to modification pattern E, and the sense strand is modified according to modification pattern b;
(9) the sense strand has the sequence of SEQ ID NO: 21 or a fragment thereof, or a modified sequence of the sequence or the fragment thereof; and the antisense strand has the sequence of SEQ ID NO: 214 or a fragment thereof, or a modified sequence of the sequence or the fragment thereof; wherein the antisense strand is modified according to modification pattern A, and the sense strand is modified according to modification pattern a; nucleotides at positions 5 and 7 of the antisense strand are replaced with DNA, and the nucleotide at the position of the sense strand that is complementary to position 7 of the antisense strand is also replaced with DNA;
(10) the sense strand has the sequence of SEQ ID NO: 184 or a fragment thereof, or a modified sequence of the sequence or the fragment thereof; and the antisense strand has the sequence of SEQ ID NO: 377 or a fragment thereof, or a modified sequence of the sequence or the fragment thereof; wherein the antisense strand is modified according to modification pattern C, and the sense strand is modified according to modification pattern a; nucleotides at positions 5 and 7 of the antisense strand are replaced with DNA, and the nucleotide at the position of the sense strand that is complementary to position 7 of the antisense strand is also replaced with DNA;
(11) the sense strand has the sequence of SEQ ID NO: 76 or a fragment thereof, or a modified sequence of the sequence or the fragment thereof; and the antisense strand has the sequence of SEQ ID NO: 269 or a fragment thereof, or a modified sequence of the sequence or the fragment thereof; wherein the antisense strand is modified according to modification pattern E, and the sense strand is modified according to modification pattern b; nucleotides at positions 5 and 7 of the antisense strand are replaced with DNA, and the nucleotide at the position of the sense strand that is complementary to position 7 of the antisense strand is also replaced with DNA;
   wherein either or both of the sense strand and the antisense strand may be linked to the conjugate moiety G4, G5, G6, G7, G101, G102, G103, G105, or G106.

In some embodiments, either or both of the sense strand and the antisense strand are linked to the conjugate moiety G101.

In some embodiments, the conjugate moiety is attached to the 3' end of the sense strand.

The present disclosure also provides a nucleic acid-protein complex comprising a duplex region of the double-stranded RNAi agent or siRNA conjugate described above or an antisense strand of the duplex region, and a nuclease.

In the present disclosure, the term "nucleic acid-protein complex" refers to an RNA-induced silencing complex (RISC) formed by binding of siRNA to Argonaute protein (AGO). The siRNA duplex is then unwound into a sense strand and an antisense strand. The sense strand is degraded, while the antisense strand (guide strand) directs the RISC to bind to a target mRNA homologous to the siRNA via base pairing. The RISC possesses nuclease activity, and the siRNA-guided RISC cleaves the homologous single-stranded mRNA, resulting in loss of mRNA function, *i.e.,* preventing translation into protein and thereby achieving gene "silencing".

The present disclosure also provides a recombinant vector comprising a nucleic acid molecule encoding the siRNA described herein.

In some embodiments, the vector backbone of the recombinant vector is selected from recombinant virus-derived circular RNA vectors, tRNA scaffolds, rRNA scaffolds, and chimeric tRNA/pre-miRNA vectors.

The present disclosure also provides a recombinant cell comprising the siRNA or recombinant vector described above.

In some embodiments, the recombinant cell is selected from *Rhodovulum sulfidophilum* and ribonuclease III-deficient *Corynebacterium glutamicum.*

As used herein, the term "recombinant vector" preferably refers to a vector comprising a regulatory sequence operably linked to a nucleotide sequence encoding the sense strand contained in the nucleic acid molecule of the present disclosure. The term "recombinant cell" refers to a cell into which at least one recombinant vector capable of expressing the nucleic acid molecule or at least one strand of the nucleic acid molecule has been introduced.

The present disclosure also provides a method of preparing the siRNA described herein, comprising culturing the recombinant cell described above, or directly obtaining the siRNA by means of chemical synthesis and mixing.

The present disclosure also provides a pharmaceutical composition comprising the siRNA duplex or the corresponding siRNA conjugate, and a pharmaceutically acceptable carrier.

In one example, provided herein is a pharmaceutical composition comprising the siRNA duplex described herein and a pharmaceutically acceptable carrier. The iRNA-containing pharmaceutical composition may be used for treating or preventing a disease or disorder associated with the expression or activity of the LPA gene, such as atherosclerosis. Such pharmaceutical compositions are formulated based on the mode of delivery. One example is a composition formulated for systemic administration via parenteral delivery, *e.g*., via subcutaneous (s.c.) injection. Another example is a composition formulated for direct delivery into the brain parenchyma, *e.g*., via infusion into the brain, such as by continuous pump infusion.

The pharmaceutical composition comprising the RNAi agent of the present disclosure may be, for example, a solution with or without a buffer, or a composition comprising a pharmaceutically acceptable carrier. Such compositions include, for example, aqueous or crystalline compositions, liposomal formulations, micellar formulations, emulsions, and gene therapy vectors.

In the method of the present disclosure, the siRNA may be administered in a solution. A free siRNA may be administered in an unbuffered solution, *e.g*., in saline or in water. Alternatively, the free siRNA may also be administered in a suitable buffered solution. The buffered solution may comprise acetate, citrate, prolamin, carbonate, or phosphate, or any combination thereof. In a preferred example, the buffered solution is phosphate-buffered saline (PBS). The pH and osmolarity of the buffered solution comprising the siRNA may be adjusted such that it is suitable for administration to a subject.

In some examples, the buffered solution further comprises an agent for controlling the osmolarity of the solution, such that the osmolarity is maintained at a desired value, *e.g*., at the physiological value of human plasma. Solutes that may be added to the buffered solution to control the osmolarity include, but are not limited to, proteins, peptides, amino acids, non-metabolized polymers, vitamins, ions, sugars, metabolites, organic acids, lipids, or salts. In some examples, the agent for controlling the osmolarity of the solution is a salt. In certain examples, the agent for controlling the osmolarity of the solution is sodium chloride or potassium chloride.

The pharmaceutical composition of the present disclosure may be administered at a dose sufficient to inhibit LPA gene expression. Generally, suitable doses of the siRNA of the present disclosure range from about 0.001 to about 200.0 mg/kg body weight per day, typically from about 1 to 50 mg/kg body weight per day. For example, the siRNA (*e.g*., siRNA conjugate) may be administered at a single dose of about 0.01, 0.05, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 31, 32, 33, 34, 34, 35, 36, 37, 38, 39, 40, 41, 42, 43, 44, 45, 46, 47, 48, 49, or about 50 mg/kg.

The pharmaceutical composition may be administered once daily, or multiple times at various intervals ranging from 1 to 365 days, or the siRNA may be administered as two, three, or more sub-doses at appropriate intervals within a year, or even using continuous infusion or delivery via a controlled-release formulation. In such cases, the amount of siRNA in each sub-dose must be correspondingly reduced in order to achieve the total daily dose. Dosage units may also be compounded for delivery over several days, *e.g*., using a conventional sustained-release formulation that provides sustained release of siRNA over a period of several days. Sustained-release formulations are well known in the art and are particularly useful for delivering agents to specific sites, and thus may be used with the agents of the present disclosure. In such examples, the dosage unit contains a corresponding plurality of daily doses.

In other examples, a single dose of the pharmaceutical composition may be long-acting, such that subsequent doses are administered at intervals of no more than 3, 4, or 5 days, or no more than 1, 2, 3, or 4 weeks. In some examples of the present disclosure, a single dose of the pharmaceutical composition of the present disclosure is administered once weekly. In other examples of the present disclosure, a single dose of the pharmaceutical composition of the present disclosure is administered once every two months.

Those skilled in the art will appreciate that certain factors may influence the dosage and dosing schedule required for effective treatment of a subject, including, but not limited to, the severity of the disease or disorder, prior treatments, the general health and/or age of the subject, and other existing diseases. Furthermore, treating a subject with a therapeutically effective amount of the composition may involve a single treatment or a series of treatments. As described elsewhere herein, the effective dose and *in vivo* half-life for each siRNA encompassed by the present disclosure may be estimated using conventional methods or on the basis of *in vivo* testing using suitable animal models.

The pharmaceutical composition of the present disclosure may be administered in various ways depending upon whether local or systemic treatment is desired and upon the area to be treated. Administration may be topical *(e.g.,* via a transdermal patch); pulmonary; *e.g.,* via inhalation or insufflation of a powder or aerosol, including via a nebulizer; intratracheal; intranasal; epidermal and transdermal, oral, or parenteral. Parenteral administration includes intravenous, intra-arterial, subcutaneous, intraperitoneal, or intramuscular injection or infusion; subdermal administration, *e.g.,* via an implantable device; or intracranial administration, *e.g.,* intraparenchymal, intrathecal, or intraventricular administration.

The siRNA for use in the compositions and methods of the present disclosure may be formulated for delivery in a membranous molecular assemblies, such as liposomes or micelles. As used herein, the term "liposome" refers to a vesicle composed of amphipathic lipids arranged in at least one lipid bilayer *(e.g.,* one bilayer or a plurality of bilayers), having an external membrane formed by lipophilic materials and an internal aqueous compartment. The lipophilic material separates the internal aqueous compartment from the external aqueous environment, which typically does not contain the siRNA composition (although in some instances it may). Liposomes are useful for transferring and delivering active ingredients to sites of action. Because liposomal membranes are structurally similar to biological membranes, when liposomes are applied to a tissue, the liposomal bilayer fuses with the bilayer of the cell membrane. As the fusion of liposomes with cells proceeds, the internal aqueous contents, including the siRNA, are delivered into the cell, where the siRNA can specifically bind to a target RNA and mediate RNA interference (RNAi). In some cases, these liposomes are also specifically targeted, *e.g.,* to direct the siRNA to particular cell types.

Liposomes containing siRNA may be prepared by a variety of methods. In one example, a lipid component of the liposome is dissolved in a detergent such that micelles are formed with the lipid component. For example, the lipid component may be an amphipathic cationic lipid or a lipid conjugate. The detergent may have a high critical micelle concentration and may be nonionic. Exemplary detergents include cholate, CHAPS, octyl glucoside, deoxycholate, and lauroyl sarcosinate. The siRNA formulation is then added to the micelles containing the lipid component. The cationic groups on the lipid interact with the siRNA and condense around the siRNA to form liposomes. After condensation, the detergent is removed, *e.g*., by dialysis, to obtain the corresponding liposomal formulation of the siRNA.

The siRNA, such as the RNA duplex of the present disclosure, may be encapsulated in a lipid formulation (*e.g*., a lipid nanoparticle (LNP) or another nucleic acid-lipid particle).

As used herein, the term "LNP" refers to a stable nucleic acid-lipid particle. The LNP comprises a cationic lipid, a non-cationic lipid, and a lipid that prevents aggregation of the particles (*e.g.,* a PEG-lipid conjugate). LNPs are extremely useful for synthetic applications, as they exhibit a prolonged circulation lifetime following intravenous (i.v.) injection and accumulate at distal sites (*e.g*., sites physically separated from the site of administration).

In one example, the mass ratio of lipid to siRNA duplex is about 1:1 to about 50:1, about 1:1 to about 25:1, about 3:1 to about 15:1, about 4:1 to about 10:1, about 5:1 to about 9:1, or about 6:1 to about 9:1.

In some preferred embodiments, the lipid nanoparticle comprise a cationic lipid, a neutral lipid, a structured lipid, and a polymer-conjugated lipid.

In some preferred embodiments, the cationic lipid is a compound having the structure of formula (I), or an N-oxide, a solvate, a pharmaceutically acceptable salt, or a stereoisomer thereof, wherein G₁ is C₁₋₆ alkylene; G₂ is C₂₋₈ alkylene; G₃ is C₁₋₃ alkylene; L₁ is C₆₋₁₅ linear alkyl; L₂ is C₁₂₋₂₅ branched alkyl; for example, YK-009 having the structure of formula (I-I) (see patent CN114044741B, the contents of which are incorporated herein by reference in their entirety, particularly including the general formulas and specific compounds described therein).

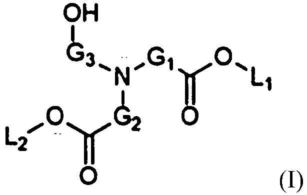

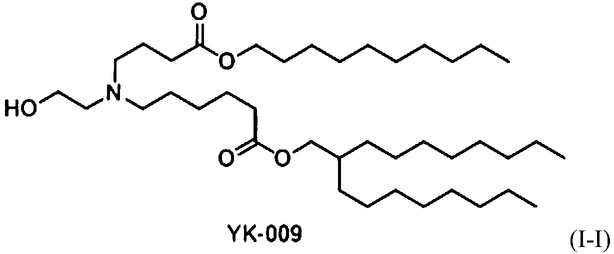

In some preferred embodiments, the cationic lipid is a compound having the structure of formula (II), or an N-oxide, a solvate, a pharmaceutically acceptable salt, or a stereoisomer thereof, wherein G₁ is C₂₋₈ alkylene; G₂ is C₂₋₈ alkylene; L₁ is -C(O)O- or -OC(O)-; L₂ is - C(O)O- or -OC(O)-; R₁ is C₆₋₂₅ linear or branched alkyl; R₂ is C₆₋₂₅ linear or branched alkyl; G₃ is HO(CH₂)₂- or HO(CH₂)₃-; G₄ is HO(CH₂)₂- or HO(CH₂)₃-; L is (CH₂)₂-, -(CH₂)₃-, or - (CH₂)₄-; for example, YK-401 having the structure of formula (II-I), YK-402 having the structure of formula (II-II), or YK-407 having the structure of formula (II-III) (see patent CN115784921B, the contents of which are incorporated herein by reference in their entirety, particularly including the general formulas and specific compounds described therein).

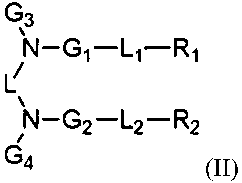

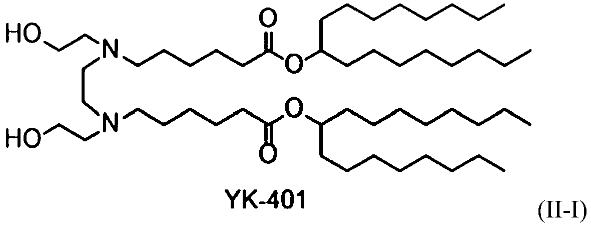

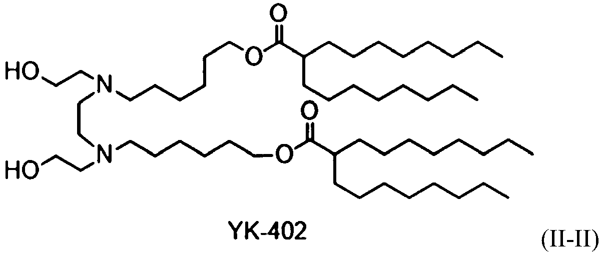

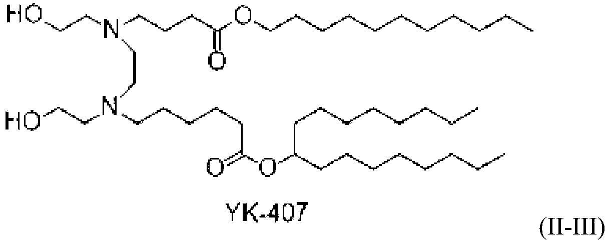

In some preferred embodiments, the cationic lipid is a compound having the structure of formula (III), or an N-oxide, a solvate, a pharmaceutically acceptable salt, or a stereoisomer thereof, wherein G₁ is C₁₋₆ alkylene; G₂ is C₂₋₈ alkylene; R₁ is C₆₋₂₀ linear or branched alkyl; R₂ is C₁₂₋₂₅ branched alkyl; G₃ is HO(CH₂)₂N(CH₃)(CH₂)₂-, HO(CH₂)₂N(CH₂CH₃)(CH₂)₂-, (HO(CH₂)₂)₂N(CH₂)₂-, CH₃O(CH₂)₂N(CH₃)(CH₂)₂-, (CH₃)₂N(CH₂)₃SC(O)O(CH₂)₂-, (CH₃)₂N(CH₂)₃SC(O)-, CH₃NH(CH₂)₂N(CH₃)(CH₂)₂-, or CH₃CH₂NH(CH₂)₂-; for example, YK-201 having the structure of formula (III-I) or YK-202 having the structure of formula (III-II) (see patent CN115677518B, the contents of which are incorporated herein by reference in their entirety, particularly including the general formulas and specific compounds described therein).

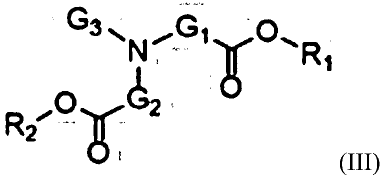

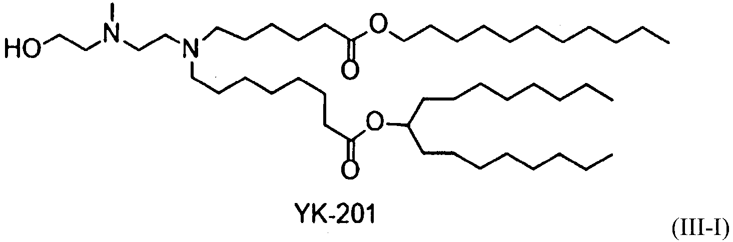

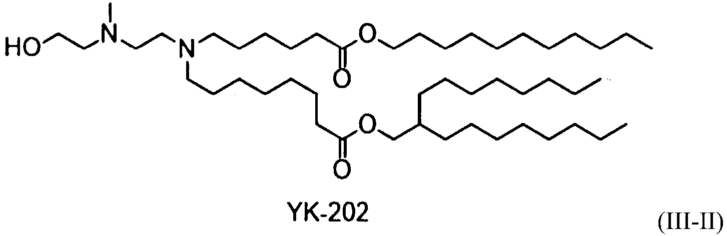

In some preferred embodiments, the cationic lipid is a compound having the structure of formula (IV), or an N-oxide, a solvate, a pharmaceutically acceptable salt, or a stereoisomer thereof, wherein G₁ is C₁₋₈ alkylene; G₂ is C₂₋₈ alkylene; R₁ is C₆₋₂₅ linear or branched alkyl; R₂ is C₁₂₋₂₅ linear or branched alkyl; G₃ is HO(CH₂)₂N(R₃)CH₂CH(OH)CH₂-, wherein R₃ is - CH₃, -CH₂CH₃, or -CH₂CH₂OH; for example, YK-305 having the structure of formula (IV-I) or YK-310 having the structure of formula (IV-II) (see patent CN115745820B, the contents of which are incorporated herein by reference in their entirety, particularly including the general formulas and specific compounds described therein).

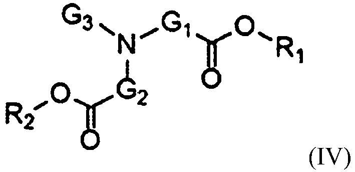

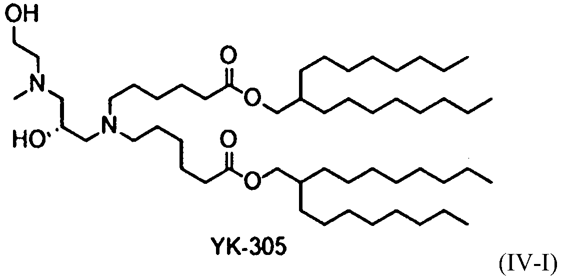

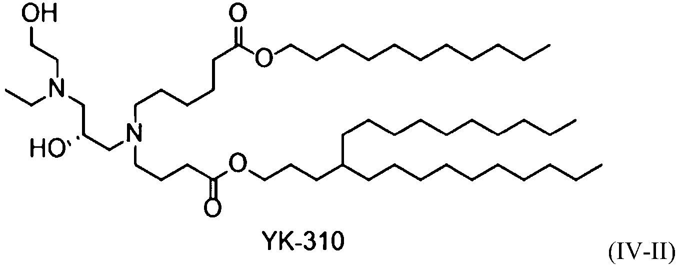

In some preferred embodiments, the cationic lipid is a compound having the structure of formula (V), or an N-oxide, a solvate, a pharmaceutically acceptable salt, or a stereoisomer thereof, wherein G¹ and G² are each independently unsubstituted C₆-C₁₀ alkylene; G₃ is unsubstituted C₁-C₁₂ alkylene; R¹ and R² are each independently C₆-C₂₄ alkyl or C₆-C₂₄ alkenyl; R³ is OR⁵, N, -C(=O)OR⁴, -OC(=O)R⁴, or -NR⁵C(=O)R⁴; R⁴ is C₁-C₁₂ hydrocarbyl; and R⁵ is H or C₁-C₆ hydrocarbyl; for example, ALC0315 having the structure of formula (V-I) (see patent CN108368028B, the contents of which are incorporated herein by reference in their entirety, particularly including the general formulas and specific compounds described therein).

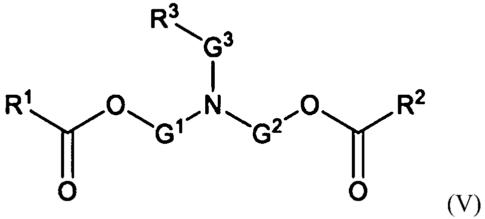

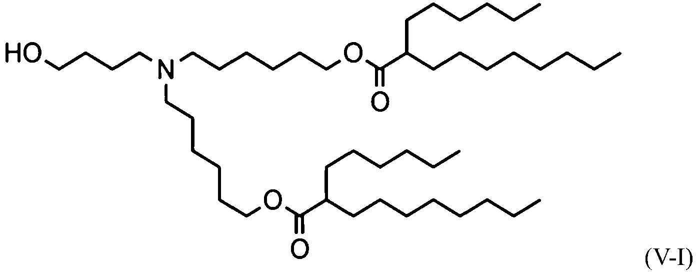

In some preferred embodiments, the cationic lipid is a compound having the structure of formula (VI), or an N-oxide, a solvate, a pharmaceutically acceptable salt, or a stereoisomer thereof, wherein R₄ is selected from -(CH₂)ₙQ and -(CH₂)ₙCHQR; Q is selected from the group consisting of -OR, -OH, -O(CH₂)ₙN(R)₂, -OC(O)R, -CX₃, -CN, -N(R)C(O)R, -N(H)C(O)R, - N(R)S(O)₂R, -N(H)S(O)₂R, -N(R)C(O)N(R)₂, -N(H)C(O)N(R)₂, -N(H)C(O)N(H)(R), - N(R)C(S)N(R)₂, -N(H)C(S)N(R)₂, -N(H)C(S)N(H)(R), -N(R)S(O)₂R₈, and heterocycle; n is 1, 2, or 3; for example, SM102 having the structure of formula (VI-I) (see patent CN110520409A, the contents of which are incorporated herein by reference in their entirety, particularly including the general formulas and specific compounds described therein).

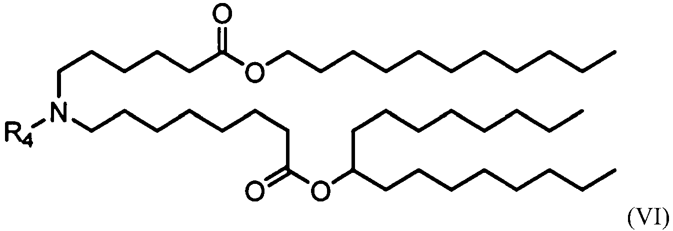

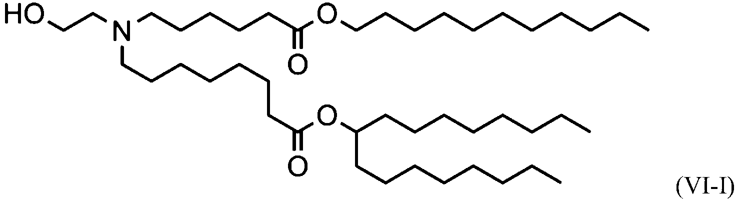

In some preferred embodiments, the cationic lipid is a compound having the structure of formula (VII), or an N-oxide, a solvate, a pharmaceutically acceptable salt, or a stereoisomer thereof (see patent CN102625696B, DLIN-MC3-DMA, the contents of which are incorporated herein by reference in their entirety, particularly including the general formulas and specific compounds described therein).

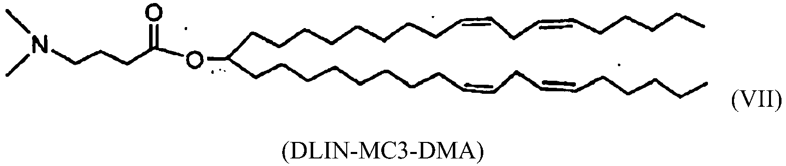

In some more preferred embodiments, the cationic lipid is any one selected from the group consisting of YK-009, YK-407, YK-305, ALC0315, SM102, and DLIN-MC3-DMA, or a combination of at least two thereof.

In some preferred embodiments, the molar ratio of the cationic lipid to the neutral lipid is 1:1 to 10:1.

In some preferred embodiments, the molar ratio of the cationic lipid to the structured lipid is 1:1 to 5:1.

In some preferred embodiments, the molar ratio of the cationic lipid, the neutral lipid, the structured lipid, and the polymer-conjugated lipid is (25 to 65):(5 to 25):(25 to 70):(0.5 to 5).

In some preferred embodiments, the molar ratio of the cationic lipid, the neutral lipid, the structured lipid, and the polymer-conjugated lipid is (25 to 65):(5 to 25):(25 to 45):(0.5 to 5).

In some more preferred embodiments, the molar ratio of the cationic lipid, the neutral lipid, the structured lipid, and the polymer-conjugated lipid is 50:10:38.5:1.5 or 49:10:39.5:1.5.

In some preferred embodiments, the neutral lipid is any one selected from the group consisting of phosphatidylcholine, phosphatidylethanolamine, sphingomyelin, ceramide, sterols, and derivatives thereof, or a combination of at least two thereof.

In some more preferred embodiments, the neutral lipid is any one selected from the group consisting of 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoyl phosphatidylglycerol (DPPG), palmitoyl oleoyl phosphatidylethanolamine (POPE), distearoyl-phosphatidyl-ethanolamine (DSPE), dipalmitoyl phosphatidylethanolamine (DPPE), dimyristoyl phosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, lysophosphatidylethanolamine (LPE), and mixtures thereof, or a combination of at least two thereof.

In some more preferred embodiments, the neutral lipid is DOPE and/or DSPC.

In some preferred embodiments, the structured lipid is any one selected from the group consisting of cholesterol, nonsterols, sitosterol, ergosterol, campesterol, stigmasterol, brassinosterol, tomatidine, ursolic acid, α-tocopherol, and corticosteroids, or a combination of at least two thereof.

In some more preferred embodiments, the structured lipid is cholesterol.

In some preferred embodiments, the polymer-conjugated lipid is any one selected from the group consisting of PEG-modified phosphatidylethanolamine, PEG-modified phosphatidic acid, PEG-modified ceramide, PEG-modified dialkylamine, PEG-modified diacylglycerol, and PEG-modified dialkylglycerol, or a combination of at least two thereof.

In some more preferred embodiments, the polymer-conjugated lipid is any one selected from the group consisting of distearoyl phosphatidylethanolamine polyethylene glycol 2000 (DSPE-PEG2000), dimyristoylglycero-3-methoxypolyethylene glycol 2000 (DMG-PEG2000), and methoxypolyethylene glycol ditetradecylacetamide (ALC-0159), or a combination of at least two thereof.

Examples of pharmaceutical compositions of the present disclosure include, but are not limited to, aqueous formulations, emulsion formulations, and liposome-containing formulations. These compositions may be derived from a variety of components, examples of which include, but are not limited to, preformed liquids, self-emulsifying solids, and self-emulsifying semisolids. For example, formulations targeting the liver are preferred when treating hepatic disorders (*e.g*., hepatocellular carcinoma).

The pharmaceutical formulations of the present disclosure (which may conveniently be presented in unit dosage forms) may be prepared using conventional techniques well known in the pharmaceutical industry. Such techniques include the step of bringing into association the active ingredients with the pharmaceutical carrier(s) or excipient(s). Generally, the formulations are prepared by uniformly and intimately bringing into association the active ingredients with a liquid carrier or a finely divided solid carrier or both, and then, if necessary, shaping the product.

The compositions of the present disclosure may be formulated into any of a variety of possible dosage forms, including, but not limited to, tablets, capsules, gelatin capsules, liquid syrups, soft capsules, suppositories, and enemas. The compositions of the present disclosure may also be formulated as suspensions in aqueous, non-aqueous, or mixed media. Aqueous suspensions may further comprise substances that increase the viscosity of the suspension, including, for example, sodium carboxymethylcellulose, sorbitol, and/or dextran. The suspension may also comprise a stabilizer.

Certain compositions of the present disclosure further incorporate a carrier compound into the formulation. As used herein, the term "carrier compound" or "carrier" may refer to a nucleic acid or analog thereof that is inert (*i.e.,* lacks biological activity per se) but is recognized as a nucleic acid during *in vivo* processes that reduce the bioavailability of a biologically active nucleic acid, for example by degrading the biologically active nucleic acid or facilitating its removal from circulation. Co-administration of a nucleic acid and a carrier compound (typically the latter in excess) may result in a substantial reduction in the amount of nucleic acid recovered in the liver, kidney, or other extracirculatory reservoirs, presumably due to competition between the carrier compound and the nucleic acid for common receptors. For example, when co-administered with polyinosinic acid, dextran sulfate, polycytidylic acid, or 4-acetamido-4'-isothiocyano-stilbene-2,2'-disulfonic acid, the recovery of partially phosphorothioated dsRNA in hepatic tissue may be reduced (Miyao et al., DsRNA Res. Dev., 1995, 5: 115-121; Takakura et al., DsRNA & Nucl. Acid Drug Dev., 1996, 6: 177-183).

In contrast to carrier compounds, a "pharmaceutical carrier" or "excipient" refers to a pharmaceutically acceptable solvent, suspending agent, or any other pharmaceutically inert vehicle for delivering one or more nucleic acids to an animal. The excipient may be liquid or solid and is selected with reference to the intended mode of administration in order to provide the desired bulk, consistency, and other properties, when combined with the nucleic acid and other components of a particular pharmaceutical composition Typical pharmaceutical carriers include, but are not limited to, binders (*e.g*., pregelatinized corn starch, polyvinylpyrrolidone, or hydroxypropyl methylcellulose); fillers (*e.g.,* lactose and other sugars, microcrystalline cellulose, pectin, gelatin, calcium sulfate, ethyl cellulose, polyacrylates, or calcium hydrogen phosphate); lubricants (*e.g*., magnesium stearate, talc, silica, colloidal silicon dioxide, stearic acid, metallic stearate, hydrogenated vegetable oil, corn starch, polyethylene glycol, sodium benzoate, or sodium acetate); disintegrants (*e.g*., starch or sodium starch glycolate); and wetting agents (*e.g.*, sodium lauryl sulfate).

Pharmaceutically acceptable organic or inorganic excipients suitable for non-parenteral administration that do not deleteriously react with nucleic acids may also be used to formulate the compositions of the present disclosure. Suitable pharmaceutically acceptable carriers include, but are not limited to, water, saline solutions, alcohols, polyethylene glycol, gelatin, lactose, amylose, magnesium stearate, talc, silicic acid, viscous paraffin, hydroxymethyl cellulose, and polyvinylpyrrolidone.

Formulations suitable for topical administration of nucleic acids may include sterile or non-sterile aqueous solutions or non-aqueous solutions in common solvents such as alcohols, or nucleic acid solutions in liquid or solid oil bases. These solutions may further include buffers, diluents, and other suitable additives. Pharmaceutically acceptable organic or inorganic excipients suitable for non-parenteral administration that do not deleteriously react with nucleic acids may be used.

Suitable pharmaceutically acceptable excipients include, but are not limited to, water, saline solutions, alcohols, polyethylene glycol, gelatin, lactose, amylose, magnesium stearate, talc, silicic acid, viscous paraffin, hydroxymethyl cellulose, and polyvinylpyrrolidone.

The present disclosure also provides methods of treating or preventing diseases and disorders that can be modulated by downregulating the LPA gene expression. Examples include Berger's disease, peripheral arterial disease, coronary artery disease, metabolic syndrome, acute coronary syndrome, aortic stenosis, aortic regurgitation, aortic dissection, retinal artery occlusion, cerebrovascular disease, mesenteric ischemia, superior mesenteric artery occlusion, renal artery stenosis, stable or unstable angina, acute coronary syndrome, heterozygous or homozygous familial hypercholesterolemia, hyperapobetalipoproteinemia, cerebrovascular atherosclerosis, cerebrovascular disease and venous thrombosis, stroke, atherosclerosis, thrombosis, coronary heart disease or aortic stenosis, and/or any other disease associated with elevated levels of Lp(a) particles, as well as other unidentified related disorders, pathologies, or syndromes.

The siRNA of the present disclosure may be administered to a subject using any route of administration known in the art, including, but not limited to, subcutaneous, intravenous, intramuscular, intraocular, intrabronchial, intrapleural, intraperitoneal, intra-arterial, lymphatic, or cerebrospinal administration, and any combination thereof. In a preferred example, the agents are administered subcutaneously.

In another example, the siRNA is administered in combination with an additional therapeutic agent. The siRNA and the additional therapeutic agent may be co-administered in the same composition, e.g., parenterally, or the additional therapeutic agent may be administered as part of a separate composition or by another method described herein.

Examples of additional therapeutic agents include agents known for treating LPA-associated diseases or disorders. For example, additional therapeutic agents include: administering to a subject one or more siRNAs of the present disclosure; administering to a subject a non-LPA RNAi therapeutic agent; and implementing behavioral modifications in a subject. In some embodiments, the non-LPA RNAi therapeutic agent is one of the following additional therapeutic agents: HMG Co-A reductase inhibitors (statins), ezetimibe, PCSK-9 inhibitors, CTEP inhibitors, therapies targeting ANGPTL3, therapies targeting APOC3, and niacin, or any combination thereof.

In one example, the iRNA agent is administered to a patient, followed by administration of the additional therapeutic agent (or vice versa). In another example, the iRNA agent and the additional therapeutic agent are administered simultaneously.

The abbreviations for nucleotides used herein are as follows:
A = adenosine-3'-phosphate
Am = 2'-O-methyladenosine-3'-phosphate
Ams = 2'-O-methyladenosine-3'-phosphorothioate
Af = 2'-fluoroadenosine-3'-phosphate
Afs = 2'-fluoroadenosine-3'-phosphorothioate
G = guanosine-3'-phosphate
Gm = 2'-O-methylguanosine-3'-phosphate
Gms = 2'-O-methylguanosine-3'-phosphorothioate
Gf = 2'-fluoroguanosine-3'-phosphate
Gfs = 2'-fluoroguanosine-3'-phosphorothioate
C = cytidine-3'-phosphate
Cm = 2'-O-methylcytidine-3'-phosphate
Cms = 2'-O-methylcytidine-3'-phosphorothioate
Cf = 2'-fluorocytidine-3'-phosphate
Cfs = 2'-fluorocytidine-3'-phosphorothioate
U = uridine-3'-phosphate
Um = 2'-O-methyluridine-3'-phosphate
Ums = 2'-O-methyluridine-3'-phosphorothioate
Uf = 2'-fluorouridine-3'-phosphate
Ufs = 2'-fluorouridine-3'-phosphorothioate
AmsEVP = 5'-vinyl-(E)-phosphonate-2'-O-methyladenosine-3'-phosphorothioate
UmsEVP = 5'-vinyl-(E)-phosphonate-2'-O-methyluridine-3'-phosphorothioate
Agna = adenosine-glycol nucleic acid
Cgna = cytidine-glycol nucleic acid
Ggna = guanosine-glycol nucleic acid
Tgna = thymidine-glycol nucleic acid
Ugna = uridine-glycol nucleic acid

The coding rules for siRNA motifs (or motifs), siRNA derivatives, and siRNA conjugates used herein are illustrated as follows:
Sequences with alternating 2'-O-methyl (2'-OMe) and 2'-fluoro (2'-F) modifications are designated by adding the suffix "-AL" after the motif code. For example, the code for the alternately modified B01023 is B01023-AL.

The siRNA derivatives such as those in Examples 4 and 6 involve the use of the modification templates DV25P to DV27P, DV29P, DV32P, DV34P, DV38P, DV25SP to DV27SP, DV29SP, DV32SP, DV34SP, and DV38SP disclosed herein. The corresponding coding rule involves changing the first letter of the motif code for the siRNA derivative from "B" to "C", followed by addition of the name of the corresponding template for the siRNA derivative after the code. For example, for motif B01023, a derivative obtained using the template DV25P is designated as C01023-DV25P.

The siRNA derivatives/siRNA conjugates such as those in Examples 7 to 10 involve the use of the modification templates DV25P to DV27P, DV29P, DV32P, DV34P, DV38P, DV25SP to DV27SP, DV29SP, DV32SP, DV34SP, and DV38SP disclosed herein, and may further incorporate anti-off-target modifications, with a conjugate moiety attached to the 3' end of the sense strand. The coding rule involves changing the first letter of the motif code from "B" to "D," followed by sequential addition of the names of the corresponding template and/or anti-off-target modifications after the code, and concluding with the name of the conjugate moiety such as "G103" or "G101". For example, the siRNA motif B01023, which is modified using the template DV25P and incorporating the anti-off-target modification d7B5 along with attachment of the conjugate moiety G101, is designated as D01023-DV25Pd7B5G101.

**Table I: Examples of codes for siRNA duplexes (motifs, derivatives, and conjugates) in the present disclosure**

| Type of siRNA duplex | siRNA motif | Alternately modified siRNA derivative | Template-modified siRNA derivative | Template-modified and anti-off-target modified siRNA derivative | Template-modified and anti-off-target modified siRNA conjugate |
|---|---|---|---|---|---|
| Examples of codes | B01023 | B01023-AL | C01023-DV25P | D01023-DV25Pd7B5 | D01023-DV25Pd7B5G101 |

### Examples

In the following examples, for experimental data used in comparison among groups, *P* < 0.05 is considered to indicate a statistically significant difference.

Additional objectives, features, and advantages of the present disclosure will become apparent from the following detailed description. However, it should be understood that the detailed description and specific examples, although indicating specific embodiments of the present disclosure, are provided for illustrative purposes only, since various changes and modifications made within the spirit and scope of the present disclosure will become apparent to those skilled in the art upon reading the detailed description.

Unless otherwise specified, the experimental techniques and methods used in the following examples are conventional methods. For example, experimental methods for which specific conditions are not indicated in the following examples are typically performed under conventional conditions, such as those described in Sambrook et al., Molecular Cloning: A Laboratory Manual (New York: Cold Spring Harbor Laboratory Press, 1989), or according to the conditions recommended by the respective manufacturers. Unless otherwise specified, the materials and reagents used in the examples are commercially available.

Those skilled in the art will appreciate that in various examples of the present disclosure, when the test substance in an experiment is an siRNA conjugate, the experimental data and results (including, but not limited to, inhibition rate, IC₅₀, and IC₄₀) can accordingly reflect the inhibition rate, IC₅₀, IC₄₀, and other parameters of the corresponding siRNA derivative of the siRNA conjugate. There is no obstacle to understanding for those skilled in the art.

### Example 1: Inhibitory effect of siRNA motifs on LPA gene expression

A total of 192 siRNA motifs were designed and synthesized based on the human LPA mRNA sequence (NM_005577.4). The inhibitory effect of each siRNA motif on LPA gene expression was evaluated using a dual-luciferase assay, as shown in Table 2.

### 1.1 Synthesis of siRNA motifs

Instruments and reagents: 192 P DNA/RNA automatic synthesizer (Tsingke), using a universal solid-phase support consisting of cross-linked polystyrene beads; model: Primer Support 5G Unylinker 350 (manufactured by Cytiva).

### Preparation method:

Nucleotide monomers were dissolved in acetonitrile at a concentration of 0.15 M, including DMT-A-2'-O-TBDMS phosphoramidite monomer (Formula 11), DMT-C-2'-O-TBDMS phosphoramidite monomer (Formula 12), DMT-G-2'-O-TBDMS phosphoramidite monomer (Formula 13), and DMT-U-2'-O-TBDMS phosphoramidite monomer (Formula 14).

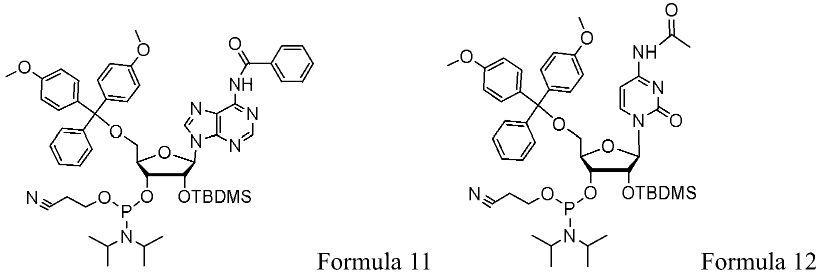

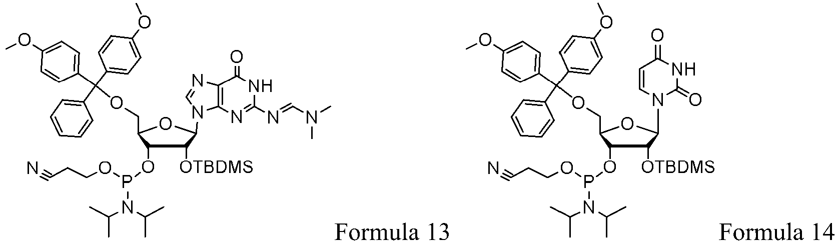

Nucleotide monomers were sequentially attached in the 3' to 5' direction according to the nucleotide arrangement using the solid-phase phosphoramidite method. The process of attaching each nucleotide monomer involves four steps: deprotection, coupling, oxidation or sulfurization, and hydroxyl protection. Specifically, when two nucleotides are linked via a phosphate linkage, the process of attaching a subsequent nucleotide monomer involves four steps: deprotection, coupling, oxidation, and hydroxyl protection. When two nucleotides are linked via a phosphorothioate linkage, the process of attaching a subsequent nucleotide monomer involves four steps: deprotection, coupling, sulfurization, and hydroxyl protection.

The preparation was carried out according to the following steps:
The solid-phase support was loaded into the designated position of the synthesizer, followed by multiple synthesis cycles to obtain the desired product, each cycle consisting of (1) deprotection, (2) coupling, (3) oxidation/sulfurization, and (4) hydroxyl protection. The cycle process and reagents used are described as follows:

### (1) Deprotection

3% dichloroacetic acid in toluene was used as a deprotection reagent to remove the DMT protecting group, followed by washing with acetonitrile.

### (2) Coupling

Each nucleotide monomer in acetonitrile was coupled using 0.25 M 5-ethylthio-1*H-*tetrazole as an activator, followed by washing with acetonitrile.

### (3) Oxidation/sulfurization

Oxidation: 0.05 M iodine in pyridine/water (90/10) was used as an oxidant for oxidation, followed by washing with acetonitrile.

Sulfurization: 3% xanthane hydride in pyridine was used as a sulfurizing agent for sulfurization, followed by washing with acetonitrile.

### (4) Hydroxyl protection

10% acetic anhydride in tetrahydrofuran (CAP A) and tetrahydrofuran/pyridine/N-methylimidazole (74/10/16, v/v/v) (CAP B) were used as hydroxyl protecting reagents for hydroxyl protection, followed by washing with acetonitrile.

The aforementioned steps were repeated according to the specified nucleotide arrangement to obtain a product with the specific sequence arrangement in either the sense strand or the antisense strand.

(5) 3% dichloroacetic acid in toluene was used as a deprotection reagent to remove the DMT protecting group from the last nucleotide, followed by washing with acetonitrile.

### (6) Ammonolysis and purification

The solid-phase support was transferred to a reactor and treated with concentrated aqueous ammonia (25 to 28%) at 60°C for 12 hours. The system was then cooled to room temperature, and the mixture was filtered. The filter cake was washed with a mixture of purified water and ethanol. The filtrates were combined, subjected to column chromatography, concentrated, and lyophilized to obtain a 2'-O-TBDMS-protected product.

### (7) Removal of TBDMS protecting groups

The resulting product was treated with DMSO and triethylamine hydrofluoride at 60°C for 2 hours. An aqueous ammonium acetate solution was then added to the reaction mixture and vortexed thoroughly. The resulting mixture was added with anhydrous ethanol, vortexed thoroughly, followed by crystallization at -20°C for 8 to 12 hours. After centrifugation, the supernatant was discarded, and the precipitate was washed with anhydrous ethanol to obtain an unmodified single-stranded product.

### (8) Annealing

The sense and antisense strands of the resulting siRNA motif were mixed at a molar ratio of 1:1, heated to 95°C and maintained for 3 minutes, and slowly cooled to room temperature to form a double-stranded siRNA motif.

A total of 193 siRNAs listed in Table 2 were synthesized using the method described above, wherein ANC represents a nonsense sequence used as a negative control.

### 1.2 Dual-luciferase assay for evaluation of inhibitory effect of siRNA motifs on LPA gene expression

The experimental materials and methods are as follows:

### 1.2.1 Experimental materials

**Table 1: Experimental materials**

| **Name** | **Brand** | **Cat. No.** |
|---|---|---|
| Huh7 cells | Wuhan Pricella Biotechnology Co., Ltd. | CL-0120 |
| RNAiMAX transfection reagent | Invitrogen | 13778-150 |
| Opti-MEM reduced serum medium | Gibco | 31985-070 |
| Dulbecco's modified Eagle's medium (DMEM) | Macgene | CM10013 |
| Fetal bovine serum (FBS) | Vistech | SE100-011 |
| Fugene-HD transfection reagent | Promega | E2311 |
| Dual-Glo luciferase assay system | Promega | E2940 |

psiCHECK2-LPA plasmid construction: The full-length LPA mRNA sequence (NM_007755.4) was cloned into the dual-luciferase vector psiCHECK^{™}-2 by VectorBuilder (Guangzhou) Co., Ltd. (Cat. No.: VB240227-1779ggq).

### 1.2.2 Experimental methods

### 1) Plasmid transfection and cell seeding

### Day 0: Transfection of the psiCHECK2-target gene plasmid into Huh7 cells

The psiCHECK2-target gene plasmid was diluted to 10 ng/µL with Opti-MEM. Huh7 cells were washed with DPBS, then trypsinized, and adjusted to a density of 1 × 10⁵ cells/mL. Fugene-HD transfection reagent and 10 ng/µL psiCHECK2-target gene plasmid dilution were mixed at a volume ratio of 3:100 and incubated at room temperature for 10 minutes. After incubation, the mixture was added to the Huh7 cells, which were seeded into a 96-well plate at a density of 10,000 cells per well in 100 µL of culture medium. The cells were incubated overnight in an incubator at 37°C with 5% CO₂.

### 2) siRNA preparation and cell transfection

### Day 1: siRNA preparation

RNAiMAX transfection reagent and Opti-MEM were mixed at a volume ratio of 1.5:48.5 to obtain mixture X, which was incubated at room temperature for 15 minutes. siRNA to be tested (with an initial concentration of 12 nM) and mixture X were mixed at a volume ratio of 1:1 to obtain mixture Y, which was incubated at room temperature for 15 minutes. After incubation, 20 µL of mixture Y was added to 100 µL of fresh DMEM at a volume ratio of 1:5 and mixed thoroughly to obtain mixture Z, resulting in a final siRNA transfection concentration of 1 nM. The supernatant was discarded from the 96-well plate incubated overnight in step 1). 120 µL of mixture Z was added to each well of the 96-well plate, followed by incubation in a CO₂ incubator for 48 hours.

### 3) Cell harvesting and luciferase assay

### Day 2/Day 3: Reporter gene assay

### Reagent preparation:

Reagent A: The contents of one bottle of Dual-Glo^{®} Luciferase Buffer were transferred to one bottle of Dual-Glo^{®} Luciferase Substrate to prepare the Dual-Glo^{®} Luciferase Reagent, which should be aliquoted and stored at -80°C in the dark.

Reagent B: The required amount of Dual-Glo^{®} Stop & Glo^{®} Reagent for the experiment was calculated. Using a new container, Dual-Glo^{®} Stop & Glo^{®} Substrate was diluted 1:100 in Dual-Glo^{®} Stop & Glo^{®} Buffer to prepare the desired volume of Dual-Glo^{®} Stop & Glo^{®} Reagent, which should be freshly prepared and used immediately.

### Sample addition and measurement:

The cell culture supernatant was removed using a pipette. 75 µL of fresh DMEM containing 10% FBS was added to each well, and the plate was equilibrated to room temperature. 75 µL of Reagent A was then added to each well, and the plate was shaken at room temperature for 10 minutes to lyse the cells.

After complete cell lysis was confirmed under a microscope, the luminescence of Firefly luciferase (Firefly Lum) was measured using a microplate reader. The instrument parameters were set as follows: chemiluminescence (Lum) detection mode, full wavelength, integration time of 1 second, and detection height of 1 mm.

After completion of the measurement, 75 µL of Reagent B was added to each well of the plate from the previous step. The plate was shaken at room temperature for 10 minutes, and the luminescence of Renilla luciferase (Renilla Lum) was measured. The instrument parameters were set as follows: chemiluminescence (Lum) detection mode, full wavelength, integration time of 1 second, and detection height of 1 mm.

In the experiment, a control group was included in which Opti-MEM was used instead of the siRNA described above, with all other conditions identical to those of the experimental group; a blank group was also included, consisting of Huh7 cells that were not transfected with the psiCHECK2-target gene plasmid and were not treated with siRNA.

### 4) Calculation of inhibition rate of siRNA on target gene expression

The ratio of Renilla Lum to Firefly Lum was denoted as α, and was calculated according to the following formula: 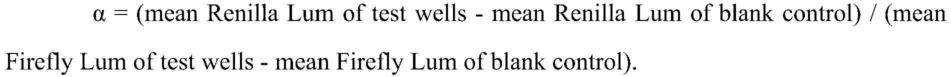 α = (mean Renilla Lum of test wells - mean Renilla Lum of blank control)/(mean Firefly Lum of test wells - mean Firefly Lum of blank control).

Based on the above formula, the ratio calculated for the experimental group was denoted as α (experimental group), and the ratio calculated for the control group was denoted as α (transfection reagent control group).

The inhibition rate of siRNA on target gene expression was calculated according to the following formula: 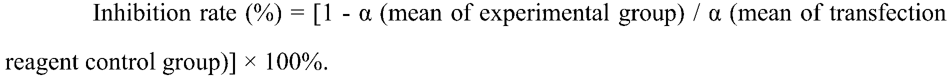 Inhibition rate (%) = [1 - α (mean of experimental group)/α (mean of transfection reagent control group)] × 100%.

Three independent experiments were performed in triplicate. The inhibition rates of the siRNA motifs on LPA mRNA expression are shown in Table 2 below. A total of 114 siRNA motifs exhibited an inhibition rate greater than 25% on LPA gene expression, demonstrating favorable inhibitory activity.

**Table 2: Inhibition of siRNA core sequences on LPA gene expression**

| siRNA ID | Sense strand sequence (5'-3') | SEQ ID NO. | Antisense strand sequence (5'-3') | SEQ ID NO. | Inhibition rate (%) |
|---|---|---|---|---|---|
| B01001 | CCAUGGUAAUGGACAGAGUUA | 1 | UAACUCUGUCCAUUACCAUGGUA | 194 | 56.2 |
| B01002 | UUGCUACCAUGGUGAUGGACA | 2 | UGUCCAUCACCAUGGUAGCAAUC | 195 | 3.2 |
| B01003 | UGCUACCACGGUAAUGGACA | 3 | UGUCCAUUACCGUGGUAGCACU | 196 | -5.8 |
| B01004 | GUGCUACCACGGAAAUGGACA | 4 | UGUCCAUUUCCGUGGUAGCACUC | 197 | -6.2 |
| B01005 | UGCUACUACCAUUAUGGACA | 5 | UGUCCAUAAUGGUAGUAGCAGU | 198 | 1.3 |
| B01006 | CUGCUACCAUGGUGAUGGACA | 6 | UGUCCAUCACCAUGGUAGCAGUC | 199 | -3.7 |
| B01007 | UGCUACCAUGGUAAUGGACA | 7 | UGUCCAUUACCAUGGUAGCACU | 200 | -1.2 |
| B01008 | AGUGCUACCAUGGUAAUGGAA | 8 | UUCCAUUACCAUGGUAGCACUCC | 201 | 11.4 |
| B01009 | AGUGCUACCACGGUAAUGGAA | 9 | UUCCAUUACCGUGGUAGCACUCC | 202 | 10.5 |
| B01010 | GUGAUGGACAGAGUUAUCGA | 10 | UCGAUAACUCUGUCCAUCACCA | 203 | 32.4 |
| B01013 | GUGAUGGACAGAGUUAUCGAA | 11 | UUCGAUAACUCUGUCCAUCACCA | 204 | 67.2 |
| B01014 | UGGUCCAGGAUUGCUACCAU | 12 | AUGGUAGCAAUCCUGGACCACA | 205 | 3.9 |
| B01015 | GGGUGCAGGAGUGCUACCAC | 13 | GUGGUAGCACUCCUGCACCCCA | 206 | -9.4 |
| B01016 | UAAUGGACAGAGUUAUCGAA | 14 | UUCGAUAACUCUGUCCAUUACC | 207 | 41.5 |
| B01017 | GGGUGCAGGAGUGCUACCAU | 15 | AUGGUAGCACUCCUGCACCCCA | 208 | -11.3 |
| B01018 | AUGGACAGAGUUAUCGAGGCA | 16 | UGCCUCGAUAACUCUGUCCAUUA | 209 | 46.8 |
| B01019 | AUGGACAGAGUUAUCGAGGCU | 17 | AGCCUCGAUAACUCUGUCCAUCA | 210 | 59.8 |
| B01020 | GGACAGAGUUAUCGAGGCACA | 18 | UGUGCCUCGAUAACUCUGUCCAU | 211 | 17.4 |
| B01021 | GACAGAGUUAUCAAGGCACA | 19 | UGUGCCUUGAUAACUCUGUCCA | 212 | 3.9 |
| B01022 | GGACAGAGUUAUCGAGGCUCA | 20 | UGAGCCUCGAUAACUCUGUCCAU | 213 | 24.3 |
| B01023 | GACAGAGUUAUCAAGGCACAU | 21 | AUGUGCCUUGAUAACUCUGUCCA | 214 | 67.9 |
| B01024 | ACAGAGUUAUCGAGGCUCAU | 22 | AUGAGCCUCGAUAACUCUGUCC | 215 | 21.1 |
| B01025 | ACAGAGUUAUCGAGGCACAU | 23 | AUGUGCCUCGAUAACUCUGUCC | 216 | 14.5 |
| B01026 | CAGAGUUAUCAAGGCACAUA | 24 | UAUGUGCCUUGAUAACUCUGUC | 217 | 3.8 |
| B01027 | CAGAGUUAUCGAGGCUCAUU | 25 | AAUGAGCCUCGAUAACUCUGUC | 218 | 28.6 |
| B01028 | CAGAGUUAUCGAGGCACAUA | 26 | UAUGUGCCUCGAUAACUCUGUC | 219 | 10.6 |
| B01029 | GCUACCAUGGUAAUGGACAGA | 27 | UCUGUCCAUUACCAUGGUAGCAC | 220 | 37.7 |
| B01030 | GAUUGCUACCAUGGUGAUGGA | 28 | UCCAUCACCAUGGUAGCAAUCCU | 221 | 43.3 |
| B01031 | GCUACCACGGUAAUGGACAGA | 29 | UCUGUCCAUUACCGUGGUAGCAC | 222 | 21.1 |
| B01032 | AGUGCUACCACGGUAAUGGA | 30 | UCCAUUACCGUGGUAGCACUCC | 223 | 9.1 |
| B01033 | GCUACTACCAUUAUGGACAGA | 31 | UCUGUCCAUAAUGGUAGUAGCAG | 224 | -4.3 |
| B01034 | AGAGUUAUCGAGGCACAUACU | 32 | AGUAUGUGCCUCGAUAACUCUGU | 225 | 24.4 |
| B01035 | AGUGCUACCAUGGUAAUGGA | 33 | UCCAUUACCAUGGUAGCACUCC | 226 | 13.1 |
| B01037 | UACCAUGGUAAUGGACAGAGU | 34 | ACUCUGUCCAUUACCAUGGUAGC | 227 | 72.8 |
| B01038 | ACCACGGUAAUGGACAGAGU | 35 | ACUCUGUCCAUUACCGUGGUAG | 228 | 11.7 |
| B01039 | UACTACCAUUAUGGACAGAGU | 36 | ACUCUGUCCAUAAUGGUAGUAGC | 229 | 61.0 |
| B01040 | ACCACGGUAAUGGACAGAGUU | 37 | AACUCUGUCCAUUACCGUGGUAG | 230 | 57.7 |
| B01041 | ACTACCAUUAUGGACAGAGUU | 38 | AACUCUGUCCAUAAUGGUAGUAG | 231 | 35.6 |
| B01042 | CCAUGGUAAUGGACAGAGUU | 39 | AACUCUGUCCAUUACCAUGGUA | 232 | 55.3 |
| B01043 | CACGGUAAUGGACAGAGUUA | 40 | UAACUCUGUCCAUUACCGUGGU | 233 | 23.1 |
| B01044 | AUGGUGAUGGACAGAGUUAU | 41 | AUAACUCUGUCCAUCACCAUGG | 234 | 24.2 |
| B01045 | CACGGUAAUGGACAGAGUUAU | 42 | AUAACUCUGUCCAUUACCGUGGU | 235 | 77.5 |
| B01046 | ACCAUUAUGGACAGAGUUAU | 43 | AUAACUCUGUCCAUAAUGGUAG | 236 | 24.9 |
| B01047 | AUGGUAAUGGACAGAGUUAU | 44 | AUAACUCUGUCCAUUACCAUGG | 237 | 47.4 |
| B01048 | UGGUGAUGGACAGAGUUAUA | 45 | UAUAACUCUGUCCAUCACCAUG | 238 | 34.6 |
| B01049 | ACGGUAAUGGACAGAGUUAUA | 46 | UAUAACUCUGUCCAUUACCGUGG | 239 | 62.3 |
| B01050 | UGGUAAUGGACAGAGUUAUA | 47 | UAUAACUCUGUCCAUUACCAUG | 240 | 35.5 |
| B01051 | GGUGAUGGACAGAGUUAUCA | 48 | UGAUAACUCUGUCCAUCACCAU | 241 | 10.5 |
| B01052 | GGUAAUGGACAGAGUUAUCA | 49 | UGAUAACUCUGUCCAUUACCAU | 242 | 25.2 |
| B01053 | GUAAUGGACAGAGUUAUCGA | 50 | UCGAUAACUCUGUCCAUUACCA | 243 | 53.1 |
| B01501 | GGUGAUGGACAGAGUUAUCGA | 51 | UCGAUAACUCUGUCCAUCACCUC | 244 | 75.8 |
| B01502 | GUGGUCCAGGAUUGCUACCAU | 52 | AUGGUAGCAAUCCUGGACCACAG | 245 | 72.0 |
| B01503 | CCAGAGUUAUCGAGGCACAUU | 53 | AAUGUGCCUCGAUAACUCUGGCC | 246 | 34.5 |
| B01504 | GUUAUCGAGGCACAUUCUCCA | 54 | UGGAGAAUGUGCCUCGAUAACUC | 247 | 74.7 |
| B01505 | GAGUUAUCGAGGCACAUUCU | 55 | AGAAUGUGCCUCGAUAACUCUG | 248 | 7.9 |
| B01506 | GAGUUAUCGAGGCACAUUCUA | 56 | UAGAAUGUGCCUCGAUAACUCUG | 249 | 32.4 |
| B01507 | GCAGUGCUACCAUGGUAAUGA | 57 | UCAUUACCAUGGUAGCACUGCCG | 250 | 48.3 |
| B01508 | GAGGUGAUGGACAGAGUUAUA | 58 | UAUAACUCUGUCCAUCACCUCGG | 251 | 58.0 |
| B01509 | AGGUGAUGGACAGAGUUAUCA | 59 | UGAUAACUCUGUCCAUCACCUCG | 252 | 58.4 |
| B02401 | CCAAGCUUGGUCAUCUAUGAA | 60 | UUCAUAGAUGACCAAGCUUGGCA | 253 | 59.7 |
| B02402 | GUCACAGGAAGGACCUGCCAA | 61 | UUGGCAGGUCCUUCCUGUGACAG | 254 | 27.3 |
| B02403 | GUCACAGGAAGGACAUGUCAA | 62 | UUGACAUGUCCUUCCUGUGACAG | 255 | 39.8 |
| B02404 | AUCACAGGAAGAACAUGUCAA | 63 | UUGACAUGUUCUUCCUGUGAUAG | 256 | 43.2 |
| B02405 | ACAGGAAGAACCUGCCAAGCU | 64 | AGCUUGGCAGGUUCUUCCUGUGA | 257 | 25.2 |
| B02406 | ACAGGAAGGACCUGCCAAGCU | 65 | AGCUUGGCAGGUCCUUCCUGUGA | 258 | 18.3 |
| B02407 | ACAGGAAGGACAUGUCAAUCU | 66 | AGAUUGACAUGUCCUUCCUGUGA | 259 | 32.5 |
| B02408 | ACAGGAAGAACUUGCCAAGCU | 67 | AGCUUGGCAAGUUCUUCCUGUGA | 260 | 43.0 |
| B02409 | ACAGGAAGGACAUGUCAGUCU | 68 | AGACUGACAUGUCCUUCCUGUGA | 261 | 13.2 |
| B02410 | ACAGGAAGAACAUGUCAGUCU | 69 | AGACUGACAUGUUCUUCCUGUGA | 262 | 31.3 |
| B02411 | CAGGAAGAACCUGCCAAGCUU | 70 | AAGCUUGGCAGGUUCUUCCUGUG | 263 | 60.2 |
| B02412 | CAGGAAGGACCUGCCAAGCUU | 71 | AAGCUUGGCAGGUCCUUCCUGUG | 264 | 66.9 |
| B02413 | AGGAAGGACAUGUCAAUCUU | 72 | AAGAUUGACAUGUCCUUCCUGU | 265 | 0.7 |
| B02414 | CAGGAAGAACUUGCCAAGCUU | 73 | AAGCUUGGCAAGUUCUUCCUGUG | 266 | 75.8 |
| B02415 | AGGAAGGACAUGUCAGUCUU | 74 | AAGACUGACAUGUCCUUCCUGU | 267 | 28.3 |
| B02416 | CAGGAAGGACAUGUCAGUCUU | 75 | AAGACUGACAUGUCCUUCCUGUA | 268 | 60.4 |
| B02417 | CAGGAAGAACAUGUCAGUCUU | 76 | AAGACUGACAUGUUCUUCCUGUG | 269 | 58.9 |
| B02418 | GAAGAACCUGCCAAGCUUGGU | 77 | ACCAAGCUUGGCAGGUUCUUCCU | 270 | 21.2 |
| B02419 | GAAGGACCUGCCAAGCUUGGU | 78 | ACCAAGCUUGGCAGGUCCUUCCU | 271 | 16.6 |
| B02420 | GAAGGACAUGUCAAUCUUGGU | 79 | ACCAAGAUUGACAUGUCCUUCCU | 272 | 48.1 |
| B02421 | GAAGGACAUGUCAGUCUUGGU | 80 | ACCAAGACUGACAUGUCCUUCCU | 273 | 49.9 |
| B02422 | GAAGAACAUGUCAGUCUUGGU | 81 | ACCAAGACUGACAUGUUCUUCCU | 274 | 42.9 |
| B02423 | AGAACCUGCCAAGCUUGGUCA | 82 | UGACCAAGCUUGGCAGGUUCUUC | 275 | 25.9 |
| B02425 | AGGACAUGUCAAUCUUGGUCA | 83 | UGACCAAGAUUGACAUGUCCUUC | 276 | 39.6 |
| B02426 | AGAACUUGCCAAGCUUGGUCA | 84 | UGACCAAGCUUGGCAAGUUCUUC | 277 | 46.5 |
| B02427 | AGGACAUGUCAGUCUUGGUCA | 85 | UGACCAAGACUGACAUGUCCUUC | 278 | 53.4 |
| B02428 | CAUGUCAAUCUUGGUCAUCCA | 86 | UGGAUGACCAAGAUUGACAUGUC | 279 | 40.5 |
| B02429 | CAUGUCAGUCUUGGUCCUCUA | 87 | UAGAGGACCAAGACUGACAUGUC | 280 | 43.1 |
| B02430 | CCUGUCAAUCUUGGUCAUCUA | 88 | UAGAUGACCAAGAUUGACAGGUC | 281 | 74.2 |
| B02431 | CUGCCAAGCUUGGUCAUCUA | 89 | UAGAUGACCAAGCUUGGCAGGU | 282 | 22.9 |
| B02432 | CUGCCAAGCUUGGUCAUCUAU | 90 | AUAGAUGACCAAGCUUGGCAGGU | 283 | 38.9 |
| B02433 | AUGUCAAUCUUGGUCAUCCAU | 91 | AUGGAUGACCAAGAUUGACAUGU | 284 | 28.9 |
| B02434 | UUGCCAAGCUUGGUCAUCUAU | 92 | AUAGAUGACCAAGCUUGGCAAGU | 285 | 52.7 |
| B02435 | AUGUCAGUCUUGGUCCUCUAU | 93 | AUAGAGGACCAAGACUGACAUGU | 286 | 38.1 |
| B02436 | GUCAAUCUUGGUCAUCCAUGA | 94 | UCAUGGAUGACCAAGAUUGACAU | 287 | 38.8 |
| B02437 | GUCAGUCUUGGUCCUCUAUGA | 95 | UCAUAGAGGACCAAGACUGACAU | 288 | 40.4 |
| B02438 | UCAAUCUUGGUCAUCUAUGA | 96 | UCAUAGAUGACCAAGAUUGACA | 289 | 27.1 |
| B02439 | CCAAGCUUGGUCAUCUAUGA | 97 | UCAUAGAUGACCAAGCUUGGCA | 290 | 13.6 |
| B02440 | UCAAUCUUGGUCAUCCAUGAC | 98 | GUCAUGGAUGACCAAGAUUGACA | 291 | 16.9 |
| B02441 | UCAGUCUUGGUCCUCUAUGAC | 99 | GUCAUAGAGGACCAAGACUGACA | 292 | 15.6 |
| B02442 | UCAAUCUUGGUCAUCUAUGAU | 100 | AUCAUAGAUGACCAAGAUUGACA | 293 | 53.8 |
| B02443 | CAAGCUUGGUCAUCUAUGAC | 101 | GUCAUAGAUGACCAAGCUUGGC | 294 | 28.8 |
| B02444 | CAAGCUUGGUCAUCUAUGACA | 102 | UGUCAUAGAUGACCAAGCUUGGC | 295 | 29.3 |
| B02445 | CAAUCUUGGUCAUCCAUGACA | 103 | UGUCAUGGAUGACCAAGAUUGAC | 296 | 21.4 |
| B02446 | CAGUCUUGGUCCUCUAUGACA | 104 | UGUCAUAGAGGACCAAGACUGAC | 297 | 32.7 |
| B02447 | CAAUCUUGGUCAUCUAUGAUA | 105 | UAUCAUAGAUGACCAAGAUUGAC | 298 | 33.3 |
| B02448 | AAUCUUGGUCAUCCAUGACAA | 106 | UUGUCAUGGAUGACCAAGAUUGA | 299 | 48.4 |
| B02449 | AGUCUUGGUCCUCUAUGACAA | 107 | UUGUCAUAGAGGACCAAGACUGA | 300 | 48.4 |
| B02450 | AAUCUUGGUCAUCUAUGAUAA | 108 | UUAUCAUAGAUGACCAAGAUUGA | 301 | 55.8 |
| B02451 | UCUUGGUCAUCCAUGACACCA | 109 | UGGUGUCAUGGAUGACCAAGAUU | 302 | 8.5 |
| B02452 | UCUUGGUCCUCUAUGACACCA | 110 | UGGUGUCAUAGAGGACCAAGACU | 303 | 24.5 |
| B02453 | UCUUGGUCAUCUAUGAUACCA | 111 | UGGUAUCAUAGAUGACCAAGAUU | 304 | 25.9 |
| B02454 | UUGGUCAUCCAUGACACCACA | 112 | UGUGGUGUCAUGGAUGACCAAGA | 305 | 29.2 |
| B02455 | UUGGUCCUCUAUGACACCACA | 113 | UGUGGUGUCAUAGAGGACCAAGA | 306 | 28.3 |
| B02456 | UUGGUCAUCUAUGAUACCACA | 114 | UGUGGUAUCAUAGAUGACCAAGA | 307 | 47.8 |
| B04101 | CUGCAGGAAUCCAGAUGCUGU | 115 | ACAGCAUCUGGAUUCCUGCAGUA | 308 | 63.0 |
| B04102 | AAGAACUACUGCCGAAAUCCA | 116 | UGGAUUUCGGCAGUAGUUCUUGA | 309 | 4.6 |
| B04103 | AACUACUGCCGAAAUCCAGAU | 117 | AUCUGGAUUUCGGCAGUAGUUCU | 310 | 30.6 |
| B04104 | ACUACUGCCGAAAUCCAGAUU | 118 | AAUCUGGAUUUCGGCAGUAGUUC | 311 | 28.1 |
| B04105 | UACUGCCGAAAUCCAGAUCCU | 119 | AGGAUCUGGAUUUCGGCAGUAGU | 312 | 34.6 |
| B04106 | ACUGCAGGAAUCCAGAUCCUA | 120 | UAGGAUCUGGAUUCCUGCAGUAG | 313 | 34.2 |
| B04107 | ACUGCCGAAAUCCAGAUCCUA | 121 | UAGGAUCUGGAUUUCGGCAGUAG | 314 | 36.6 |
| B04108 | CUGCAGGAAUCCAGAUCCUGU | 122 | ACAGGAUCUGGAUUCCUGCAGUA | 315 | 24.7 |
| B04109 | CUGCCGAAAUCCAGAUCCUGU | 123 | ACAGGAUCUGGAUUUCGGCAGUA | 316 | 34.3 |
| B04110 | GCAGGAAUCCAGAUCCUGUGA | 124 | UCACAGGAUCUGGAUUCCUGCAG | 317 | 0.8 |
| B04111 | GCCGAAAUCCAGAUCCUGUGA | 125 | UCACAGGAUCUGGAUUUCGGCAG | 318 | 9.2 |
| B04401 | AAUCCAGAUGCUGAGAUUCGA | 126 | UCGAAUCUCAGCAUCUGGAUUCC | 319 | 41.8 |
| B04402 | GCAGGAAUCCAGAUGCUGAGA | 127 | UCUCAGCAUCUGGAUUCCUGCAG | 320 | 34.5 |
| B04501 | AGAUGCUGAGAUUAGUCCUUA | 128 | UAAGGACUAAUCUCAGCAUCUGG | 321 | 57.0 |
| B04502 | ACUGCAGGAAUCCAGAUUCUA | 129 | UAGAAUCUGGAUUCCUGCAGUAG | 322 | 37.8 |
| B04503 | CUGCAGGAAUCCAGAUUCUGA | 130 | UCAGAAUCUGGAUUCCUGCAGUA | 323 | 65.3 |
| B04504 | GCAGGAAUCCAGAUUCUGGGA | 131 | UCCCAGAAUCUGGAUUCCUGCAG | 324 | 21.6 |
| B06001 | GUACUGCAACCUGACGCGAUA | 132 | UAUCGCGUCAGGUUGCAGUACUC | 325 | 57.5 |
| B06002 | GAGUACUGCAACCUGACGCGA | 133 | UCGCGUCAGGUUGCAGUACUCCC | 326 | 11.5 |
| B06003 | AGUACUGCAACCUGACACAA | 134 | UUGUGUCAGGUUGCAGUACUCC | 327 | 9.3 |
| B06004 | GAGUACUGCAACCUGACACGA | 135 | UCGUGUCAGGUUGCAGUACUCCC | 328 | 19.9 |
| B06005 | AGUACUGCAACCUGACGCGAU | 136 | AUCGCGUCAGGUUGCAGUACUCC | 329 | 5.7 |
| B06006 | AGUACUGCAACCUGACACAAU | 137 | AUUGUGUCAGGUUGCAGUACUCC | 330 | 8.0 |
| B06007 | AGUACUGCAACCUGACACGAU | 138 | AUCGUGUCAGGUUGCAGUACUCC | 331 | 0.6 |
| B06008 | UACUGCAACCUGACACAAUA | 139 | UAUUGUGUCAGGUUGCAGUACU | 332 | -1.1 |
| B06009 | GUACUGCAACCUGACACGAUA | 140 | UAUCGUGUCAGGUUGCAGUACUC | 333 | 49.8 |
| B06010 | UACUGCAACCUGACGCGAUGA | 141 | UCAUCGCGUCAGGUUGCAGUACU | 334 | 17.9 |
| B06011 | UACUGCAACCUGACACAAUGU | 142 | ACAUUGUGUCAGGUUGCAGUACU | 335 | 41.3 |
| B06012 | UACUGCAACCUGACACGAUGU | 143 | ACAUCGUGUCAGGUUGCAGUACU | 336 | 28.5 |
| B06013 | ACUGCAACCUGACGCGAUGCU | 144 | AGCAUCGCGUCAGGUUGCAGUAC | 337 | 10.4 |
| B06014 | ACUGCAACCUGACACAAUGCU | 145 | AGCAUUGUGUCAGGUUGCAGUAC | 338 | 1.5 |
| B06015 | ACUGCAACCUGACACGAUGCU | 146 | AGCAUCGUGUCAGGUUGCAGUAC | 339 | 0.2 |
| B06016 | ACUGCAACCUGACACAAUGUU | 147 | AACAUUGUGUCAGGUUGCAGUAC | 340 | 10.8 |
| B06017 | GCAACCUGACGCGAUGCUCA | 148 | UGAGCAUCGCGUCAGGUUGCAG | 341 | 3.1 |
| B06018 | GCAACCUGACACAAUGCUCA | 149 | UGAGCAUUGUGUCAGGUUGCAG | 342 | 2.5 |
| B06019 | GCAACCUGACACGAUGCUCA | 150 | UGAGCAUCGUGUCAGGUUGCAG | 343 | 1.1 |
| B06020 | GCAACCUGACACAAUGUCCA | 151 | UGGACAUUGUGUCAGGUUGCAG | 344 | 1.1 |
| B06021 | CAACCUGACGCGAUGCUCAGA | 152 | UCUGAGCAUCGCGUCAGGUUGCA | 345 | 15.2 |
| B06022 | CAACCUGACACAAUGCUCAGA | 153 | UCUGAGCAUUGUGUCAGGUUGCA | 346 | 17.8 |
| B06023 | CAACCUGACACGAUGCUCAGA | 154 | UCUGAGCAUCGUGUCAGGUUGCA | 347 | 9.2 |
| B06024 | CAACCUGACACAAUGUCCAGU | 155 | ACUGGACAUUGUGUCAGGUUGCA | 348 | 12.7 |
| B06025 | CCUGACGCGAUGCUCAGACA | 156 | UGUCUGAGCAUCGCGUCAGGUU | 349 | 12.8 |
| B06026 | ACCUGACACGAUGCUCAGAUA | 157 | UAUCUGAGCAUCGUGUCAGGUUG | 350 | 44.7 |
| B06027 | CCUGACACAAUGUCCAGUGA | 158 | UCACUGGACAUUGUGUCAGGUU | 351 | -1.0 |
| B06028 | AUCUGACACAAUGCUCAGAAA | 159 | UUUCUGAGCAUUGUGUCAGAUUG | 352 | 40.0 |
| B06601 | GGGUCCAGGAUUGCUACCAUA | 160 | UAUGGUAGCAAUCCUGGACCCCG | 353 | 55.0 |
| B06602 | GAAGAAGCACCAACGGAGCAA | 161 | UUGCUCCGUUGGUGCUUCUUCAG | 354 | 6.5 |
| B06604 | GAAGCACCAACGGAGCAAAGA | 162 | UCUUUGCUCCGUUGGUGCUUCUU | 355 | -6.4 |
| B06605 | GGUCCAGGAUUGCUACCAUGA | 163 | UCAUGGUAGCAAUCCUGGACCCC | 356 | 41.0 |
| B06606 | GUCCAGGAUUGCUACCAUGGU | 164 | ACCAUGGUAGCAAUCCUGGACCC | 357 | 48.0 |
| B06607 | CAGGAUUGCUACCAUGGUGA | 165 | UCACCAUGGUAGCAAUCCUGGA | 358 | 17.3 |
| B06608 | CAGGAUUGCUACCAUGGUGAU | 166 | AUCACCAUGGUAGCAAUCCUGGA | 359 | 30.3 |
| B06609 | GGAUUGCUACCAUGGUGAUGA | 167 | UCAUCACCAUGGUAGCAAUCCUG | 360 | 50.7 |
| B00101 | UGGCAUCAGAGGACAACAGAA | 168 | UUCUGUUGUCCUCUGAUGCCAGU | 361 | 31.4 |
| B00201 | AAGAAGCACCAACUGAAAACA | 169 | UGUUUUCAGUUGGUGCUUCUUCA | 362 | 20.7 |
| B00301 | UAUCACAGGAAGAACAUGUCA | 170 | UGACAUGUUCUUCCUGUGAUAGU | 363 | 21.7 |
| B00401 | CUGAGAUUCGCCCUUGGUGUU | 171 | AACACCAAGGGCGAAUCUCAGCA | 364 | 11.8 |
| B00501 | AAGAAAUGUCCUGGAAGCAUU | 172 | AAUGCUUCCAGGACAUUUCUUCG | 365 | 40.9 |
| B00601 | CACGGUAAUGGACAGAGUUAU | 173 | AUAACUCUGUCCAUUACCGUGGU | 366 | 70.9 |
| B00701 | ACACGAUGCUCAGAUGCAGAA | 174 | UUCUGCAUCUGAGCAUCGUGUCA | 367 | 20.7 |
| B00801 | CAGGAAGGACAUGUCAGUCUU | 175 | AAGACUGACAUGUCCUUCCUGUG | 368 | 53.1 |
| B00901 | AAGCAGCACCAACUGAGCAAA | 176 | UUUGCUCAGUUGGUGCUGCUUCA | 369 | 7.0 |
| B02001 | ACGGCUGUUUCUGAACAAGCA | 177 | UGCUUGUUCAGAAACAGCCGUGG | 370 | -4.3 |
| B01101 | CUGAGAUUCGCCCUUGGUGUU | 178 | AACACCAAGGGCGAAUCUCAGCA | 371 | 32.4 |
| B01201 | UCGCCCUUGGUGUUACACCAU | 179 | AUGGUGUAACACCAAGGGCGAAU | 372 | 47.2 |
| B01401 | UCCAGUGACAGAAUCAAGUGU | 180 | ACACUUGAUUCUGUCACUGGACA | 373 | 46.5 |
| B01501 | CUGUUUCUGAACAAGCACCAA | 181 | UUGGUGCUUGUUCAGAAACAGCC | 374 | 35.7 |
| B01601 | UUCGCCCUUGGUGUUAUACCA | 182 | UGGUAUAACACCAAGGGCGAAUC | 375 | 18.2 |
| B01701 | AAGCACAGAGGCUCCUUCUGA | 183 | UCAGAAGGAGCCUCUGUGCUUGG | 376 | 35.6 |
| B01801 | ACAGAGGCUCCUUCUGAACAA | 184 | UUGUUCAGAAGGAGCCUCUGUGC | 377 | 55.6 |
| B01901 | CUGAACAAGCACCACCUGAGA | 185 | UCUCAGGUGGUGCUUGUUCAGAA | 378 | 40.2 |
| B01012 | UGGACAGAGUUAUCAAGGCAA | 186 | UUGCCUUGAUAACUCUGUCCAUU | 379 | 67.6 |
| B01011 | CAGAGUUAUCAAGGCACAUAA | 187 | UUAUGUGCCUUGAUAACUCUGUC | 380 | 53.9 |
| B02457 | AGGAAGGACAUGUCAGUCUUA | 188 | UAAGACUGACAUGUCCUUCCUGU | 381 | 45.8 |
| B02458 | CACAGGAAGGACAUGUCAGUA | 189 | UACUGACAUGUCCUUCCUGUGAC | 382 | 42.6 |
| B02459 | AGGAAGAACAUGUCAGUCUUA | 190 | UAAGACUGACAUGUUCUUCCUGU | 383 | 43.2 |
| B02460 | CACAGGAAGAACAUGUCAGUA | 191 | UACUGACAUGUUCUUCCUGUGAU | 384 | 44.9 |
| B06029 | UACUGCAACCUGACGCGAUGA | 192 | UCAUCGCGUCAGGUUGCAGUACU | 385 | 45.6 |
| ANC | AUGGUACCUCUUCGCAAUACU | 193 | UCUUGAAGGAGAUCCAAUGAG | 386 | -2.9 |

### Example 2: Synthesis of alternately modified siRNA derivatives

To enhance the inhibitory activity and stability, the siRNA motifs listed in Table 2 were subjected to alternating modifications with 2'-O-methyl (2'-OMe) and 2'-fluoro (2'-F), and 3',5'-phosphorothioate linkages were introduced between nucleotides at the 5' end and/or the 3' end.

### 2.1 Synthesis of alternately modified siRNA derivatives

In the present disclosure, the rules for alternating modification are as follows: nucleotides at odd-numbered positions of the sense strand and nucleotides at even-numbered positions of the antisense strand are modified with 2'-F, while nucleotides at all other positions are modified with 2'-OMe. Additionally, a 3',5'-phosphorothioate linkage is introduced between nucleotides at positions 1 and 2 and between nucleotides at positions 2 and 3 from the 5' end of the sense strand; a 3',5'-phosphorothioate linkage is introduced between nucleotides at positions 1 and 2 and between nucleotides at positions 2 and 3 from the 5' end of the antisense strand, and a 3',5'-phosphorothioate linkage is introduced between nucleotides at positions 1 and 2 and between nucleotides at positions 2 and 3 from the 3' end of the antisense strand. The alternately modified siRNA derivatives designed according to the rules are designated by adding the suffix "-AL" after the code of the original core sequence, as shown in Table 3.

Instruments and reagents: 192 P DNA/RNA automatic synthesizer (Tsingke), using a universal solid-phase support consisting of cross-linked polystyrene beads; model: Primer Support 5G Unylinker 350 (manufactured by Cytiva).

### For example, the preparation method may include:

Nucleotide monomers were dissolved in acetonitrile at a concentration of 0.15 M, including DMT-A-OMe phosphoramidite monomer (Formula 1), DMT-C-OMe phosphoramidite monomer (Formula 2), DMT-G-OMe phosphoramidite monomer (Formula 3), DMT-U-OMe phosphoramidite monomer (Formula 4), DMT-A-F phosphoramidite monomer (Formula 5), DMT-C-F phosphoramidite monomer (Formula 6), DMT-G-F phosphoramidite monomer (Formula 7), and DMT-U-F phosphoramidite monomer (Formula 8).

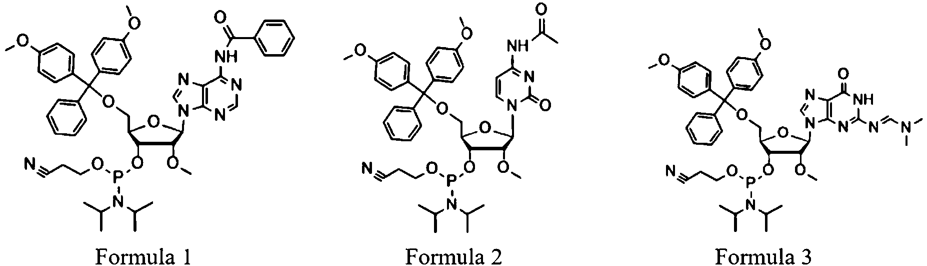

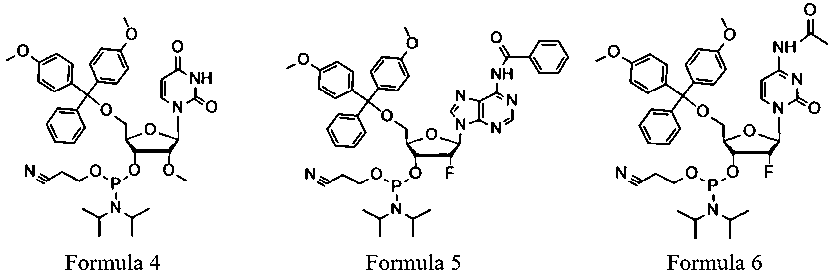

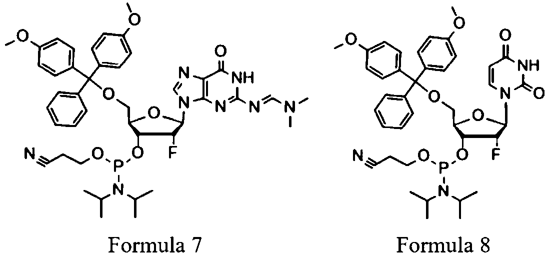

Nucleotide monomers were sequentially attached in the 3' to 5' direction according to the nucleotide arrangement using the solid-phase phosphoramidite method. The process of attaching each nucleotide monomer involves four steps: deprotection, coupling, oxidation or sulfurization, and hydroxyl protection. Specifically, when two nucleotides are linked via a phosphate linkage, the process of attaching a subsequent nucleotide monomer involves four steps: deprotection, coupling, oxidation, and hydroxyl protection. When two nucleotides are linked via a phosphorothioate linkage, the process of attaching a subsequent nucleotide monomer involves four steps: deprotection, coupling, sulfurization, and hydroxyl protection.

### (1) Deprotection

3% dichloroacetic acid in toluene was used as a deprotection reagent to remove the DMT protecting group, followed by washing with acetonitrile.

### (2) Coupling

Each nucleotide monomer in acetonitrile was coupled using 0.25 M 5-ethylthio-1*H-*tetrazole as an activator, followed by washing with acetonitrile.

### (3) Oxidation/sulfurization

Oxidation: 0.05 M iodine in pyridine/water (90/10) was used as an oxidant for oxidation, followed by washing with acetonitrile.

Sulfurization: 3% xanthane hydride in pyridine was used as a sulfurizing agent for sulfurization, followed by washing with acetonitrile.

### (4) Hydroxyl protection

10% acetic anhydride in tetrahydrofuran (CAP A) and tetrahydrofuran/pyridine/N-methylimidazole (74/10/16, v/v/v) (CAP B) were used as hydroxyl protecting reagents for hydroxyl protection, followed by washing with acetonitrile.

The aforementioned steps were repeated according to the specified nucleotide arrangement to obtain a product with the specific sequence arrangement in either the sense strand or the antisense strand.

(5) 3% dichloroacetic acid in toluene was used as a deprotection reagent to remove the DMT protecting group from the last nucleotide, followed by washing with acetonitrile.

### (6) Ammonolysis and purification

The solid-phase support was transferred to a reactor and treated with concentrated aqueous ammonia (25 to 28%) at 60°C for 12 hours. The system was then cooled to room temperature, and the mixture was transferred to a pressure filter for filtration. The filter cake was washed with a mixture of purified water and ethanol. The filtrates were combined, subjected to column chromatography, concentrated, and lyophilized to obtain 2'-OMe and 2'-F modified single-stranded products.

### (7) Annealing

The purified sense and antisense strands were mixed at a molar ratio of 1:1, heated to 95°C and maintained for 3 minutes, and slowly cooled to room temperature to form an siRNA duplex.

The sense and antisense strands of the siRNAs listed in Table 3 were synthesized using the method described above, totaling 194 siRNAs. Specifically, APC-AL serves as a positive control, corresponding to the original sequence of Olpasiran, a drug has entered Phase III clinical trials (the core sequence of the sense strand is set forth in SEQ ID NO: 387 and the core sequence of the antisense strand is set forth in SEQ ID NO: 388, both subjected to the alternating modifications described above). ANC-AL serves as a negative control, corresponding to an alternately modified nonsense sequence (the core sequence is shown in Table 2).
SEQ ID NO: 387: CAGCCCCUUAUUGUUAUACGA
SEQ ID NO: 388: UCGUAUAACAAUAAGGGGCUG

### Example 3: Inhibitory effect of alternately modified siRNA derivatives on LPA gene expression

The inhibitory effects of the alternately modified siRNA derivatives synthesized in Example 2 on LPA gene expression were evaluated using two methods: a dual-luciferase assay and a transfection assay in primary human hepatocytes.

### 3.1 Dual-luciferase assay

All 194 alternately modified derivatives listed in Table 3 were evaluated for their inhibitory effects on LPA gene expression using a dual-luciferase assay. The experimental materials and methods were the same as those described in Sections 1.2.1 and 1.2.2 of Example 1.

### 3.1.1 Experimental results

Three independent experiments were performed in triplicate for each siRNA derivative. The inhibitory effects of the siRNA derivatives listed in Table 3 on LPA gene expression were evaluated using a dual-luciferase assay.

**Table 3: Inhibition of alternately modified siRNA derivatives on LPA gene expression**

| siRNA ID | Inhibition rate (%) | siRNA ID | Inhibition rate (%) | siRNA ID | Inhibition rate (%) | siRNA ID | Inhibition rate (%) |
|---|---|---|---|---|---|---|---|
| B01001-AL | 64.0 | B01049-AL | 60.8 | B02433-AL | 20.6 | B06005-AL | 0.7 |
| B01002-AL | 18.5 | B01050-AL | 38.9 | B02434-AL | 47.6 | B06006-AL | 10.2 |
| B01003-AL | 10.9 | B01051-AL | 13.1 | B02435-AL | 30.8 | B06007-AL | -3.5 |
| B01004-AL | 13.6 | B01052-AL | 28.4 | B02436-AL | 32.5 | B06008-AL | -8.1 |
| B01005-AL | 12.4 | B01053-AL | 50.6 | B02437-AL | 35.3 | B06009-AL | 44.5 |
| B01006-AL | 20.0 | A01501-AL | 76.3 | B02438-AL | 12.9 | B06010-AL | 22.4 |
| B01007-AL | 15.8 | B01502-AL | 71.4 | B02439-AL | 11.4 | B06011-AL | 38.0 |
| B01008-AL | 20.7 | B01503-AL | 37.8 | B02440-AL | 6.9 | B06012-AL | 28.1 |
| B01009-AL | 16.1 | B01504-AL | 72.9 | B02441-AL | 6.1 | B06013-AL | 13.5 |
| B01010-AL | 38.1 | B01505-AL | 6.9 | B02442-AL | 49.3 | B06014-AL | 11.8 |
| B01013-AL | 73.3 | B01506-AL | 29.2 | B02443-AL | 11.7 | B06015-AL | 3.1 |
| B01014-AL | 11.8 | B01507-AL | 49.1 | B02444-AL | 26.7 | B06016-AL | 10.8 |
| B01015-AL | 6.0 | B01508-AL | 53.3 | B02445-AL | 12.1 | B06017-AL | 9.4 |
| B01016-AL | 50.7 | B01509-AL | 50.7 | B02446-AL | 15.5 | B06018-AL | 15.9 |
| B01017-AL | 5.7 | B02401-AL | 53.3 | B02447-AL | 23.2 | B06019-AL | 3.9 |
| B01018-AL | 52.9 | B02402-AL | 26.7 | B02448-AL | 42.4 | B06020-AL | 1.9 |
| B01019-AL | 61.4 | B02403-AL | 40.3 | B02449-AL | 41.4 | B06021-AL | 12.9 |
| B01020-AL | 28.3 | B02404-AL | 40.3 | B02450-AL | 47.6 | B06022-AL | 15.5 |
| B01021-AL | 19.4 | B02405-AL | 26.8 | B02451-AL | 2.0 | B06023-AL | 4.3 |
| B01022-AL | 34.9 | B02406-AL | 13.9 | B02452-AL | 11.0 | B06024-AL | 10.0 |
| B01023-AL | 71.3 | B02407-AL | 24.2 | B02453-AL | 16.4 | B06025-AL | 8.4 |
| B01024-AL | 34.0 | B02408-AL | 32.9 | B02454-AL | 18.8 | B06026-AL | 50.3 |
| B01025-AL | 12.7 | B02409-AL | 10.2 | B02455-AL | 25.5 | B06027-AL | 0.8 |
| B01026-AL | 16.2 | B02410-AL | 20.0 | B02456-AL | 46.6 | B06028-AL | 38.8 |
| B01027-AL | 31.1 | B02411-AL | 51.9 | B04101-AL | 57.0 | B06601-AL | 52.7 |
| B01028-AL | 16.9 | B02412-AL | 58.1 | B04102-AL | 4.0 | B06602-AL | 2.7 |
| B01029-AL | 44.2 | B02413-AL | 1.8 | B04103-AL | 31.0 | B06604-AL | -4.1 |
| B01030-AL | 48.2 | B02414-AL | 72.8 | B04104-AL | 24.1 | B06605-AL | 38.0 |
| B01031-AL | 23.0 | B02415-AL | 19.9 | B04105-AL | 28.2 | B06606-AL | 41.9 |
| B01032-AL | 10.8 | B02416-AL | 51.9 | B04106-AL | 29.5 | B06607-AL | 13.9 |
| B01033-AL | 6.0 | B02417-AL | 54.9 | B04107-AL | 34.1 | B06608-AL | 29.0 |
| B01034-AL | 26.8 | B02418-AL | 13.2 | B04108-AL | 20.9 | B06609-AL | 45.0 |
| B01035-AL | 17.8 | B02419-AL | 9.3 | B04109-AL | 28.6 | B00101-AL | 19.2 |
| B01037-AL | 72.3 | B02420-AL | 45.3 | B04110-AL | -4.6 | B00201-AL | 17.1 |
| B01038-AL | 15.8 | B02421-AL | 44.7 | B04111-AL | 7.3 | B00301-AL | 11.4 |
| B01039-AL | 61.9 | B02422-AL | 39.3 | B04401-AL | 31.5 | B00401-AL | 8.5 |
| B01040-AL | 59.7 | B02423-AL | 19.7 | B04402-AL | 29.2 | B00501-AL | 37.3 |
| B01041-AL | 38.5 | B02425-AL | 41.6 | B04501-AL | 51.5 | B00601-AL | 67.9 |
| B01042-AL | 53.0 | B02426-AL | 40.2 | B04502-AL | 33.7 | B00701-AL | 16.7 |
| B01043-AL | 23.3 | B02427-AL | 44.9 | B04503-AL | 58.0 | B00801-AL | 52.0 |
| B01044-AL | 29.5 | B02428-AL | 34.8 | B04504-AL | 12.5 | B00901-AL | 8.4 |
| B01045-AL | 77.1 | B02429-AL | 36.4 | B06001-AL | 51.3 | B02001-AL | -5.7 |
| B01046-AL | 22.4 | B02430-AL | 69.8 | B06002-AL | 12.2 | B01101-AL | 36.2 |
| B01047-AL | 50.7 | B02431-AL | 15.2 | B06003-AL | 3.0 | B01201-AL | 42.6 |
| B01048-AL | 39.8 | B02432-AL | 35.2 | B06004-AL | 16.4 | B01401-AL | 40.2 |
| B01501-AL | 33.2 | B01801-AL | 52.3 | B01011-AL | 50.1 | B02459-AL | 46.2 |
| B01601-AL | 25.3 | B01901-AL | 45.6 | B02457-AL | 44.2 | B02460-AL | 45.8 |
| B01701-AL | 39.2 | B01012-AL | 70.9 | B02458-AL | 48.2 | B06002-AL | 47.2 |
| APC-AL | 47.7 | ANC-AL | -3.6 | / | / | / | / |

### 3.2 Transfection assay for evaluation of inhibitory effect of alternately modified siRNA derivatives on LPA gene expression in primary human hepatocytes

A total of 18 siRNA derivatives with relatively high activity were selected from the 194 alternately modified siRNA derivatives listed in Table 3 (see Table 5). These derivatives were transfected into primary human hepatocytes using Lipofectamine RNAiMAX transfection reagent. The inhibitory effects of each sequence on LPA gene expression were measured by qPCR, and dose-response curves were fitted to calculate IC₄₀ values. The siRNA transfection was performed starting at a concentration of 10 nM, followed by 3-fold serial dilutions across eight concentrations (10 nM, 3.3 nM, 1.1 nM, 0.37 nM, 0.12 nM, 0.04 nM, 14 pM, and 5 pM), to evaluate the inhibitory activity against the LPA gene.

### 3.2.1 Experimental materials

**Table 4: Experimental materials**

| **Name** | **Brand** | **Cat. No.** | **ID No.** |
|---|---|---|---|
| Primary human hepatocytes (PHHs) | Liver Biotechnology | LV-PHH001 | - |
| PHH thawing medium | Liver Biotechnology | LV-REC001 | - |
| PHH seeding medium | Liver Biotechnology | LV-WEP001 | - |
| RNAiMAX transfection reagent | Invitrogen | 13778-150 | - |
| RNA extraction kit | QIAGEN | 74106 | - |
| FastKing RT Kit (with gDNase) | TIANGEN | KR116-02 | - |
| TaqMan Fast Advanced Master Mix | Thermo Fisher | 4444557 | - |
| LPA probe | Thermo Fisher | 4351370 | Hs00916691_ml |
| GAPDH probe | Thermo Fisher | 4448490 | Hs02786624_gl |

Solvent: sterilized RNase-free water.

### 3.2.2 Experimental methods

### 1) Thawing of primary human hepatocytes

The culture medium was pre-warmed at a constant temperature of 37°C for at least 30 minutes. 120 µL of PBS was added to each well of a collagen-coated 96-well plate, which was shaken several times, and the PBS was aspirated. A cryovial containing hepatocytes was removed from liquid nitrogen, placed in a 37°C water bath, and gently shaken until only a small amount of ice crystals remained in the vial. The cell suspension was poured into thawing medium in one step. 1 mL of thawing medium was pipetted to rinse the inner wall of the cryovial 2 to 3 times. The cell suspension in thawing medium was mixed by inversion. The mixture was centrifuged at 150 g for 5 minutes at room temperature. The supernatant was discarded, and the cells were resuspended in pre-warmed seeding medium. Cell counting was performed using a cell counter.

### 2) Seeding of primary human hepatocytes and siRNA transfection

**Preparation of siRNA transfection complex:** RNAiMAX transfection reagent and Opti-MEM were mixed at a volume ratio of 1.5:23.5 to obtain Solution A, which was incubated at room temperature for 15 minutes. The siRNA derivatives were diluted with Opti-MEM according to the desired transfection concentration to obtain Solution B, such that the siRNA concentration was 20× the final transfection concentration. Solution A and Solution B were mixed at a volume ratio of 1:1 to obtain Solution C, which was incubated at room temperature for 15 minutes and added to a collagen-coated 96-well plate at 10 µL per well.

**Cell dilution and seeding:** After counting the PHH cells, seeding medium was added to the cell suspension based on the counting results to adjust the cell density to 5 × 10⁵ to 6 × 10⁵ cells/mL, and mixed thoroughly. The cell suspension was aliquoted into the prepared collagen-coated 96-well plate at 90 µL per well using a multichannel pipette.

**Culture:** The plate was incubated at 37°C in a 5% CO₂ incubator with 95% relative humidity for 48 hours.

### 3) RNA extraction and reverse transcription

At 24 hours post-transfection, the culture medium was removed and the cells were collected for RNA extraction. Total RNA was extracted using the RNeasy Mini Kit (QIAGEN, 74106) according to the instructions provided by the manufacturer. Subsequently, cDNA was synthesized using the FastKing RT Kit (with gDNase) (TIANGEN, KR116-02) according to the instructions provided by the manufacturer.

### 4) RT-qPCR

mRNA levels of the target gene human LPA (Thermo, Hs00916691_ml) and the internal reference gene human GAPDH (Thermo, Hs02786624_gl) were quantified using TaqMan Fast Advanced Master Mix (Thermo, 4444557) according to the instructions provided by the manufacturer.

### 5) Data analysis

The RNA expression levels of the target gene in each sample were calculated based on Ct values using the ΔΔCt relative quantification method. Relative expression of the target gene was expressed as 2^{-ΔΔCt}.

### The calculation formula is as follows:

$ΔCt = mean Ct value of the target gene - mean Ct value of the reference gene ;$$ΔΔCt = ΔCt \left(treatment group\right) - ΔCt \left(RNAiMAX control group\right) ;$

Relative mRNA expression of the target gene = 2^{-ΔΔCT}; 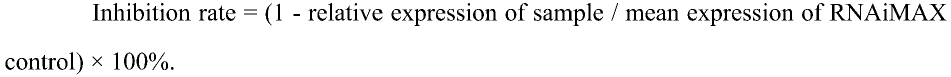 Inhibition rate = (1 - relative expression of sample/mean expression of RNAiMAX control) × 100%.

Dose-response curves were fitted using GraphPad Prism software (Nonlinear four-parameter logistic equation).

### 3.2.3 Experimental results

After three independent qPCR experiments, the inhibition rates of the siRNA derivatives at different concentrations on LPA gene expression in primary human hepatocytes are shown in Table 5 below. All of the derivatives exhibited certain inhibitory effects on LPA, among which B01023-AL achieved an inhibition rate of 90.0% at 10 nM. In addition, some of the derivatives exhibited superior IC₄₀ values compared to the positive control APC-AL. For example, B01023-AL exhibited an IC₄₀ value of 0.10 nM, outperforming that of the positive control (10.53 nM).

**Table 5: Inhibition rates and IC₄₀/IC₅₀ values of alternately modified siRNA derivatives at different concentrations on LPA in primary human hepatocytes**

| **siRNA ID** | **Inhibition rate (%)** | | | | | | | | **IC₄₀ (nM)** | **IC₅₀ (nM)** |
|---|---|---|---|---|---|---|---|---|---|---|
| | **10 nM** | **3.3 nM** | **1.1 nM** | **0.37 nM** | **0.12 nM** | **0.04 nM** | **14 pM** | **5 pM** | | |
| B02414-AL | 38.0 | 20.6 | 32.1 | 23.3 | 18.0 | 13.4 | -9.1 | 1.2 | - | - |
| B01023-AL | 90.0 | 88.3 | 83.9 | 57.5 | 47.7 | 25.5 | 6.6 | 9.5 | 0.10 | 0.17 |
| B01039-AL | 27.9 | 19.0 | 21.1 | 14.2 | 4.6 | -1.2 | -2.1 | 7.3 | - | - |
| B02412-AL | 26.2 | 17.3 | 15.4 | 13.5 | 2.2 | 9.9 | -4.7 | -11.8 | - | - |
| B01049-AL | 38.9 | 28.0 | 25.6 | 18.7 | 19.3 | 9.9 | -^{c} | 13.0 | 11.91 | - |
| B01040-AL | 53.8 | 31.4 | 25.5 | 22.1 | 13.2 | 6.7 | -3.4 | 8.2 | 4.49 | 8.90 |
| B01001-AL | 57.1 | 47.0 | 25.1 | 31.4 | -0.4 | -5.2 | -7.6 | 4.3 | 1.85 | 4.56 |
| B02417-AL | 70.9 | 57.2 | 37.3 | 38.8 | 25.5 | 24.2 | 7.9 | 16.1 | 0.79 | 2.11 |
| B06001-AL | 72.2 | 59.8 | 26.9 | 33.7 | 22.0 | 0.6 | 0.9 | 15.6 | 1.29 | 2.46 |
| B02401-AL | 38.3 | 52.9 | 17.9 | 23.1 | 11.9 | -5.6 | -1.6 | 6.6 | 3.52 | - |
| B02411-AL | 35.8 | 27.8 | 16.9 | 23.5 | 0.1 | -17.3 | -0.3 | 3.9 | - | - |
| B02434-AL | 42.0 | 25.1 | 21.8 | 15.5 | 20.7 | -1.3 | -6.0 | 7.8 | 9.89 | - |
| B02416-AL | 46.0 | 44.0 | 48.1 | 44.0 | 32.8 | 4.7 | 5.5 | 4.5 | 0.15 | - |
| B01042-AL | 59.6 | 40.6 | 11.1 | 20.3 | 4.9 | 3.9 | 3.4 | 5.0 | 3.83 | 6.14 |
| B01047-AL | 47.0 | 36.7 | 11.1 | 21.4 | 12.9 | -4.2 | -6.9 | 3.7 | 5.89 | 11.86 |
| B01507-AL | 12.3 | 17.6 | 6.5 | 16.0 | 10.2 | 2.1 | 5.9 | 12.7 | - | - |
| B06601-AL | 39.7 | 34.2 | 32.6 | 28.8 | 31.0 | 4.0 | 11.6 | 10.4 | - | - |
| B06026-AL | 22.9 | 23.6 | 30.1 | 13.6 | 6.5 | 6.1 | 9.8 | 16.5 | - | - |
| APC-AL | 40.0 | 32.5 | 16.5 | 10.3 | 11.0 | 4.3 | 17.0 | 15.9 | 10.53 | - |

### Example 4: Inhibitory effect of siRNA derivatives on LPA gene expression

In this example, 17 core sequences screened in Examples 1 and 3, namely B01001, B01012, B01023, B01011, B01040, B01042, B01047, B01801, B02460, B02458, B02416, B02417, B02459, B02457, B02434, B06001, and B06029, were modified using modification templates. Specifically, DV25P, DV26P, DV27P, DV29P, DV32P, DV34P, DV38P, DV25SP, DV26SP, DV27SP, DV29SP, DV32SP, DV34SP, and DV38SP are novel modification templates of the present disclosure; DV22 is the Advanced ESC modification template disclosed in the prior art (Foster, D. J., et al. (2018). "Advanced siRNA Designs Further Improve In Vivo Performance of GalNAc-siRNA Conjugates." Mol Ther 26(3): 708-717); DV22S is a modification template based on DV22 for siRNA derivatives or siRNA conjugates with sense and antisense strands of 20 and 22 nucleotides, respectively.

DV22 antisense strand (5'-3'): From the 5' end of the antisense strand, nucleotides at positions 1, 3, 4, 5, 7, 8, 9, 10, 11, 12, 13, 15, 17, 18, 19, 20, 21, 22, and 23 are 2'-O-methyl-modified nucleotides; nucleotides at positions 2, 6, 14, and 16 are 2'-fluoro-modified nucleotides; and 3',5'-phosphorothioate linkages are present between nucleotides at positions 1, 2, 21, and 22 and their adjacent nucleotides on the right.

DV22 sense strand (5'-3'): From the 5' end of the sense strand, nucleotides at positions 1, 2, 3, 4, 5, 6, 8, 12, 13, 14, 15, 16, 17, 18, 19, 20, and 21 are 2'-O-methyl-modified nucleotides; nucleotides at positions 7, 9, 10, and 11 are 2'-fluoro-modified nucleotides; and 3',5'-phosphorothioate linkages are present between nucleotides at positions 1 and 2 and their adjacent nucleotides on the right.

DV22S antisense strand (5'-3'): From the 5' end of the antisense strand, nucleotides at positions 1, 3, 4, 5, 7, 8, 9, 10, 11, 12, 13, 15, 16, 17, 18, 19, 20, 21, and 22 are 2'-O-methyl-modified nucleotides; nucleotides at positions 2, 6, and 14 are 2'-fluoro-modified nucleotides; and 3',5'-phosphorothioate linkages are present between nucleotides at positions 1, 2, 20, and 21 and their adjacent nucleotides on the right.

DV22S sense strand (5'-3'): From the 5' end of the sense strand, nucleotides at positions 1, 2, 3, 4, 5, 7, 11, 12, 13, 14, 15, 16, 17, 18, 19, and 20 are 2'-O-methyl-modified nucleotides; nucleotides at positions 6, 8, 9, and 10 are 2'-fluoro-modified nucleotides; and 3',5'-phosphorothioate linkages are present between nucleotides at positions 1 and 2 and their adjacent nucleotides on the right.

Natural 5' phosphorylation or simple direct 5' phosphorylation may undergo dephosphorylation in cells. Directly 5'-phosphorylated oligonucleotide strands may undergo 90% dephosphorylation after 2 hours of circulation in blood and complete loss after 24 hours. A 5'-E-VP phosphorylation design was adopted, in which E-vinylphosphonate replaces the bridging oxygen, thereby improving phosphorylation and stability. The antisense strands of the modification templates DV25P to DV27P, DV29P, DV32P, DV34P, DV38P, DV25SP to DV27SP, DV29SP, DV32SP, DV34SP, and DV38SP disclosed herein all contain a 5'-vinylphosphonate group.

### 4.1 Sequence synthesis

The siRNA sequences were synthesized with reference to the method described in Example 1, except that the base at the 5' end of the antisense strand (the last base) was synthesized using a monomer containing a phosphonate group at the 5' end, such as vinyl-(E)-phosphonate-A-OMe phosphoramidite monomer (Formula 9) or vinyl-(E)-phosphonate-U-OMe phosphoramidite monomer (Formula 10), the structures of which are as follows:

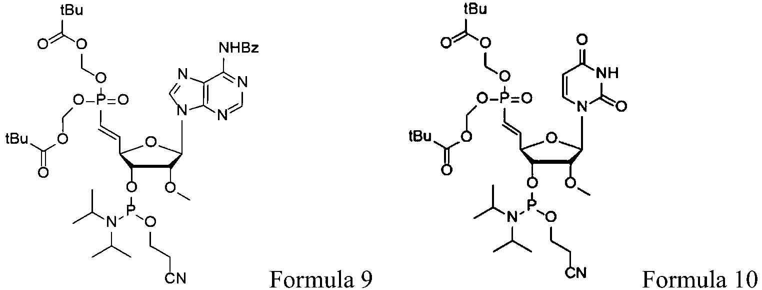

### 4.2 Modification templates DV25P to DV27P, DV29P, DV32P, DV34P, DV38P, DV25SP to DV27SP, DV29SP, DV32SP, DV34SP, and DV38SP

The modification rules for the templates disclosed herein are as follows:
For siRNA derivatives or siRNA conjugates with sense and antisense strands of 21 and 23 nucleotides, respectively:
The antisense strand adopts any one of the modification patterns shown in Table 6:

**Table 6: Modification of antisense strand**

| No. | **Nucleotide position (5'-3')** | | | | | | | | | | | |
|---|---|---|---|---|---|---|---|---|---|---|---|---|
| **A** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **B** | **1** | **2** | **3** | **4** | **5** | **6** | **7** | **8** | **9** | **10** | **11** | **12** |
| | 2'-OMe +PS +EVP | **2'-F +PS** | **2'-F** | **2'-F** | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **C** | **1** | **2** | **3** | **4** | **5** | **6** | **7** | **8** | **9** | **10** | **11** | **12** |
| | 2'-OMe +PS +EVP | **2'-F +PS** | 2'-OMe | **2'-F** | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **D** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **E** | **1** | **2** | **3** | **4** | **5** | **6** | **7** | **8** | **9** | **10** | **11** | **12** |
| | 2'-OMe +PS +EVP | **2'-F +PS** | 2'-OMe | **2'-F** | **2'-F** | 2'-OMe | 2'-OMe | 2'-OMe | **2'-F** | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe | 2'-OMe | 2'-OMe | |
| | | | | | | | | | +PS | +PS | | |
| **F** | **1** | **2** | **3** | **4** | **5** | **6** | **7** | **8** | **9** | **10** | **11** | **12** |
| | 2'-OMe +PS +EVP | **2'-F +PS** | **2'-F** | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |

The sense strand adopts any one of the modification patterns shown in Table 7:

**Table 7: Modification of sense strand**

| No. | **Nucleotide position (5'-3')** | | | | | | | | | | |
|---|---|---|---|---|---|---|---|---|---|---|---|
| **a** | **1** | **2** | **3** | **4** | **5** | **6** | **7** | **8** | **9** | **10** | **11** |
| | 2'-OMe+PS | 2'-OMe+PS | 2'-OMe | 2'-OMe | 2'-OMe | 2'-OMe | **2'-F** | 2'-OMe | **2'-F** | **2'-F** | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | 2'-F | 2'-OMe | 2'-OMe | 2'-OMe | 2'-OMe | |
| **b** | **1** | **2** | **3** | **4** | **5** | **6** | **7** | **8** | **9** | **10** | **11** |
| | 2'-OMe+PS | 2'-OMe+PS | 2'-OMe | 2'-OMe | 2'-OMe | 2'-OMe | **2'-F** | 2'-OMe | **2'-F** | 2'-OMe | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | **2'-F** | 2'-OMe | 2'-OMe | 2'-OMe | 2'-OMe | |

wherein 2'-OMe represents a 2'-O-methyl modification; 2'-F represents a 2'-fluoro modification; PS indicates that, starting from the 5' end, the nucleotide at the position is linked to the adjacent nucleotide on the right via a 3',5'-phosphorothioate bond.

The siRNA modification template, in which the antisense strand was modified according to modification pattern A and the sense strand was modified according to modification pattern a, was designated as DV25P.

The siRNA modification template, in which the antisense strand was modified according to modification pattern B and the sense strand was modified according to modification pattern a, was designated as DV26P.

The siRNA modification template, in which the antisense strand was modified according to modification pattern C and the sense strand was modified according to modification pattern a, was designated as DV27P.

The siRNA modification template, in which the antisense strand was modified according to modification pattern C and the sense strand was modified according to modification pattern b, was designated as DV29P.

The siRNA modification template, in which the antisense strand was modified according to modification pattern D and the sense strand was modified according to modification pattern b, was designated as DV32P.

The siRNA modification template, in which the antisense strand was modified according to modification pattern E and the sense strand was modified according to modification pattern b, was designated as DV38P.

The siRNA modification template, in which the antisense strand was modified according to modification pattern F and the sense strand was modified according to modification pattern b, was designated as DV34P.

For siRNA derivatives or siRNA conjugates with sense and antisense strands of 20 and 22 nucleotides, respectively:

The antisense strand adopts any one of the modification patterns shown in Table 8:

**Table 8: Modification of antisense strand**

| No. | **Nucleotide position (5'-3')** | | | | | | | | | | |
|---|---|---|---|---|---|---|---|---|---|---|---|
| **A'** | **1** | **2** | **3** | **4** | **5** | **6** | **7** | **8** | **9** | **10** | **11** |
| | 2'-OMe +PS +EVP | **2'-F +PS** | 2'-OMe | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| | **1** | **2** | **3** | **4** | **5** | **6** | **7** | **8** | **9** | **10** | **11** |
| **B'** | 2'-OMe +PS +EVP | **2'-F +PS** | **2'-F** | **2'-F** | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **C'** | **1** | **2** | **3** | **4** | **5** | **6** | **7** | **8** | **9** | **10** | **11** |
| | 2'-OMe +PS +EVP | **2'-F +PS** | 2'-OMe | **2'-F** | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **D'** | **1** | **2** | **3** | **4** | **5** | **6** | **7** | **8** | **9** | **10** | **11** |
| | 2'-OMe +PS +EVP | **2'-F +PS** | 2'-OMe | 2'-OMe | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **E'** | **1** | **2** | **3** | **4** | **5** | **6** | **7** | **8** | **9** | **10** | **11** |
| | 2'-OMe +PS +EVP | **2'-F +PS** | 2'-OMe | **2'-F** | **2'-F** | 2'-OMe | 2'-OMe | 2'-OMe | **2'-F** | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **F'** | **1** | **2** | **3** | **4** | **5** | **6** | **7** | **8** | **9** | **10** | **11** |
| | 2'-OMe +PS +EVP | **2'-F +PS** | **2'-F** | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |

The sense strand adopts any one of the modification patterns shown in Table 9:

**Table 9: Modification of sense strand**

| No. | **Nucleotide position (5'-3')** | | | | | | | | | |
|---|---|---|---|---|---|---|---|---|---|---|
| **a'** | **1** | **2** | **3** | **4** | **5** | **6** | **7** | **8** | **9** | **10** |
| | 2'-OMe+PS | 2'-OMe+PS | 2'-OMe | 2'-OMe | 2'-OMe | **2'-F** | 2'-OMe | **2'-F** | **2'-F** | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | 2'-F | 2'-OMe | 2'-OMe | 2'-OMe | 2'-OMe |
| **b'** | **1** | **2** | **3** | **4** | **5** | **6** | **7** | **8** | **9** | **10** |
| | 2'-OMe+PS | 2'-OMe+PS | 2'-OMe | 2'-OMe | 2'-OMe | **2'-F** | 2'-OMe | **2'-F** | 2'-OMe | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | **2'-F** | 2'-OMe | 2'-OMe | 2'-OMe | 2'-OMe |

wherein 2'-OMe represents a 2'-O-methyl modification; 2'-F represents a 2'-fluoro modification; PS indicates that, starting from the 5' end, the nucleotide at the position is linked to the adjacent nucleotide on the right via a 3',5'-phosphorothioate bond.

The siRNA modification template, in which the antisense strand was modified according to modification pattern A' and the sense strand was modified according to modification pattern a', was designated as DV25SP.

The siRNA modification template, in which the antisense strand was modified according to modification pattern B' and the sense strand was modified according to modification pattern a', was designated as DV26SP.

The siRNA modification template, in which the antisense strand was modified according to modification pattern C' and the sense strand was modified according to modification pattern a', was designated as DV27SP.

The siRNA modification template, in which the antisense strand was modified according to modification pattern C' and the sense strand was modified according to modification pattern b', was designated as DV29SP.

The siRNA modification template, in which the antisense strand was modified according to modification pattern D' and the sense strand was modified according to modification pattern b', was designated as DV32SP.

The siRNA modification template, in which the antisense strand was modified according to modification pattern E' and the sense strand was modified according to modification pattern b', was designated as DV38SP.

The siRNA modification template, in which the antisense strand was modified according to modification pattern F' and the sense strand was modified according to modification pattern b', was designated as DV34SP.

The template-modified siRNA derivatives are listed in Table 10, which were synthesized with reference to the method described in Example 1.

The inhibitory effects of the template-modified siRNA derivatives listed in Table 10 on LPA gene expression were evaluated using two methods: a dual-luciferase assay and a transfection assay in primary human hepatocytes.

### 4.3 Dual-luciferase assay for evaluation of inhibitory effect of template-modified siRNA derivatives on LPA gene expression

A total of 120 template-modified siRNA derivatives listed in Table 10 were evaluated for their inhibitory effects on LPA gene expression using a dual-luciferase assay. Specifically, the positive control APC-OL is the original modified sequence of Olpasiran without the GalNAc moiety, and the negative control ANC-DV29P is a nonsense sequence modified using the template DV29.

The experimental materials and methods were the same as those described in Sections 1.2.1 and 1.2.2 of Example 1.

Three independent experiments were performed in triplicate for each test substance. The inhibition rates of the template-modified siRNA derivatives on LPA mRNA expression are shown in Table 10 below.

**Table 10: Inhibition of template-modified siRNA derivatives on LPA gene expression**

| **siRNA ID** | SEO ID NO of sense motif | Sense modification pattern | SEQ ID NO of antisense motif | Antisense modification pattern | Inhibition rate (%) |
|---|---|---|---|---|---|
| C01001-DV25P | 1 | a | 194 | A | 81.9 |
| C01001-DV26P | 1 | a | 194 | B | 81.1 |
| C01001-DV27P | 1 | a | 194 | C | 83.7 |
| C01001-DV29P | 1 | b | 194 | C | 64.6 |
| C01001-DV32P | 1 | b | 194 | D | 61.7 |
| C01001-DV22 | 1 | | 194 | | 62.5 |
| C01012-DV25P | 186 | a | 379 | A | 79.7 |
| C01012-DV26P | 186 | a | 379 | B | 77.0 |
| C01012-DV27P | 186 | a | 379 | C | 77.5 |
| C01012-DV29P | 186 | b | 379 | C | 81.8 |
| C01012-DV32P | 186 | b | 379 | D | 80.3 |
| C01012-DV34P | 186 | b | 379 | F | 81.2 |
| C01012-DV38P | 186 | b | 379 | E | 80.7 |
| C01012-DV22 | 186 | | 379 | | 75.8 |
| C01023-DV25P | 21 | a | 214 | A | 81.2 |
| C01023-DV26P | 21 | a | 214 | B | 83.6 |
| C01023-DV27P | 21 | a | 214 | C | 85.3 |
| C01023-DV29P | 21 | b | 214 | C | 74.0 |
| C01023-DV32P | 21 | b | 214 | D | 73.7 |
| C01023-DV34P | 21 | b | 214 | F | 59.9 |
| C01023-DV38P | 21 | b | 214 | E | 73.8 |
| C01023-DV22 | 21 | | 214 | | 70.4 |
| C01011-DV25P | 187 | a | 380 | A | 68.4 |
| C01011-DV26P | 187 | a | 380 | B | 66.2 |
| C01011-DV27P | 187 | a | 380 | C | 70.3 |
| C01011-DV29P | 187 | b | 380 | C | 69.1 |
| C01011-DV32P | 187 | b | 380 | D | 63.9 |
| C01011-DV34P | 187 | b | 380 | F | 62.8 |
| C01011-DV38P | 187 | b | 380 | E | 70.1 |
| C01011-DV22 | 187 | | 380 | | 64.1 |
| C01040-DV25P | 37 | a | 230 | A | 69.7 |
| C01040-DV26P | 37 | a | 230 | B | 71.6 |
| C01040-DV27P | 37 | a | 230 | C | 78.8 |
| C01040-DV29P | 37 | b | 230 | C | 65.0 |
| C01040-DV32P | 37 | b | 230 | D | 53.5 |
| C01040-DV22 | 37 | | 230 | | 59.6 |
| C01042-DV25SP | 39 | a' | 232 | A' | 81.6 |
| C01042-DV26SP | 39 | a' | 232 | B' | 80.0 |
| C01042-DV27SP | 39 | a' | 232 | C' | 81.6 |
| C01042-DV29SP | 39 | b' | 232 | C' | 78.9 |
| C01042-DV32SP | 39 | b' | 232 | D' | 75.9 |
| C01042-DV22S | 39 | | 232 | | 74.8 |
| C01047-DV25SP | 44 | a' | 237 | A' | 89.6 |
| C01047-DV26SP | 44 | a' | 237 | B' | 91.5 |
| C01047-DV27SP | 44 | a' | 237 | C' | 88.5 |
| C01047-DV29SP | 44 | b' | 237 | C' | 87.6 |
| C01047-DV32SP | 44 | b' | 237 | D' | 85.6 |
| C01047-DV22S | 44 | | 237 | | 86.8 |
| C01801-DV25P | 184 | a | 377 | A | 75.1 |
| C01801-DV26P | 184 | a | 377 | B | 76.0 |
| C01801-DV27P | 184 | a | 377 | C | 78.0 |
| C01801-DV29P | 184 | b | 377 | C | 68.6 |
| C01801-DV32P | 184 | b | 377 | D | 66.0 |
| C01801-DV22 | 184 | | 377 | | 64.1 |
| C02460-DV25P | 191 | a | 384 | A | 56.7 |
| C02460-DV26P | 191 | a | 384 | B | 62.9 |
| C02460-DV27P | 191 | a | 384 | C | 58.6 |
| C02460-DV29P | 191 | b | 384 | C | 50.6 |
| C02460-DV32P | 191 | b | 384 | D | 52.8 |
| C02460-DV34P | 191 | b | 384 | F | 56.6 |
| C02460-DV38P | 191 | b | 384 | E | 50.6 |
| C02460-DV22 | 191 | | 384 | | 52.5 |
| C02458-DV25P | 189 | a | 382 | A | 56.9 |
| C02458-DV26P | 189 | a | 382 | B | 59.8 |
| C02458-DV27P | 189 | a | 382 | C | 60.5 |
| C02458-DV29P | 189 | b | 382 | C | 59.6 |
| C02458-DV32P | 189 | b | 382 | D | 54.5 |
| C02458-DV22 | 189 | | 382 | | 55.7 |
| C02416-DV25P | 75 | a | 268 | A | 65.3 |
| C02416-DV26P | 75 | a | 268 | B | 60.9 |
| C02416-DV27P | 75 | a | 268 | C | 65.1 |
| C02416-DV29P | 75 | b | 268 | C | 64.1 |
| C02416-DV32P | 75 | b | 268 | D | 60.5 |
| C02416-DV22 | 75 | | 268 | | 61.8 |
| C02417-DV25P | 76 | a | 269 | A | 66.1 |
| C02417-DV26P | 76 | a | 269 | B | 62.5 |
| C02417-DV27P | 76 | a | 269 | C | 68.6 |
| C02417-DV29P | 76 | b | 269 | C | 67.5 |
| C02417-DV32P | 76 | b | 269 | D | 69.5 |
| C02417-DV34P | 76 | b | 269 | F | 62.9 |
| C02417-DV38P | 76 | b | 269 | E | 70.6 |
| C02417-DV22 | 76 | | 269 | | 63.4 |
| C02459-DV25P | 190 | a | 383 | A | 55.7 |
| C02459-DV26P | 190 | a | 383 | B | 59.2 |
| C02459-DV27P | 190 | a | 383 | C | 55.8 |
| C02459-DV29P | 190 | b | 383 | C | 33.4 |
| C02459-DV32P | 190 | b | 383 | D | 20.8 |
| C02459-DV34P | 190 | b | 383 | F | 23.8 |
| C02459-DV38P | 190 | b | 383 | E | 36.4 |
| C02459-DV22 | 190 | | 383 | | 41.2 |
| C02457-DV25P | 188 | a | 381 | A | 57.3 |
| C02457-DV26P | 188 | a | 381 | B | 63.4 |
| C02457-DV27P | 188 | a | 381 | C | 55.2 |
| C02457-DV29P | 188 | b | 381 | C | 42.9 |
| C02457-DV32P | 188 | b | 381 | D | 36.5 |
| C02457-DV22 | 188 | | 381 | | 45.7 |
| C02434-DV25P | 92 | a | 285 | A | 60.0 |
| C02434-DV26P | 92 | a | 285 | B | 74.2 |
| C02434-DV27P | 92 | a | 285 | C | 76.5 |
| C02434-DV29P | 92 | b | 285 | C | 35.1 |
| C02434-DV32P | 92 | b | 285 | D | 22.7 |
| C02434-DV22 | 92 | | 285 | | 36.5 |
| C06001-DV25P | 132 | a | 325 | A | 70.2 |
| C06001-DV26P | 132 | a | 325 | B | 72.4 |
| C06001-DV27P | 132 | a | 325 | C | 71.9 |
| C06001-DV29P | 132 | b | 325 | C | 69.0 |
| C06001-DV32P | 132 | b | 325 | D | 67.5 |
| C06001-DV34P | 132 | b | 325 | F | 70.3 |
| C06001-DV38P | 132 | b | 325 | E | 69.8 |
| C06001-DV22 | 132 | | 325 | | 65.2 |
| C06029-DV25P | 192 | a | 385 | A | 55.7 |
| C06029-DV26P | 192 | a | 385 | B | 57.8 |
| C06029-DV27P | 192 | a | 385 | C | 67.0 |
| C06029-DV29P | 192 | b | 385 | C | 22.5 |
| C06029-DV32P | 192 | b | 385 | D | 16.8 |
| C06029-DV34P | 192 | b | 385 | F | 10.9 |
| C06029-DV38P | 192 | b | 385 | E | 40.8 |
| C06029-DV22 | 192 | | 385 | | 55.4 |
| APC-OL | / | / | / | / | 60.7 |
| ANC-DV29P | 193 | b | 386 | C | 0.0 |

### APC-OL:

Sense strand (SEQ ID NO: 389): Cms-Ams-Gm-Cm-Cm-Cm-Cm-Um-Uf-Af-Uf-Um-Gm-Um-Um-Am-Um-Am-Cm-Gms-d'A
Antisense strand (SEQ ID NO: 390): Ums-Cfs-Gm-Uf-Am-Uf-Am-Am-Cm-Am-Am-Uf-Am-Af-Gm-Gf-Gm-Gf-Cms-Ufs-Gm
d'A: DNA linked via a 3'-3' phosphodiester bond.

The results demonstrated that:
(1) The 17 selected siRNA motifs B01001, B01012, B01023, B01011, B01040, B01042, B01047, B01801, B02460, B02458, B02416, B02417, B02459, B02457, B02434, B06001, and B06029 were modified using the templates DV25P to DV27P, DV29P, DV32P, DV34P, DV38P, DV25SP to DV27SP, DV29SP, DV32SP, DV34SP, and DV38SP. The resulting siRNA derivatives exhibited significant inhibitory effects on LPA gene expression. A total of 91 template-modified siRNA derivatives exhibited inhibition rates exceeding 40%. For example, C01023-DV25P, C02417-DV38P, and C01801-DV27P exhibited inhibition rates of 81.2%, 78.0%, and 70.6%, respectively.
(2) The 17 sequences listed above modified using the templates DV25P to DV27P, DV29P, DV32P, DV34P, DV38P, DV25SP to DV27SP, DV29SP, DV32SP, DV34SP, and DV38SP disclosed herein showed significantly enhanced inhibition on LPA gene expression compared to the alternately modified sequences. For example, for the siRNA motif B01023 modified using the template DV25P, the resulting siRNA derivative C01023-DV25P exhibited a 9.9% increase in inhibition compared to the alternately modified derivative; for the siRNA motif B02417 modified using the template DV38P, the resulting siRNA derivative C02417-DV38P exhibited a 15.7% increase in inhibition compared to the alternately modified derivative.
(3) For identical sequences, modification using the templates DV25P to DV27P, DV29P, DV32P, DV34P, DV38P, DV25SP to DV27SP, DV29SP, DV32SP, DV34SP, and DV38SP disclosed herein showed significantly enhanced inhibition on LPA gene expression compared to modification using the template disclosed in the prior art. For example, the siRNA motif B01023 modified using the template DV25P disclosed herein exhibited a 10.8% increase in inhibition compared to that modified using the disclosed Advanced ESC template DV22; the siRNA motif B02417 modified using the template DV38P disclosed herein exhibited a 7.2% increase in inhibition compared to that modified using the disclosed Advanced ESC template DV22.
(4) The sequences modified using different templates varied greatly in activity. For example, the siRNA motif B01023 modified with DV25P exhibited a 21.3% increase in inhibition compared to that modified with DV34; the siRNA motif B01801 modified with DV27P exhibited a 12.0% increase in inhibition compared to that modified with DV32P. Thus, it is uncertain which modification template would confer high activity to an siRNA sequence.

### 4.4 Transfection assay for evaluation of inhibitory effect of template-modified sequences on LPA gene expression in primary human hepatocytes

Among the 101 template-modified sequences listed in Table 10, those with relatively high activity (see Table 11) were evaluated for their inhibitory effects on LPA gene expression using a transfection assay in primary human hepatocytes.

These sequences were transfected into primary human hepatocytes using Lipofectamine RNAiMAX transfection reagent. The inhibitory effects of each sequence on LPA gene expression were measured by qPCR, and dose-response curves were fitted to calculate IC₅₀ values. The siRNA transfection was performed starting at a concentration of 2.5 nM, followed by 4-fold serial dilutions across six concentrations (2.5 nM, 0.625 nM, 0.156 nM, 0.039 nM, 0.01 nM, and 0.002 nM).

The experimental materials and methods were the same as those described in Sections 3.2.1 and 3.2.2 of Example 3.

After three independent qPCR experiments, the inhibition rates of the sequences at different concentrations on LPA gene expression in primary human hepatocytes are shown in Table 11 below. All of the sequences modified using the templates disclosed herein exhibited certain inhibitory effects on LPA, among which 19 sequences showed IC₅₀ values ranging from 0.016 to 0.417 nM, indicating that these sequences can effectively inhibit LPA gene expression at low concentrations. Some of the sequences exhibited superior IC₅₀ values compared to the positive control APC-OL (the original modified sequence of Olpasiran). For example, C01023-DV25P exhibited an IC₅₀ value of 0.025 nM, which was lower than that of the positive control (2.178 nM).

For identical sequences, modification using the templates DV25P to DV27P, DV29P, DV32P, DV34P, DV38P, DV25SP to DV27SP, DV29SP, DV32SP, DV34SP, and DV38SP disclosed herein showed significantly enhanced inhibition on LPA gene expression and lower IC₅₀ values compared to modification using the Advanced ESC template DV22 disclosed in the prior art. For example, the siRNA motif B01023 modified using the template DV25P disclosed herein exhibited a 13.2% increase in inhibition against LPA in primary human hepatocytes and a decrease in IC₅₀ from 0.067 nM to 0.025 nM compared to that modified using the disclosed Advanced ESC template DV22; the siRNA motif B02417 modified using the template DV38P disclosed herein exhibited a 10.8% increase in inhibition against LPA in primary human hepatocytes and a decrease in IC₅₀ from 0.777 nM to 0.041 nM compared to that modified using the disclosed Advanced ESC template DV22.

**Table 11: Inhibition rates and IC₅₀ values of template-modified siRNA derivatives at different concentrations on LPA in primary human hepatocytes**

| **siRNA ID** | **Inhibition rate (%)** | | | | | | **IC₅₀/nM** |
|---|---|---|---|---|---|---|---|
| | **2.5 nM** | **0.625 nM** | **0.156 nM** | **0.039 nM** | **0.01 nM** | **0.002 nM** | |
| C01001-DV25P | 63.1 | 64.0 | 69.4 | 50.5 | 31.8 | 22.9 | 0.034 |
| C01001-DV26P | 51.0 | 64.2 | 55.6 | 38.9 | 33.1 | 16.7 | 0.085 |
| C01001-DV27P | 65.2 | 69.5 | 71.5 | 43.1 | 30.7 | 6.1 | 0.038 |
| C01001-DV22 | 53.4 | 55.2 | 50.7 | 36.8 | 28.5 | 12.7 | 0.198 |
| C01012-DV29P | 58.2 | 54.1 | 48.2 | 16.8 | 16.6 | -12.3 | 0.417 |
| C01012-DV34P | 54.4 | 49.6 | 43.9 | 23.8 | 5.1 | 4.1 | 0.384 |
| C01012-DV38P | 54.7 | 56.8 | 50.5 | 21.8 | 11.3 | -8.6 | 0.240 |
| C01012-DV22 | 52.8 | 47.4 | 42.5 | 22.4 | 7.5 | -4.7 | 0.821 |
| C01023-DV25P | 53.6 | 63.2 | 68.0 | 50.9 | 37.8 | 22.2 | 0.025 |
| C01023-DV26P | 68.1 | 68.9 | 66.0 | 60.9 | 41.6 | 21.1 | 0.016 |
| C01023-DV27P | 61.5 | 61.0 | 58.5 | 48.0 | 28.0 | 9.4 | 0.046 |
| C01023-DV22 | 52.0 | 54.8 | 54.2 | 45.6 | 27.1 | 3.5 | 0.067 |
| C01011-DV27P | 54.6 | 51.8 | 40.9 | -1.4 | 17.4 | -8.1 | 0.282 |
| C01011-DV22 | 52.5 | 47.5 | 35.4 | -2.5 | 4.6 | -8.9 | 0.682 |
| C01042-DV25SP | 66.9 | 65.4 | 54.6 | 26.8 | 21.0 | -4.0 | 0.138 |
| C01042-DV27SP | 64.4 | 68.8 | 51.6 | 44.8 | 27.0 | 7.8 | 0.074 |
| C01042-DV22S | 53.4 | 56.2 | 45.6 | 29.8 | 24.6 | 6.2 | 0.376 |
| C01047-DV25SP | 67.5 | 58.2 | 55.7 | 25.8 | 27.3 | 15.5 | 0.120 |
| C01047-DV26SP | 71.6 | 63.2 | 62.7 | 45.8 | 36.0 | 22.2 | 0.048 |
| C01047-DV27SP | 64.6 | 66.4 | 57.9 | 39.1 | 32.9 | 17.7 | 0.075 |
| C01047-DV29SP | 58.6 | 68.3 | 72.5 | 56.9 | 39.1 | 25.9 | 0.020 |
| C01047-DV22S | 62.1 | 56.4 | 53.2 | 25.9 | 25.4 | 7.2 | 0.199 |
| C01801-DV27P | 64.9 | 66.2 | 61.9 | 55.2 | 39.6 | 19.2 | 0.022 |
| C01801-DV22 | 53.8 | 53.2 | 52.6 | 43.0 | 30.1 | 6.6 | 0.114 |
| C02417-DV38P | 61.1 | 60.4 | 63.3 | 46.7 | 35.4 | 36.0 | 0.041 |
| C02417-DV22 | 52.5 | 51.2 | 40.5 | 25.4 | 17.1 | 2.1 | 0.777 |
| C06001-DV26P | 58.9 | 61.8 | 55.2 | 39.3 | 35.7 | 10.6 | 0.082 |
| C06001-DV22 | 52.6 | 56.3 | 45.4 | 29.3 | 12.8 | 9.5 | 0.241 |
| C01040-DV27P | 48.1 | 48.4 | 29.1 | 28.0 | 25.1 | 2.4 | - |
| C02434-DV27P | 32.3 | 34.8 | 42.0 | 35.6 | 26.7 | 10.8 | - |
| APC-OL | 53.5 | 26.6 | 18.2 | -9.1 | 30.6 | 12.9 | 2.178 |

### Example 5: Comparison of inhibitory effect of sequences disclosed in the prior art on LPA gene expression

In this example, for eight siRNA motifs with relatively high activity disclosed herein, namely B01001, B01012, B01023, B01042, B01047, B01801, B02417, and B06001, their corresponding alternately modified derivatives and derivatives modified using the templates DV25P to DV27P, DV29P, DV32P, DV34P, DV38P, DV25SP to DV27SP, DV29SP, DV32SP, DV34SP, and DV38SP were compared with the siRNA motifs disclosed in the prior art that are similar to the eight sequences and their corresponding template-modified sequences, in terms of inhibition on LPA gene expression.

### 1. Experimental materials

### Test samples:

(1) Unmodified motifs and modified derivatives disclosed in the prior art, as shown in Table 12.

**Table 12: Sequences disclosed in the prior art**

| **siRNA ID** | **Sense strand (5'-3')** | **SEQ ID NO.** | **Antisense strand (5'-3')** | **SEQ ID NO.** | **Source** |
|---|---|---|---|---|---|
| B01023P | GACAGAGUUAUCAAG GCACAUACTT | 391 | AAGUAUGUGCCUUGAU AACUCUGUCCA | 405 | US20240401053 A1 |
| B01001P | CUACCAUGGUAAUGG ACAGAGUUAT | 392 | AUAACUCUGUCCAUUA CCAUGGUAGCA | 406 | US20240401053 A1 |
| B01801P | GCACAGAGGCUCCUU CUGAACAAGC | 393 | GCUUGUUCAGAAGGAG CCUCUGUGCUU | 407 | US20240401053 A1 |
| B01042P | UGCUACCAUGGUAAU GGACAGAGTT | 394 | AACUCUGUCCAUUACC AUGGUAGCACU | 408 | US20240401053 A1 |
| B02417P | AUCACAGGAAGAACA UGUCAGUCTT | 395 | AAGACUGACAUGUUCU UCCUGUGAUAG | 409 | US20240401053 A1 |
| B01012P | GGACAGAGUUAUCAA GGCAAAUACT | 396 | AGUAUUUGCCUUGAUA ACUCUGUCCAU | 410 | US20240401053 A1 |
| B06001P1 | UACUGCAACCUGACG CGAUACUCAG | 397 | CUGAGUAUCGCGUCAG GUUGCAGUACU | 411 | US20240401053 A1 |
| B06001P2 | AGUACUGCAACCUGA CGCGAU | 398 | AUCGCGUCAGGUUGCA GUACUCC | 412 | WO2025007872 A1 |
| B01047P1 | GGAAGGACCUGCCAA GCUU | 399 | AUAACUCUGUCCAUUA CCAUGGU | 413 | CN115851723A |
| B01047P1 M1 | CsAsUGGUfAAfUfGfGA CAGAGUUsAsU | 400 | AsfUsAACUCUGUCCAfU UACCAUGsGsU | 414 | CN115851723A |
| B01047P2 | CAUGGUAAUGGACAG AGUUAUCGAG | 401 | CUCGAUAACUCUGUCC AUUACCAUGGU | 415 | US20240401053 A1 |
| B01047P3 | CAUGGUAAUGGACAG AGUUAU | 402 | AUAACUCUGUCCAUUA CCAUGGU | 416 | US20240271140 A1 |
| B01047P3 M1 | CmsAmsUmGmGmUmAf AmUfGfGfAmCmAmGm AmGmUmUmAmUm | 403 | AmsUfsAmAmCfUmCmU mGmUmCmCmAmUfUmA fCmCmAmUmGmsGmsUm | 417 | US20240271140 A1 |
| B01047P3 M2 | CmsAmsUmGmGmUmA mAmUfGfGfAmCmAmG mAmGmUmUmAmUms | 404 | AmsUfsAmAmCfUmCmU mGmUmCmCmAmUfUmA fCmCmAmUmGmsGmsUm | 418 | US20240271140 A1 |

(2) Sequences disclosed herein that are similar to the sequences disclosed in the prior art listed in Table 12, including the derivatives using alternating 2'-OMe and 2'-F modifications described in Example 2, the siRNA sequences with terminal phosphorothioate modifications, the unmodified siRNA sequences described in Example 1, and the siRNA sequences modified using the templates DV25P to DV27P, DV29P, DV32P, DV34P, DV38P, DV25SP to DV27SP, DV29SP, DV32SP, DV34SP, and DV38SP described in Example 4, with their IDs listed in Table 13.

### 2. Experimental methods and results

The inhibitory effects of the siRNA motifs and modified sequences listed above on LPA gene expression at the cellular level were evaluated using a dual-luciferase assay.

The experimental materials and methods were the same as those described in Sections 1.2.1 and 1.2.2 of Example 1.

The experimental results are shown in Table 13.

**Table 13: Inhibition rates of similar siRNA sequences disclosed herein and in the prior art on LPA gene expression**

| **siRNA motif in the prior art** | | **siRNA motif disclosed herein** | | | **Alternately modified siRNA derivative disclosed herein** | | | **Template-modified derivative disclosed siRNAherein** | | |
|---|---|---|---|---|---|---|---|---|---|---|
| **Name** | **Inh%** | **siRNA ID** | **Inh%** | **Increase in inhibition vs. siRNA motif in the prior art (%)** | **siRNA ID** | **Inh%** | **Increase in inhibition vs. siRNA motif in the prior art (%)** | **siRNA ID** | **Inh%** | **Increase in inhibition vs. siRNA motif in the prior art (%)** |
| B01023P | 45.2 | B01023 | 67.9 | 22.7 | B01023-AL | 71.3 | 26.1 | C01023-DV25P | 81.2 | 36.0 |
| | | | | | | | | C01023-DV26P | 83.6 | 38.4 |
| | | | | | | | | C01023-DV27P | 85.3 | 40.1 |
| | | | | | | | | C01023-DV29P | 74.0 | 28.8 |
| | | | | | | | | C01023-DV32P | 73.7 | 28.5 |
| | | | | | | | | C01023-DV34P | 59.9 | 14.7 |
| | | | | | | | | C01023-DV38P | 73.8 | 28.6 |
| B01001P | 51.3 | B01001 | 56.2 | 4.9 | B01001-AL | 64.0 | 12.7 | C01001-DV25P | 81.9 | 30.6 |
| | | | | | | | | C01001-DV26P | 81.1 | 29.8 |
| | | | | | | | | C01001-DV27P | 83.7 | 32.4 |
| | | | | | | | | C01001-DV29P | 64.6 | 13.3 |
| | | | | | | | | C01001-DV32P | 61.7 | 10.4 |
| B01801P | 42.5 | B01801 | 55.6 | 13.1 | B01801-AL | 52.3 | 9.8 | C01801-DV25P | 75.1 | 32.6 |
| | | | | | | | | C01801-DV26P | 76.0 | 33.5 |
| | | | | | | | | C01801-DV27P | 78.0 | 35.5 |
| | | | | | | | | C01801-DV29P | 68.6 | 26.1 |
| | | | | | | | | C01801-DV32P | 66.0 | 23.5 |
| B01042P | 47.1 | B01042 | 55.3 | 8.2 | B01042-AL | 53.0 | 5.9 | C01042-DV25SP | 81.6 | 34.5 |
| | | | | | | | | C01042-DV26SP | 80.0 | 32.9 |
| | | | | | | | | C01042-DV27SP | 81.6 | 34.5 |
| | | | | | | | | C01042-DV29SP | 78.9 | 31.8 |
| | | | | | | | | C01042-DV32SP | 75.9 | 28.8 |
| B02417P | 42.2 | B02417 | 58.9 | 16.7 | B02417-AL | 54.9 | 12.7 | C02417-DV25P | 66.1 | 23.9 |
| | | | | | | | | C02417-DV26P | 62.5 | 20.3 |
| | | | | | | | | C02417-DV27P | 68.6 | 26.4 |
| | | | | | | | | C02417-DV29P | 67.5 | 25.3 |
| | | | | | | | | C02417-DV32P | 69.5 | 27.3 |
| | | | | | | | | C02417-DV34P | 62.9 | 20.7 |
| | | | | | | | | C02417-DV38P | 70.6 | 28.4 |
| B01012P | 48.3 | B01012 | 67.6 | 19.3 | B01012-AL | 70.9 | 22.6 | C01012-DV25P | 79.7 | 31.4 |
| | | | | | | | | C01012-DV26P | 77.0 | 28.7 |
| | | | | | | | | C01012-DV27P | 77.5 | 29.2 |
| | | | | | | | | C01012-DV29P | 81.8 | 33.5 |
| | | | | | | | | C01012-DV32P | 80.3 | 32.0 |
| | | | | | | | | C01012-DV34P | 81.2 | 32.9 |
| | | | | | | | | C01012-DV38P | 80.7 | 32.4 |
| B06001P1 | 45.3 | B06001 | 57.5 | 12.2 | B06001-AL | 51.3 | 6.0 | C06001-DV25P | 70.2 | 24.9 to 35.0 |
| | | | | | | | | C06001-DV26P | 72.4 | 27.1 to 37.2 |
| | | | | | | | | C06001-DV27P | 71.9 | 26.6 to 36.7 |
| B06001P2 | 35.2 | | | 22.3 | | | 16.1 | C06001-DV29P | 69.0 | 23.7 to 33.8 |
| | | | | | | | | C06001-DV32P | 67.5 | 22.2 to 32.3 |
| | | | | | | | | C06001-DV34P | 70.3 | 25.0 to 35.1 |
| | | | | | | | | C06001-DV38P | 69.8 | 24.5 to 34.6 |
| B01047P1 | 42.1 | B01047 | 47.4 | 5.3 | B01047-AL | 50.7 | 8.6 | C01047-DV25SP | 89.6 | 17.5 to 44.0 |
| B01047P1 M1 | 62.4 | | | | | | | C01047-DV26SP | 91.5 | 19.4 to 45.9 |
| B01047P2 | 43.2 | | | 4.2 | | | 7.5 | C01047-DV27SP | 88.5 | 16.4 to 42.9 |
| B01047P3 | 45.6 | | | 1.8 | | | 5.1 | C01047-DV29SP | 87.6 | 15.5 to 42.0 |
| B01047P3 M1 | 70.2 | | | | | | | C01047-DV32SP | 85.6 | 13.5 to 40.0 |
| B01047P3 M2 | 72.1 | | | | | | | | | |

The experimental results demonstrated that the siRNA motifs, the alternately modified siRNA derivatives, and the siRNA derivatives modified using the specific modification templates disclosed herein exhibited significantly enhanced inhibitory activity against LPA compared to similar siRNA motifs disclosed in the prior art. For example, compared to B01023 disclosed herein, B01023P disclosed in the prior art contains four additional bases (ACTT) at the 3' end of the sense strand and four additional bases (AAGU) at the 5' end of the antisense strand. However, B01023 disclosed herein exhibited a 22.7% increase in inhibition compared to B01023P; the alternately modified derivative B01023-AL exhibited an inhibition rate of 71.3%, which was 26.1% higher than that of B01023P; the derivative C01023-DV25P modified using the template DV25 disclosed herein exhibited an inhibition rate of 81.2%, which was 36.0% higher than that of B01023P.

### Example 6: Off-target effects of siRNA derivatives

In practical applications of siRNA, there may be unintended suppression of the expression of non-target mRNAs that are partially complementary to the antisense strand. Studies have shown that the hepatotoxicity of N-acetylgalactosamine (GalNAc)-conjugated siRNA is primarily attributed to the inhibition of unintended targets caused by off-target effects.

In this example, the siRNA derivatives with relatively high activity identified in Example 4 were evaluated for their inhibitory effects on potential off-target genes in primary human hepatocytes (PHHs), and were compared with Olpasiran.

### 1. Experimental materials

### 1) Test samples:

A total of 11 siRNA derivatives with relatively high activity identified in Example 4 and the positive control APC-OL (see Table 14), wherein APC-OL represents the corresponding sequence of Olpasiran.

### 2. Experimental methods

The inhibitory rates of the test samples on the expression of the LPA gene and potential off-target genes in PHHs were measured by qRT-PCR. Specifically, after the derivatives were transfected into PHHs using Lipofectamine RNAiMAX transfection reagent, the inhibition rates of each test sample against the LPA gene and potential off-target genes were measured by qRT-PCR.

The experimental materials and methods for thawing of PHHs, siRNA transfection, RNA extraction, and reverse transcription were the same as those described in Section 3.2.2 of Example 3. The target gene LPA and the reference gene GAPDH were amplified by qPCR using a probe-based method, as described in Section 3.2.2 of Example 3. The potential off-target genes listed in Table 14 were amplified by qPCR according to the instructions for TB Green Premix Ex Taq (Takara, RR420W).

### 3. Experimental results

The inhibition rates and IC₅₀ values of the siRNA derivatives at concentrations of 40 nM, 10 nM, 2.5 nM, and 0.625 nM against the LPA gene and potential off-target genes are shown in Table 14.

**Table 14: Inhibition rates and IC₅₀ values of siRNA derivatives at different concentrations against LPA and potential off-target genes**

| **siRNA ID** | **Gene name** | **Inhibition rate (%)** | | | | **IC₅₀/nM** | **Ratio** |
|---|---|---|---|---|---|---|---|
| | | **40 nM** | **10 nM** | **2.5 nM** | **0.625 nM** | | |
| C01047-DV29SP | PDK1 | 12.6 | 19.8 | 14.4 | 20.8 | >40 | >2000 |
| | VCAN | -17.2 | 35.1 | 18.4 | -34.1 | >40 | >2000 |
| | ITPRID2 | 24.7 | 26.3 | 30.2 | 26.4 | >40 | >2000 |
| | PLG | 21.1 | 9.0 | 9.0 | 20.5 | >40 | >2000 |
| | LPA | - | - | 58.6 | 68.3 | 0.020 | 1 |
| C01023-DV26P | DDI | 12.2 | 17.3 | 16.8 | 12.5 | >40 | >2500 |
| | CLTC | 35.1 | 31.8 | 29.3 | 30.0 | >40 | >2500 |
| | MARCHF8 | 20.6 | 16.2 | 18.7 | 11.5 | >40 | >2500 |
| | PLG | 15.9 | 9.8 | 5.4 | 13.3 | >40 | >2500 |
| | LPA | - | - | 68.1 | 68.9 | 0.016 | 1 |
| C01001-DV25P | PDK1 | 5.2 | 2.4 | 5.5 | 0.2 | >40 | >1176 |
| | VCAN | 28.7 | 36.9 | -39.4 | -29.9 | >40 | >1176 |
| | PLG | 4.4 | -3.7 | -10.5 | 14.1 | >40 | >1176 |
| | LPA | - | - | 63.1 | 64.0 | 0.034 | 1 |
| | KIN | -10.0 | -15.1 | -15.9 | -8.8 | >40 | >976 |
| | PCDH9 | 28.2 | 30.3 | 3.4 | 27.9 | >40 | >976 |
| | RALA | -9.5 | -1.8 | 0.6 | -1.6 | >40 | >976 |
| | SEM1 | -28.5 | -31.8 | -39.7 | -42.3 | >40 | >976 |
| | BRWD1 | 7.2 | 9.6 | -4.7 | 3.3 | >40 | >976 |
| | ONECUT2 | 0.5 | 15.3 | -16.4 | -10.2 | >40 | >976 |
| C02417-DV38P | APMAP | 7.5 | 1.7 | 5.5 | 3.9 | >40 | >976 |
| | PHACTR4 | 18.9 | 10.9 | 1.5 | 11.1 | >40 | >976 |
| | TAGLN | 27.0 | 19.8 | 12.1 | -6.4 | >40 | >976 |
| | DDX3Y | 8.4 | 12.6 | 12.8 | 5.5 | >40 | >976 |
| | PELI2 | 7.4 | 12.9 | 10.9 | 14.0 | >40 | >976 |
| | CNTLN | 18.7 | 5.9 | 0.4 | -4.5 | >40 | >976 |
| | PLG | 12.9 | 3.6 | -4.0 | -6.0 | >40 | >976 |
| | LPA | - | - | 61.1 | 60.4 | 0.041 | 1 |
| C01012-DV38P | DDI | 1.3 | 2.9 | 8.8 | 11.5 | >40 | >167 |
| | CLTC | 5.2 | 12.2 | 19.1 | 1.1 | >40 | >167 |
| | CREB5 | -12.2 | -17.6 | 4.5 | -27.4 | >40 | >167 |
| | PLG | 5.8 | -9.6 | -24.8 | -24.2 | >40 | >167 |
| | LPA | - | - | 54.7 | 56.8 | 0.240 | 1 |
| C01042-DV27SP | SEMA6D | -3.9 | 17.5 | 0.2 | -18.9 | >40 | >541 |
| | PDK1 | 37.8 | 33.1 | 29.2 | 27.1 | >40 | >541 |
| | VCAN | 8.6 | 28.9 | 40.7 | 25.8 | >40 | >541 |
| | PLG | 37.1 | 25.4 | 18.7 | 30.2 | >40 | >541 |
| | LPA | - | - | 64.4 | 68.8 | 0.074 | 1 |
| | NDRG3 | 22.2 | 15.7 | 20.6 | 27.0 | >40 | >1818 |
| | UNC93A | 14.2 | -78.9 | -135.3 | -117.5 | >40 | >1818 |
| | ADCY10 | 40.7 | -5.0 | -14.2 | 12.9 | >40 | >1818 |
| | CLMN | 10.4 | -6.3 | -3.0 | 6.4 | >40 | >1818 |
| | KIAA1671 | -6.8 | -16.0 | -25.3 | -12.9 | >40 | >1818 |
| | REST | -1.7 | -15.1 | -20.8 | -23.5 | >40 | >1818 |
| C01801-DV27P | ZRSR2 | 33.1 | 24.4 | 29.8 | -2.6 | >40 | >1818 |
| | SLC12A7 | 28.8 | 24.9 | 15.8 | 24.3 | >40 | >1818 |
| | STIM1 | 26.3 | 22.5 | 19.8 | 30.7 | >40 | >1818 |
| | MCRIP1 | 33.4 | 11.5 | -22.9 | -21.9 | >40 | >1818 |
| | MOV10 | 2.2 | -19.6 | -26.1 | -24.0 | >40 | >1818 |
| | PLG | 39.8 | 15.7 | 19.6 | 16.5 | >40 | >1818 |
| | LPA | - | - | 64.9 | 66.2 | 0.022 | 1 |
| C06001-DV26P | PLG | 31.8 | 6.4 | -4.5 | -3.8 | >40 | >488 |
| | LPA | - | - | 58.9 | 61.8 | 0.082 | 1 |
| C01011-DV27P | SPICE1 | 59.6 | 60.4 | 58.5 | 55.5 | <0.625 | <2.2 |
| | MARCHF8 | 9.7 | 11.2 | 1.9 | -2.5 | >40 | >142 |
| | PLG | 17.1 | -9.6 | -9.7 | -25.7 | >40 | >142 |
| | LPA | - | - | 54.6 | 51.8 | 0.282 | 1 |
| APC-OL | SP140 | 9.7 | 12.9 | 16.9 | 10.9 | >40 | >18 |
| | ACOT12 | -3.8 | -26.0 | 6.4 | -9.4 | >40 | >18 |
| | PLG | 23.9 | -2.7 | -6.0 | -23.7 | >40 | >18 |
| | LPA | - | - | 53.5 | 26.6 | 2.178 | 1 |

Note: The IC₅₀ values of each sequence against the LPA gene are derived from Table 11 in Section 4.4 of Example 4.

As shown in the table above:
(1) All of the sequences listed above are capable of inhibiting the LPA gene.
(2) Only the sequence C01011-DV27P exhibited an off-target effect on the SPICE1 gene, with an IC₅₀ ratio of less than 2.2 relative to the LPA gene.
(3) Except for C01011-DV27P, all other siRNA derivatives exhibited inhibition rates of less than 40.7% at 40 nM on potential off-target genes. Moreover, the IC₅₀ values of these siRNA derivatives for potential off-target genes were at least 167-fold higher than those for the target gene LPA, indicating that the siRNA derivatives disclosed herein exhibit minimal off-target effects.

### Example 7: Inhibition of the LPA gene and potential off-target genes by siRNA derivatives with anti-off-target modifications

In the prior art, there is a modification pattern in which the nucleotide at position 7 of the antisense strand of siRNA is replaced with glycol nucleic acid (GNA) to disrupt the seed region of the antisense strand, thereby significantly reducing off-target effects and lowering hepatotoxicity. In addition, studies have shown that replacing all base pairs at positions 1 to 8 from the 5' end of the antisense strand of siRNA with corresponding DNA nucleotides in both the sense and antisense strands can significantly reduce off-target effects without compromising the activity of siRNA.

Therefore, in this example, to reduce off-target effects, the siRNA motifs B01801, B01023, and B02417 were modified using the templates DV25P to DV27P, DV29P, DV32P, DV34P, DV38P, DV25SP to DV27SP, DV29SP, DV32SP, DV34SP, and DV38SP. Specifically, the nucleotides at position 7 or at positions 5 and 7 of the antisense strand were replaced with DNA, and the nucleotide at the position of the sense strand that is complementary to position 7 of the antisense strand was also replaced with DNA, thereby reducing off-target effects.

d7B5 anti-off-target modification: the nucleotides at positions 5 and 7 of the antisense strand were replaced with DNA, and the nucleotide at the position of the sense strand that is complementary to position 7 of the antisense strand was also replaced with DNA.

d7B anti-off-target modification: the nucleotide at position 7 of the antisense strand was replaced with DNA, and the nucleotide at the position of the sense strand that is complementary to position 7 of the antisense strand was also replaced with DNA.

The experimental results demonstrated that at concentrations ranging from 30 pM to 30 nM, the siRNA derivatives with anti-off-target modifications exhibited significant inhibitory activity against the target gene LPA, and the inhibition rates of these derivatives against potential off-target genes were significantly lower than those against the target gene. These findings indicate that the incorporation of anti-off-target modifications does not compromise the inhibitory effect of the alternately modified and template-modified siRNA derivatives disclosed herein on LPA gene expression, but can effectively reduce off-target effects in certain sequences.

### 1. Experimental materials

### 1) Test samples:

siRNA motifs B01801, B01023, and B02417 modified using template modification alone, or corresponding conjugates using template modification combined with anti-off-target modification (see Table 19 in Example 9 below for sequences).

### 2) Sequence synthesis

### I. Synthesis of template-modified siRNA derivatives

The sequences were synthesized with reference to the method described in Example 2 for alternately modified siRNA derivatives.

### II. siRNA derivatives with DNA-based anti-off-target modifications

The synthesis was performed similarly to that described in Example 2, except that the nucleotide at position 5 or 7 from the 5' end of the antisense strand, or the nucleotide in the sense strand complementary to the nucleotide at position 7 from the 5' end of the antisense strand was synthesized using the following DNA phosphoramidite monomers, including DMT-dA phosphoramidite monomer (Formula 15), DMT-dT phosphoramidite monomer (Formula 16), DMT-dC phosphoramidite monomer (Formula 17), and DMT-dG phosphoramidite monomer (Formula 18), the structures of which are as follows:

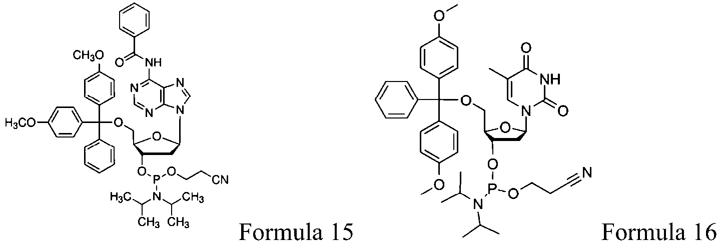

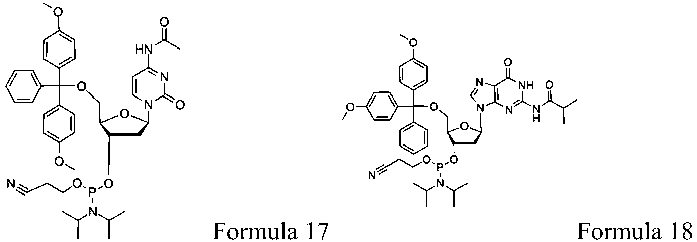

### 2. Experimental methods

The inhibitory effects of the test samples on the mRNA expression of the LPA gene and potential off-target genes in PHHs were evaluated by qRT-PCR. The experimental materials and methods were the same as those described in Example 6.

### 3. Experimental results

**(1) After template modification followed by anti-off-target modification, the resulting sequence exhibited significant inhibitory activity on LPA gene expression.** For example, after the siRNA motif B01023 was modified using the template DV25P, the resulting conjugate D01023-DV25PG101 exhibited an inhibition rate of 86.2% on LPA gene expression in primary human hepatocytes; after modification with the template DV25P followed by the anti-off-target modification d7B5, the corresponding conjugate D01023-DV25Pd7B5G101 exhibited an inhibition rate as high as 87.7%.

At concentrations of 30 nM, 1 nM, and 0.03 nM, the inhibition rates of the conjugates on LPA gene expression are as follows.

**Table 15: Inhibition rates of conjugates using template modification and anti-off-target modification on LPA gene expression in primary human hepatocytes**

| **siRNA ID** | **siRNA ID** | **Modification pattern** | **Inhibition rate (%)** | | |
|---|---|---|---|---|---|
| | | | **30 nM** | **1 nM** | **0.03 nM** |
| B01023 | D01023-DV25PG101 | Template modification | 86.2 | 90.2 | 54.5 |
| | D01023-DV25Pd7B5G101 | Template modification + d7B5 anti-off-target modification | 87.7 | 83.3 | 64.0 |
| B01801 | D01801-DV27PG101 | Template modification | 77.1 | 70.6 | 57.5 |
| | D01801-DV27Pd7BG101 | Template modification + d7B anti-off-target modification | 83.1 | 83.9 | 53.4 |
| B02417 | D02417-DV38PG101 | Template modification | 78.5 | 77.1 | 48.7 |
| | D02417-DV38Pd7B5G101 | Template modification + d7B5 anti-off-target modification | 76.7 | 74.2 | 67.0 |
| Positive control | Olpasiran | Intrinsic modification | 59.5 | 55.9 | 32.4 |

**(2) After modification using any one or more of the templates disclosed herein followed by anti-off-target modification, the resulting sequence exhibited significant anti-off-target effects on potential off-target genes, thereby reducing the inhibitory effect of siRNA on these genes.**

### As shown in Table 16, the experimental results demonstrated that:

The combination of template modification and anti-off-target modification disclosed herein can reduce the inhibitory effect on off-target genes, thereby achieving an anti-off-target effect. The derivatives/conjugates incorporating both the template and anti-off-target modifications disclosed herein can significantly reduce off-target effects, with a decrease of up to 50.7% in inhibition against off-target genes.

**Table 16: Inhibition rates of template-modified conjugates and conjugates incorporating both template and anti-off-target modifications against LPA and potential off-target genes**

| **Motif ID** | **siRNA ID** | **Potential off-target genes** | **Inhibition against off-target genes (%)** | **Reduction in inhibition vs. conjugate without anti-off-target modification (%)** |
|---|---|---|---|---|
| B01801 | D01801-DV27PG101 | UNC93A | 38.7 | 0.0 |
| | D01801-DV27Pd7BG101 | | 11.8 | 26.8 |
| | D01801-DV27PG101 | ADCY10 | 50.0 | 0.0 |
| | D01801-DV27Pd7BG101 | | 17.1 | 32.9 |
| | D01801-DV27PG101 | STIM1 | 36.7 | 0.0 |
| | D01801-DV27Pd7BG101 | | -14.0 | 50.7 |
| | D01801-DV27PG101 | MCRIP1 | 32.6 | 0.0 |
| | D01801-DV27Pd7BG101 | | 7.4 | 25.2 |
| B02417 | D02417-DV38PG101 | BRWD1 | 29.1 | 0.0 |
| | D02417-DV38Pd7B5G101 | | 8.7 | 20.4 |
| | D02417-DV38PG101 | APMAP | 33.6 | 0.0 |
| | D02417-DV38Pd7B5G101 | | 2.2 | 31.4 |
| | D02417-DV38PG101 | TAGLN | 36.4 | 0.0 |
| | D02417-DV38Pd7B5G101 | | -5.0 | 41.4 |

### 1) Template modification alone

After template modification, some conjugates exhibited certain off-target effects. For example, the conjugate D01801-DV27PG101, derived from the siRNA motif B01801 modified using the template DV27P, exhibited an inhibition rate of 50.0% against the potential off-target gene ADCY10; the conjugate D02417-DV38PG101, derived from the siRNA motif B02417 modified using the template DV38P, exhibited an inhibition rate of 36.4% against the potential off-target gene TAGLN.

### 2) Template modification combined with anti-off-target modification (denoted as "d7B5" or "d7B" in the siRNA ID coding rules disclosed herein)

The conjugates incorporating both template and anti-off-target modifications exhibited significantly reduced inhibition against potential off-target genes, demonstrating a significant anti-off-target effect. For example, D01801-DV27Pd7BG101 exhibited a 32.9% reduction in inhibition against ADCY10, indicating a significant decrease; D02417-DV38Pd7B5G101 exhibited a 41.4% reduction in inhibition against TAGLN, indicating a significant decrease.

### Example 8: Inhibitory effect of siRNA derivatives and corresponding conjugates modified using the templates disclosed herein on LPA in primary human hepatocytes

In this example, the sequences with relatively high activity identified in Example 4 were evaluated for their inhibitory effects on potential off-target genes in primary human hepatocytes, and were compared with Olpasiran. The eight motifs B01001, B01012, B01023, B01042, B01801, B02417, B06001, and B01047 were modified using the templates listed in Table 17, followed by conjugation with conjugate moieties to prepare the corresponding siRNA conjugates. These siRNA conjugates were transfected into primary human hepatocytes using a lipid-based method at concentrations ranging from 0.03 nM to 30 nM. Dose-response curves were fitted to calculate IC₅₀ values for each siRNA.

### 1. Experimental materials

### 1) Test samples:

The conjugates listed in Table 17 include those modified using the templates disclosed herein as well as those incorporating both the template and anti-off-target modifications disclosed herein, in which a GalNAc conjugate moiety G103 is attached to the 3' end of the sense strand of siRNA:

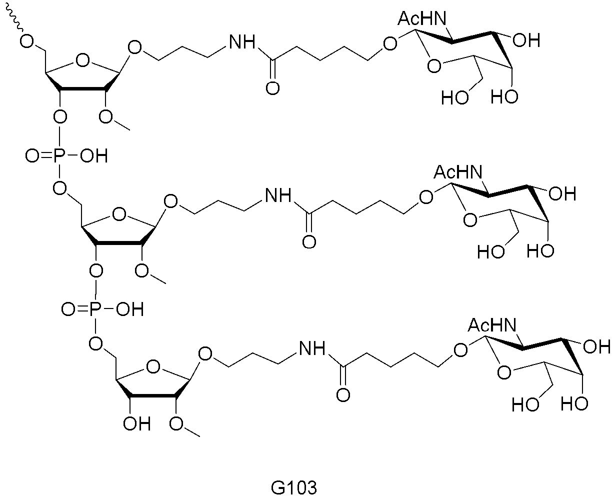

The method of conjugating the oligonucleotide to the conjugate moiety G103 may refer to Example 3 of patent application CN116854754A.

The oligonucleotide and conjugate moiety form a conjugate as shown below:

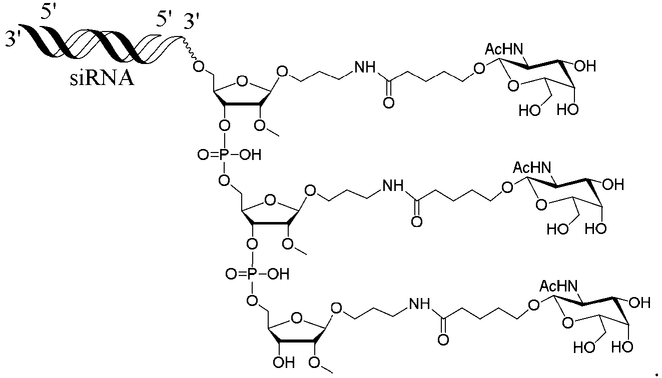

A detailed list of the conjugates and their corresponding motifs is shown in Table 17. In the siRNA IDs, "G103" indicates conjugation with the GalNAc conjugate moiety G103.

Cell type: primary human hepatocytes, provided by Liver Biotechnology Co., Ltd. (Cat. No.: LV-PHH001).

### 2. Experimental methods

The inhibitory effects of the test samples on LPA mRNA expression in primary human hepatocytes were evaluated by qRT-PCR. The experimental materials and methods were the same as those described in Section 1.2.2 of Example 1.

### 3. Experimental results

The inhibition rates of siRNA at different concentrations against LPA are shown in Table 17 below. The results demonstrated that all tested siRNAs exhibited significant inhibitory effects on LPA, with IC₅₀ values ranging from 0.005 nM to 0.030 nM. For example, at a concentration of 30 nM, D01023-DV25Pd7B5G103 achieved an inhibition rate of 86.4% against LPA, with an IC₅₀ value of 0.016 nM. In addition, some of the sequences exhibited superior IC₅₀ values compared to the positive control Olpasiran. For example, D01023-DV25Pd7B5G103 exhibited an IC₅₀ value of 0.016 nM, outperforming that of the positive control (0.027 nM).

**Table 17: Inhibition rates and IC₅₀ values of siRNAs at different concentrations on LPA in primary human hepatocytes**

| **siRNA ID** | **siRNA ID** | **Inhibition rate (%)** | | | | **IC₅₀ (nM)** |
|---|---|---|---|---|---|---|
| | | **30 nM** | **3 nM** | **0.3 nM** | **0.03 nM** | |
| B01001 | D01001-DV25Pd7B5G103 | 92.4 | 89.3 | 82.4 | 59.6 | 0.015 |
| | D01001-DV25Pd7BG103 | 88.4 | 85.5 | 85.2 | 63.9 | 0.016 |
| | D01001-DV25PG103 | 84.6 | 81.9 | 81.6 | 54.6 | 0.025 |
| | D01001-DV27Pd7B5G103 | 94.3 | 91.7 | 87.2 | 55.9 | 0.023 |
| | D01001-DV27PG103 | 86.9 | 82.6 | 78.6 | 61.1 | 0.011 |
| B01012 | D01012-DV38PG103 | 86.5 | 85.4 | 74.1 | 58.7 | 0.020 |
| B01023 | D01023-DV25Pd7B5G103 | 86.4 | 88.2 | 84.4 | 63.5 | 0.016 |
| | D01023-DV25PG103 | 82.7 | 85.0 | 72.5 | 53.8 | 0.022 |
| | D01023-DV26Pd7B5G103 | 86.2 | 87.4 | 80.8 | 69.9 | 0.004 |
| | D01023-DV26Pd7BG103 | 86.2 | 80.3 | 82.5 | 67.3 | 0.012 |
| | D01023-DV26PG103 | 79.0 | 74.2 | 70.9 | 49.9 | 0.030 |
| B01042 | D01042-DV27SPd7B5G103 | 86.6 | 88.9 | 80.8 | 64.5 | 0.010 |
| | D01042-DV27SPd7BG103 | 88.8 | 84.2 | 79.5 | 63.1 | 0.008 |
| | D01042-DV27SPG103 | 75.9 | 80.2 | 77.3 | 64.1 | 0.016 |
| B01801 | D01801-DV27Pd7B5G103 | 78.2 | 75.7 | 78.0 | 67.6 | 0.014 |
| | D01801-DV27Pd7BG103 | 78.8 | 82.8 | 82.0 | 67.1 | 0.011 |
| | D01801-DV27PG103 | 77.7 | 79.2 | 74.6 | 62.7 | 0.010 |
| B02417 | D02417-DV38Pd7B5G103 | 84.8 | 85.4 | 84.8 | 66.7 | 0.018 |
| | D02417-DV38PG103 | 90.6 | 88.5 | 85.3 | 71.5 | 0.005 |
| B06001 | D06001-DV26Pd7B5G103 | 84.0 | 85.4 | 82.6 | 66.2 | 0.013 |
| | D06001-DV26PG103 | 88.1 | 87.2 | 86.1 | 70.1 | 0.011 |
| B01047 | D01047-DV26SPd7B5G103 | 94.4 | 95.2 | 89.9 | 80.2 | 0.002 |
| | D01047-DV26SPG103 | 95.4 | 93.9 | 88.9 | 76.4 | 0.002 |
| | D01047-DV29SPd7B5G103 | 90.0 | 88.7 | 89.5 | 70.2 | 0.025 |
| | D01047-DV29SPd7BG103 | 85.7 | 85.2 | 86.4 | 70.4 | 0.025 |
| | D01047-DV29SPG103 | 76.2 | 73.2 | 78.7 | 62.0 | 0.026 |
| Positive control | Olpasiran | 64.0 | 61.0 | 56.3 | 50.3 | 0.027 |

### Example 9: Inhibitory effect of siRNA derivatives and corresponding conjugates modified using the templates disclosed herein on serum LPA in mice

In this example, certain sequences were exemplarily selected, including siRNA motifs B01001, B01012, B01023, B01042, B01047, B01801, B02417, and B06001. These motifs were modified, for example, using only the templates disclosed herein, or incorporating both the template and anti-off-target modifications disclosed herein. The positive control was Olpasiran, a candidate drug currently in Phase III clinical trials. Using transgenic mice expressing the human LPA gene, the inhibitory effects on serum LPA at different time points were evaluated by ELISA.

### 1. Experimental materials

### Test articles:

The sequences listed in Tables 18 and 19 include those modified using template modification alone as well as those incorporating both template and anti-off-target modifications, in which a GalNAc conjugate moiety G101 or G103 is attached to the 3' end of the sense strand:

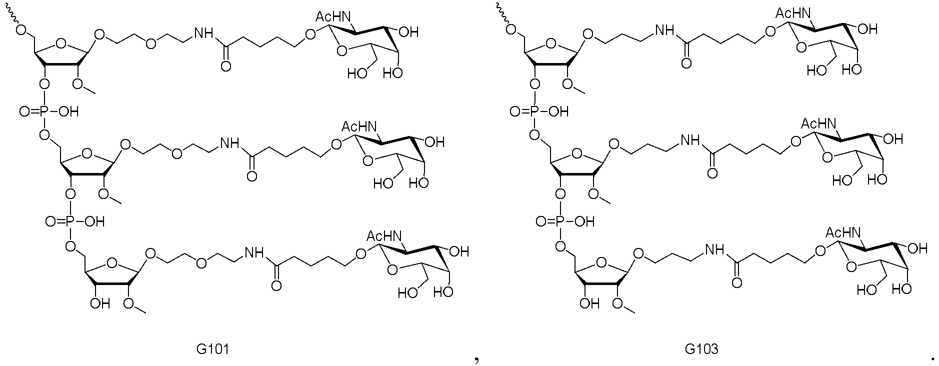

The method of conjugating the oligonucleotide with G101 or G103 may refer to Example 3 of patent application CN116854754A.

The oligonucleotide and conjugate moiety form a conjugate as shown below:

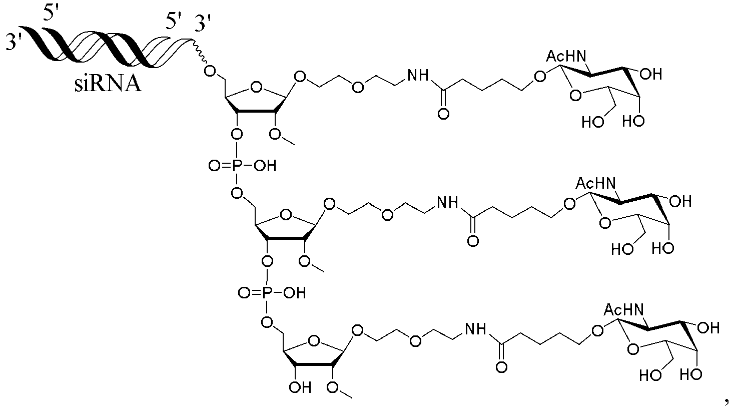

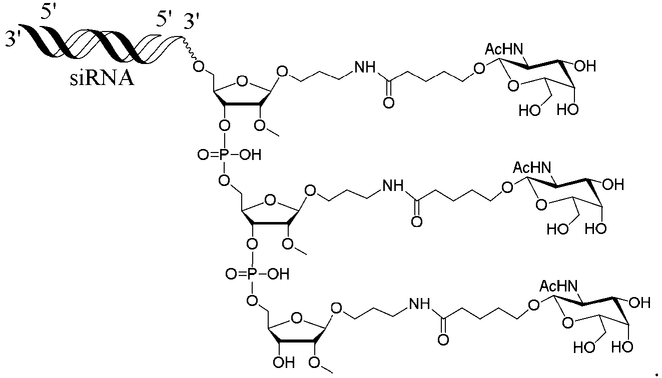

A detailed list of the conjugates is shown in Tables 18 and 19. In the siRNA IDs, "G103" indicates conjugation with the GalNAc conjugate moiety G103, while "G101" indicates conjugation with the GalNAc conjugate moiety G101. APC-OL represents the corresponding sequence of Olpasiran, in which the 5' end of the sense strand is conjugated with the GalNAc conjugate moiety NAG25 (*i.e.,* GR1), the structure of which is disclosed, for example, in CN113507920A.

In this experiment, the conjugates derived from the motif B01047 with template modification and anti-off-target modification were compared *in vivo* with the conjugate sequence Geno-1-107M disclosed in the prior art (see Qian Li et al., Application of improved GalNAc conjugation in development of cost-effective siRNA therapies targeting cardiovascular diseases, Molecular Therapy, Vol. 32, No. 3, 2024), which is similar to B01047.

**Table 18: Exemplary design of conjugation with G103**

| **siRNA ID** | **SEO ID NO of sense strand** | **Sense modification pattern** | **SEQ ID NO of antisense strand** | **Antisense modification pattern** | **Anti-off-target modification** | **Conjugate moiety** |
|---|---|---|---|---|---|---|
| D01047-DV29SPG103 | 44 | b | 237 | C | None | G103 |
| D01023-DV26PG103 | 21 | a | 214 | B | None | G103 |
| D01023-DV25PG103 | 21 | a | 214 | A | None | G103 |
| D01001-DV25PG103 | 1 | a | 194 | A | None | G103 |
| D01801-DV27PG103 | 184 | a | 377 | C | None | G103 |
| D01001-DV27PG103 | 1 | a | 194 | C | None | G103 |
| D01047-DV26SPG103 | 44 | a | 237 | B | None | G103 |
| D01042-DV27SPG103 | 39 | a | 232 | C | None | G103 |
| D02417-DV38PG103 | 76 | b | 269 | E | None | G103 |
| D06001-DV26PG103 | 132 | a | 325 | B | None | G103 |
| D01012-DV38PG103 | 186 | b | 379 | E | None | G103 |
| D01047-DV29SPd7B5G103 | 44 | b | 237 | C | d7B5 | G103 |
| D01023-DV26Pd7B5G103 | 21 | a | 214 | B | d7B5 | G103 |
| D01023-DV25Pd7B5G103 | 21 | a | 214 | A | d7B5 | G103 |
| D01001-DV25Pd7B5G103 | 1 | a | 194 | A | d7B5 | G103 |
| D01801-DV27Pd7B5G103 | 184 | a | 377 | C | d7B5 | G103 |
| D01001-DV27Pd7B5G103 | 1 | a | 194 | C | d7B5 | G103 |
| D01047-DV26SPd7B5G103 | 44 | a | 237 | B | d7B5 | G103 |
| D01042-DV27SPd7B5G103 | 39 | a | 232 | C | d7B5 | G103 |
| D02417-DV38Pd7B5G103 | 76 | b | 269 | E | d7B5 | G103 |
| D06001-DV26Pd7B5G103 | 132 | a | 325 | B | d7B5 | G103 |
| D01047-DV29SPd7BG103 | 44 | b | 237 | C | d7B | G103 |
| D01023-DV26Pd7BG103 | 21 | a | 214 | B | d7B | G103 |
| D01001-DV25Pd7BG103 | 1 | a | 194 | A | d7B | G103 |
| D01801-DV27Pd7BG103 | 184 | a | 377 | C | d7B | G103 |
| D01042-DV27SPd7BG103 | 39 | a | 232 | C | d7B | G103 |
| DNC-DV29PG103 | 193 | b | 386 | C | None | G103 |
| DPC-OLG103 | The 3' end of the sense strand of APC-OL is conjugated with G103 | | | | | G103 |

**Table 19: Exemplary design of conjugation with G101**

| **siRNA ID** | **SEO ID NO of sense motif** | **Sense modification pattern** | **SEQ ID NO of antisense motif** | **Antisense modification pattern** | **Anti-off-target modification** | **Conjugate moiety** |
|---|---|---|---|---|---|---|
| D01023-DV25PG101 | 21 | a | 214 | A | None | G101 |
| D01023-DV25Pd7B5G101 | 21 | a | 214 | A | d7B5 | G101 |
| D01801-DV27PG101 | 184 | a | 377 | C | None | G101 |
| D01801-DV27Pd7BG101 | 184 | a | 377 | C | d7B | G101 |
| D02417-DV38PG101 | 76 | b | 269 | E | None | G101 |
| D02417-DV38Pd7B5G101 | 76 | b | 269 | E | d7B5 | G101 |

| | | | | | | |
|---|---|---|---|---|---|---|
| Solvent: sterile PBS. | | | | | | |

### 2. Experimental methods

Male C57BL/6-hLPA transgenic mice (provided by Shanghai Model Organisms Center, Inc.) aged 6 to 8 weeks were acclimated in the animal facility for 7 days after arrival. Blood samples were collected and serum was isolated. hLPA protein levels in serum were measured using an ELISA kit (Abcam, ab212165). The mice were randomly grouped based on hLPA levels in serum, with 6 mice per group, and were administered subcutaneously a single dose of 1 mg/kg or 0.5 mg/kg (the day of administration was designated as Day 0 (D0)). After administration, serum samples were collected every 7 days to measure hLPA protein expression in serum. The inhibition rate on hLPA expression was calculated according to the following formula: 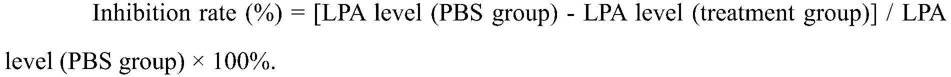 Inhibition rate (%) = [LPA level (PBS group) - LPA level (treatment group)]/LPA level (PBS group) × 100%.

### 3. Experimental results

**Table 20: Inhibition rates in hLPA transgenic mice at 1 mg/kg**

| **siRNA ID** | **siRNA ID** | **Inhibition rate (%)** | | | | | | | | | |
|---|---|---|---|---|---|---|---|---|---|---|---|
| | | **D7** | **D14** | **D21** | **D28** | **D35** | **D42** | **D49** | **D56** | **D63** | **D70** |
| | D01001-DV25Pd7B5G103 | 37.1 | 21.4 | 8.1 | 7.2 | - | - | - | - | - | - |
| | D01001-DV25Pd7BG103 | 41.8 | 55.0 | 60.0 | 63.9 | - | - | - | - | - | - |
| B01001 | D01001-DV25PG103 | 66.0 | 62.6 | 69.4 | 72.6 | 70.9 | 66.7 | 63.4 | 31.1 | - | - |
| | D01001-DV27Pd7B5G103 | 63.5 | 48.6 | 52.8 | 63.0 | - | - | - | - | - | - |
| | D01001-DV27PG103 | 50.0 | 54.1 | 63.7 | 64.5 | - | - | - | - | - | - |
| B01012 | D01012-DV38PG103 | 76.4 | 65.9 | 72.8 | 76.2 | 74.9 | 78.0 | 64.2 | 26.4 | - | - |
| B01023 | D01023-DV25Pd7B5G103 | 76.1 | 72.0 | 73.4 | 81.3 | 73.2 | 64.0 | 67.0 | 36.1 | 60.4 | 54.6 |
| | D01023-DV25PG103 | 64.2 | 68.7 | 73.3 | 72.6 | 74.4 | 65.5 | 61.2 | 40.9 | 61.8 | 56.9 |
| | D01023-DV26Pd7B5G103 | 69.2 | 65.9 | 64.3 | 71.6 | 79.1 | 72.7 | 61.6 | 22.0 | - | - |
| | D01023-DV26Pd7BG103 | 70.9 | 63.1 | 72.4 | 71.8 | 75.5 | 65.6 | 64.3 | 45.7 | - | - |
| | D01023-DV26PG103 | 55.4 | 65.9 | 66.4 | 66.9 | - | - | - | - | - | - |
| B01042 | D01042-DV27SPd7B5G103 | 68.5 | 62.2 | 62.9 | 64.1 | - | - | - | - | - | - |
| | D01042-DV27SPd7BG103 | 73.7 | 70.0 | 72.9 | 71.9 | 80.6 | 73.8 | 65.9 | 29.7 | - | - |
| | D01042-DV27SPG103 | 65.2 | 61.4 | 68.9 | 70.2 | 72.6 | 66.6 | 68.8 | 41.4 | - | - |
| B01801 | D01801-DV27Pd7B5G103 | 39.7 | 26.9 | 12.3 | 45.8 | - | - | - | - | - | - |
| | D01801-DV27Pd7BG103 | 85.4 | 77.8 | 86.4 | 76.3 | 80.1 | 71.5 | 63.7 | 60.1 | 65.0 | 47.9 |
| | D01801-DV27PG103 | 71.8 | 73.3 | 72.7 | 80.3 | 83.2 | 76.4 | 72.8 | 59.2 | 80.0 | 74.6 |
| B02417 | D02417-DV38Pd7B5G103 | 77.1 | 64.2 | 73.9 | 75.1 | 77.6 | 68.0 | 66.8 | 31.6 | 67.3 | 50.7 |
| | D02417-DV38PG103 | 75.3 | 75.3 | 80.0 | 81.2 | 86.3 | 77.4 | 74.7 | 56.8 | 76.3 | 69.8 |
| B06001 | D06001-DV26Pd7B5G103 | 79.2 | 69.9 | 69.5 | 68.1 | 69.8 | 55.6 | 64.1 | 44.4 | 64.4 | 43.8 |
| | D06001-DV26PG103 | 75.5 | 67.2 | 61.9 | 71.1 | 67.6 | 61.8 | 44.8 | 6.8 | - | - |
| B01047 | D01047-DV26SPd7B5G103 | 79.3 | 59.8 | 65.2 | 68.5 | - | - | - | - | - | - |
| | D01047-DV26SPG103 | 79.2 | 79.7 | 76.2 | 79.6 | 75.1 | 74.3 | 55.9 | 6.0 | - | - |
| | D01047-DV29SPd7B5G103 | 60.2 | 41.7 | 42.0 | 37.9 | - | - | - | - | - | - |
| | D01047-DV29SPd7BG103 | 85.9 | 73.4 | 78.2 | 75.1 | 71.5 | 61.5 | 64.3 | -24.8 | - | - |
| | D01047-DV29SPG103 | 81.6 | 70.3 | 67.8 | 73.8 | 71.8 | 61.6 | 54.7 | 40.2 | - | - |
| | Geno-1-107M | 72.1 | 70.5 | 61.4 | 54.7 | - | - | - | - | - | - |
| Negative control | DNC-DV29PG103 | -1.0 | -4.2 | 8.8 | 5.3 | - | - | - | - | - | - |
| Positive control | Olpasiran | 72.5 | 67.8 | 73.8 | 71.4 | 71.7 | 74.1 | 63.4 | 5.4 | 59.3 | 53.2 |

As shown in the table above, the conjugates formed by the conjugation of the siRNA derivatives with conjugate moieties exhibited a significant inhibitory effect on LPA protein expression in serum following a single subcutaneous dose of 1 mg/kg. For example, D01023-DV25Pd7B5G103 achieved inhibition rates of 76.1%, 72.0%, and 73.4% on Day 7, Day 14, and Day 21, respectively.

The conjugates D01047-DV26SPG103, D01047-DV29SPd7BG103, and D01047-DV29SPG103 derived from the motif B01047 disclosed herein exhibited significantly enhanced inhibitory effects in mice compared to Geno-1-107M disclosed in the prior art.

**Table 21: Inhibition rates in hLPA transgenic mice at 0.5 mg/kg**

| **siRNA ID** | **siRNA ID** | **Inhibition rate (%)** | | | | | | |
|---|---|---|---|---|---|---|---|---|
| | | **D7** | **D14** | **D21** | **D28** | **D35** | **D42** | **D49** |
| B01023 | D01023-DV25Pd7B5G101 | 67.0 | 73.5 | 77.4 | 77.7 | 60.5 | 65.6 | 58.5 |
| | D01023-DV25Pd7B5G103 | 73.0 | 67.4 | 75.5 | 69.6 | 67.9 | 61.8 | 64.0 |
| | D01023-DV25PG101 | 73.2 | 69.8 | 75.6 | 71.3 | 67.5 | 60.9 | 56.7 |
| B01801 | D01801-DV27Pd7BG101 | 80.8 | 76.1 | 81.9 | 72.7 | 69.2 | 66.4 | 60.1 |
| | D01801-DV27Pd7BG103 | 77.3 | 80.6 | 77.7 | 72.7 | 67.3 | 52.6 | 57.3 |
| | D01801-DV27PG101 | 77.7 | 78.2 | 82.8 | 81.3 | 80.4 | 78.2 | 76.3 |
| | D01801-DV27PG103 | 76.5 | 74.7 | 79.3 | 76.6 | 74.7 | 66.9 | 59.1 |
| B02417 | D02417-DV38Pd7B5G101 | 77.8 | 80.4 | 82.2 | 80.7 | 72.9 | 67.7 | 62.0 |
| | D02417-DV38Pd7B5G103 | 76.1 | 72.8 | 73.2 | 69.2 | 64.5 | 64.8 | 51.6 |
| | D02417-DV38PG101 | 78.8 | 74.9 | 76.6 | 71.9 | 65.3 | 66.8 | 65.7 |
| | D02417-DV38PG103 | 76.4 | 77.1 | 84.9 | 80.3 | 72.1 | 72.5 | 67.7 |
| Positive control | Olpasiran | 77.2 | 70.2 | 70.2 | 65.6 | 70.3 | 61.1 | 58.6 |
| Negative control | PBS | 0.0 | 0.0 | 0.0 | 0.0 | 0.0 | 0.0 | 0.0 |

As shown in the table above, the conjugates exhibited a significant inhibitory effect on LPA protein expression in serum following a single subcutaneous dose of 0.5 mg/kg. For example, D01023-DV25Pd7B5G101 exhibited inhibition rates of 67.0%, 73.5%, and 77.4% on Day 7, Day 14, and Day 21, respectively.

The *in vivo* results demonstrated that the siRNA motifs disclosed herein, such as B01001, B01012, B01023, B01042, B01047, B01801, B02417, and B06001, when modified using the templates disclosed herein, or incorporating both template and anti-off-target modifications, and further conjugated with GalNAc conjugate moieties, can be efficiently delivered to the liver of animals, significantly inhibiting LPA expression.

### Example 10: Inhibitory effect of sequences modified using the templates disclosed herein on serum LPA in cynomolgus monkeys

In this example, certain sequences were exemplarily selected, including siRNA motifs B01023, B01801, and B02417. These sequences were modified, for example, using template modification alone, or incorporating both template and anti-off-target modifications. The positive control was Olpasiran, an siRNA drug currently in Phase III clinical trials. After *in vivo* administration to cynomolgus monkeys, the inhibitory effects of the sequences listed above on serum LPA at different time points were evaluated by ELISA.

### 1. Experimental materials

**Test articles:** a total of six compounds, including D01023-DV25Pd7B5G101, D01801-DV27PG101, D01801-DV27Pd7BG101, D02417-DV38PG101, D02417-DV38Pd7B5G101, and the positive control Olpasiran. The detailed sequence design rules are shown in Table 19. The sequences were synthesized with reference to the method described in Example 9.

### Solvent: sterile saline.

### 2. Experimental methods

A total of 18 male cynomolgus monkeys aged 2.5 to 6 years and weighing 2 to 6 kg were acclimated in the animal facility for 7 days after arrival. Blood samples were collected and serum was isolated. LPA protein levels in serum were measured using an ELISA kit (Mercodia, Cat. No.: 10-1106-01). The animals were randomly grouped based on LPA levels in serum, with 3 animals per group, and were administered subcutaneously a single dose of 1 mg/kg (the day of administration was designated as Day 0 (D0)). After administration, serum samples were collected every 7 days to measure LPA protein expression in serum. The inhibition rate on LPA expression was calculated according to the following formula: 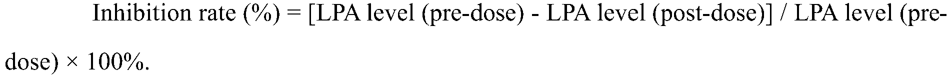 Inhibition rate (%) = [LPA level (pre-dose) - LPA level (post-dose)]/LPA level (pre-dose) × 100%.

On Day -9 (pre-dose) and Days 28 and 53 (post-dose), the animals were anesthetized. The puncture site was disinfected sequentially with iodine and alcohol. After confirming the position of the liver and the puncture site under ultrasound guidance, a biopsy needle was inserted to collect liver tissue samples. Total RNA was extracted from liver tissues, followed by qPCR amplification of LPA mRNA and GAPDH mRNA. The inhibition rate on LPA mRNA expression in liver tissues was calculated according to the following formula:
$ΔCt = mean Ct value of the target gene - mean Ct value of the reference gene ;$
$ΔΔCT = ΔCT \left(post-dose\right) - ΔCT \left(pre-dose\right) ;$
Relative mRNA expression of the target gene = 2^{-ΔΔCt}; 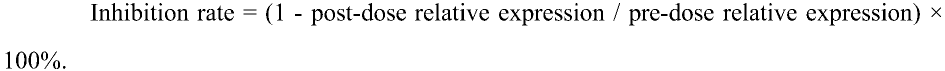 Inhibition rate = (1 - post-dose relative expression/pre-dose relative expression) × 100%.

### 3. Experimental results

The inhibition results are shown in Table 22 and FIG. 1.

**Table 22: Inhibition rates - serum**

| **siRNA ID** | **Inhibition rate (%)** | | | | | | | | | | | |
|---|---|---|---|---|---|---|---|---|---|---|---|---|
| | **D7** | **D14** | **D21** | **D28** | **D35** | **D42** | **D49** | **D56** | **D63** | **D70** | **D77** | **D84** |
| D02417-DV38PG101 | 74.4 | 80.8 | 89.6 | 86.8 | 88.1 | 87.5 | 87.6 | 89.7 | 84.6 | 88.7 | 67.9 | 71.4 |
| D01023-DV25Pd7B5G101 | 74.5 | 87.2 | 92.9 | 93.6 | 93.7 | 93.9 | 93.4 | 94.7 | 89.0 | 92.0 | 76.4 | 80.5 |
| D01801-DV27Pd7BG101 | 69.7 | 83.1 | 89.4 | 89.9 | 91.3 | 91.2 | 90.4 | 92.9 | 88.0 | 89.8 | 74.9 | 75.4 |
| D02417-DV38Pd7B5G101 | 80.9 | 86.4 | 92.9 | 84.1 | 90.4 | 91.9 | 90.6 | 91.1 | 86.0 | 88.7 | 71.4 | 74.5 |
| D01801-DV27PG101 | 60.3 | 81.5 | 87.2 | 85.4 | 73.4 | 76.9 | 78.7 | 80.2 | 71.5 | 78.3 | 52.2 | 63.6 |
| Olpasiran | 59.6 | 75.1 | 83.4 | 74.3 | 76.7 | 83.0 | 78.9 | 77.8 | 75.7 | 64.4 | 50.3 | 56.4 |

As shown in the table above, the conjugates formed by the conjugation of the sequences with GalNAc moieties exhibited a significant inhibitory effect on LPA protein expression in serum following a single subcutaneous dose of 1 mg/kg. For example, D01023-DV25Pd7B5G103 achieved an inhibition rate of 92.9% against serum LPA on Day 21 and maintained an inhibition rate of 92.0% against serum LPA on Day 70, indicating a prolonged duration of efficacy in cynomolgus monkeys. Compared to the positive control with a maximum inhibition rate of 83.4%, the sequence demonstrated a significant efficacy advantage.

The inhibition data for test compounds on LPA mRNA expression in the liver of cynomolgus monkeys are shown in Table 23 and FIG. 2.

**Table 23: Inhibition rates - liver**

| **siRNA ID** | **Inhibition rate (%)** | |
|---|---|---|
| | **D28** | **D53** |
| D02417-DV38PG101 | 91.3 | 94.2 |
| D01023-DV25Pd7B5G101 | 93.4 | 95.5 |
| D01801-DV27Pd7BG101 | 86.0 | 83.3 |
| D02417-DV38Pd7B5G101 | 89.5 | 95.0 |
| D01801-DV27PG101 | 79.1 | 84.1 |
| Olpasiran | 62.8 | 78.7 |

The results demonstrated that all five test compounds significantly inhibited LPA mRNA expression in the liver of cynomolgus monkeys on Days 28 and 53 post-dose (D28 and D53) compared to pre-dose (D-9). For example, D01023-DV25Pd7B5G101 exhibited inhibition rates of 93.4% on D28 and 95.5% on D53, which were significantly higher than those of the positive control Olpasiran at the same time points (62.8% on D28 and 78.7% on D53).

The *in vivo* results in cynomolgus monkeys demonstrated that the siRNA motifs of the present disclosure, such as B01023, B01801, and B02417, whether modified using the templates disclosed herein alone or further incorporating anti-off-target modifications, can be efficiently delivered to the liver of cynomolgus monkeys after conjugation with conjugate moieties, significantly inhibiting LPA gene expression.

## Claims

1. An siRNA duplex, wherein the siRNA duplex comprises a sense strand and an antisense strand that form a reverse complementary duplex region, wherein the antisense strand comprises a fragment of at least 18 consecutive nucleotides complementary to a target sequence of the nucleotide sequence as shown in SEQ ID NO: 420, or a modified fragment thereof;
wherein the siRNA duplex comprises any one oligonucleotide duplex selected from the following pairings of a sense strand and an antisense strand, or a combination of two thereof:
(1) the sequence of the sense strand is as shown in SEQ ID NO: 21 or a modified sequence thereof; and the sequence of the antisense strand is as shown in SEQ ID NO: 214 or a modified sequence thereof; and,
(8) the sequence of the sense strand is as shown in SEQ ID NO: 186 or a modified sequence thereof; and the sequence of the antisense strand is as shown in SEQ ID NO: 379 or a modified sequence thereof.

2. The siRNA duplex according to claim 1, wherein the sense strand or the antisense strand comprises at least one modified nucleotide selected from a deoxy-nucleotide, a 3'-deoxythymidine (dT) nucleotide, a 2'-O-methyl modified nucleotide, a 2'-fluoro modified nucleotide, a 2'-deoxy-modified nucleotide, a locked nucleotide, an unlocked nucleotide, a conformationally restricted nucleotide, a constrained ethyl nucleotide, an abasic nucleotide, a 2'-amino-modified nucleotide, a 2'-O-allyl-modified nucleotide, a 2'-C-alkyl-modified nucleotide, a 2'-hydroxy-modified nucleotide, a 2'-methoxyethyl modified nucleotide, a 2'-O-alkyl-modified nucleotide, a morpholino nucleotide, a phosphoramidate, a nucleotide comprising a non-natural base, a tetrahydropyran modified nucleotide, a 1,5-anhydrohexitol modified nucleotide, a cyclohexenyl modified nucleotide, a nucleotide comprising a phosphorothioate group, a nucleotide comprising a methylphosphonate group, a nucleotide comprising a 5'-phosphate group, and a nucleotide comprising a 5'-phosphate mimic, and combinations thereof.

3. The siRNA duplex according to claim 2, wherein all nucleotides on the sense strand and the antisense strand comprise a modification at the 2' position of the ribose of the nucleotide;
preferably, the modification at the 2' position of the ribose of the nucleotide is any one selected from 2'-O-methyl, 2'-methoxyethyl, 2'-fluoro, 2'-benzyloxy, 2'-methylcarbonylamino, and 2'-pyridinylmethoxy, or a combination thereof.

4. The siRNA duplex according to claim 1, wherein nucleotide monomers are linked to one another via a 3',5'-phosphodiester bond or a 3',5'-phosphorothioate diester bond; or,
wherein the 3',5'-phosphodiester bond between nucleotides at the terminus of the siRNA duplex comprises a phosphorothioate modification to form a stereochemically pure 3',5'-phosphorothioate diester bond, wherein the 5' ends of both the sense and antisense strands each comprise 1 to 3 phosphorothioate linkages, and the 3' end of the antisense strand comprises 1 to 3 phosphorothioate linkages; or,
wherein the 5' carbon atom of the sugar moiety of the nucleotide at the 5' end of the modified antisense strand is phosphorylated, and the phosphorylation of the 5' carbon atom is selected from the following 5'-phosphorylation groups: 5'-vinylphosphonate group (5'-E-VP), 5'-methylphosphonate group (5'-MP), 5'-C-methylphosphonate group, 5'-phosphorothioate group (5'-PS), and 5'-phosphate group (5'-P), the structures of which are as follows: 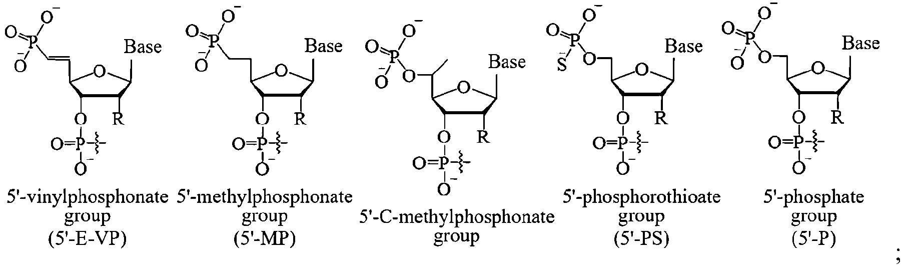
wherein R is hydrogen, hydroxyl, amino, C₁₋₄ alkyl, aryl, C₁₋₄ alkoxy, C₁₋₄ alkylcarbonylamino, or halogen;
the base is selected from adenine, guanine, cytosine, thymine, and uracil; or,
wherein nucleotides at positions 2 to 8 from the 5' end of the antisense strand are modified with a modifying moiety, wherein the modifying moiety is selected from UNA, GNA, and DNA, wherein the structures of UNA and GNA are as follows: 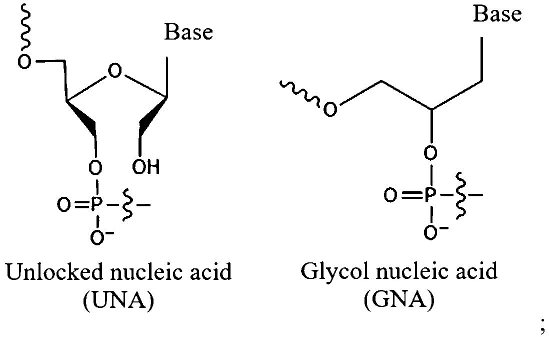
wherein the base is selected from adenine, guanine, cytosine, thymine, and uracil.

5. The siRNA duplex according to claim 1, wherein the siRNA duplex has the following modifications:
the antisense strand has any one of the following modification patterns A to F shown below:
| No. | **Nucleotide position (5'-3')** | | | | | | | | | | | |
|---|---|---|---|---|---|---|---|---|---|---|---|---|
| **A** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **B** | **1** | **2** | **3** | **4** | **5** | **6** | **7** | **8** | **9** | **10** | **11** | **12** |
| | 2'-OMe +PS +EVP | **2'-F +PS** | **2'-F** | **2'-F** | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **C** | **1** | **2** | **3** | **4** | **5** | **6** | **7** | **8** | **9** | **10** | **11** | **12** |
| | 2'-OMe +PS +EVP | **2'-F +PS** | 2'-OMe | **2'-F** | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **D** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **E** | **1** | **2** | **3** | **4** | **5** | **6** | **7** | **8** | **9** | **10** | **11** | **12** |
| | 2'-OMe +PS +EVP | **2'-F +PS** | 2'-OMe | **2'-F** | **2'-F** | 2'-OMe | 2'-OMe | 2'-OMe | **2'-F** | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **F** | **1** | **2** | **3** | **4** | **5** | **6** | **7** | **8** | **9** | **10** | **11** | **12** |
| | 2'-OMe +PS +EVP | **2'-F +PS** | **2'-F** | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
and/or, the sense strand adopts modification pattern a or b:
| No. | **Nucleotide position (5'-3')** | | | | | | | | | | |
|---|---|---|---|---|---|---|---|---|---|---|---|
| **a** | **1** | **2** | **3** | **4** | **5** | **6** | **7** | **8** | **9** | **10** | **11** |
| | 2'-OMe+PS2' | -OMe+PS | 2'-OMe | 2'-OMe | 2'-OMe | 2'-OMe | **2'-F** | 2'-OMe | **2'-F** | **2'-F** | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | 2'-F | 2'-OMe | 2'-OMe | 2'-OMe | 2'-OMe | |
| **b** | **1** | **2** | **3** | **4** | **5** | **6** | **7** | **8** | **9** | **10** | **11** |
| | 2'-OMe+PS | 2'-OMe+PS | 2'-OMe | 2'-OMe | 2'-OMe | 2'-OMe | **2'-F** | 2'-OMe | **2'-F** | 2'-OMe | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | 2'-F | 2'-OMe | 2'-OMe | 2'-OMe | 2'-OMe | |
wherein 2'-OMe represents a 2'-O-methyl modification; 2'-F represents a 2'-fluoro modification; PS indicates that, starting from the 5' end, the nucleotide at the position is linked to the adjacent nucleotide on the right via a 3',5'-phosphorothioate bond; EVP represents a 5'-vinyl-(E)-phosphonate; preferably,
the antisense strand is modified according to modification pattern A, and the sense strand is modified according to modification pattern a;
the antisense strand is modified according to modification pattern B, and the sense strand is modified according to modification pattern a;
the antisense strand is modified according to modification pattern C, and the sense strand is modified according to modification pattern a;
the antisense strand is modified according to modification pattern D, and the sense strand is modified according to modification pattern a;
the antisense strand is modified according to modification pattern E, and the sense strand is modified according to modification pattern a;
the antisense strand is modified according to modification pattern F, and the sense strand is modified according to modification pattern a;
the antisense strand is modified according to modification pattern A, and the sense strand is modified according to modification pattern b;
the antisense strand is modified according to modification pattern B, and the sense strand is modified according to modification pattern b;
the antisense strand is modified according to modification pattern C, and the sense strand is modified according to modification pattern b;
the antisense strand is modified according to modification pattern D, and the sense strand is modified according to modification pattern b;
the antisense strand is modified according to modification pattern E, and the sense strand is modified according to modification pattern b; or
the antisense strand is modified according to modification pattern F, and the sense strand is modified according to modification pattern b.

6. The siRNA duplex according to claim 5, wherein the siRNA duplex comprises any one oligonucleotide duplex selected from the following pairings of a sense strand and an antisense strand, or a combination of at least two thereof:
(1) the sense strand is the modified sequence of SEQ ID NO: 21; and the antisense strand is the modified sequence of SEQ ID NO: 214; wherein the antisense strand is modified according to modification pattern B, and the sense strand is modified according to modification pattern a;
(2) the sense strand is the modified sequence of SEQ ID NO: 186; and the antisense strand is the modified sequence of SEQ ID NO: 379; wherein the antisense strand is modified according to modification pattern E, and the sense strand is modified according to modification pattern b;
(3) the sense strand is the modified sequence of SEQ ID NO: 21; and the antisense strand is the modified sequence of SEQ ID NO: 214; wherein the antisense strand is modified according to modification pattern A, and the sense strand is modified according to modification pattern a; nucleotides at positions 5 and 7 of the antisense strand are replaced with DNA, and the nucleotide at the position of the sense strand that is complementary to position 7 of the antisense strand is also replaced with DNA;
(4) the sense strand is the modified sequence of SEQ ID NO: 21; and the antisense strand is the modified sequence of SEQ ID NO: 214; wherein the antisense strand is modified according to modification pattern A, and the sense strand is modified according to modification pattern a;
(5) the sense strand is the modified sequence of SEQ ID NO: 21; and the antisense strand is the modified sequence of SEQ ID NO: 214; wherein the antisense strand is modified according to modification pattern C, and the sense strand is modified according to modification pattern a;
(6) the sense strand is the modified sequence of SEQ ID NO: 21; and the antisense strand is the modified sequence of SEQ ID NO: 214; wherein the antisense strand is modified according to modification pattern C, and the sense strand is modified according to modification pattern b;
(7) the sense strand is the modified sequence of SEQ ID NO: 21; and the antisense strand is the modified sequence of SEQ ID NO: 214; wherein the antisense strand is modified according to modification pattern D, and the sense strand is modified according to modification pattern b;
(8) the sense strand is the modified sequence of SEQ ID NO: 21; and the antisense strand is the modified sequence of SEQ ID NO: 214; wherein the antisense strand is modified according to modification pattern E, and the sense strand is modified according to modification pattern b;
(9) the sense strand is the modified sequence of SEQ ID NO: 21; and the antisense strand is the modified sequence of SEQ ID NO: 214; wherein the antisense strand is modified according to modification pattern B, and the sense strand is modified according to modification pattern a; nucleotides at positions 5 and 7 of the antisense strand are replaced with DNA, and the nucleotide at the position of the sense strand that is complementary to position 7 of the antisense strand is also replaced with DNA;
(10) the sense strand is the modified sequence of SEQ ID NO: 21; and the antisense strand is the modified sequence of SEQ ID NO: 214; wherein the antisense strand is modified according to modification pattern B, and the sense strand is modified according to modification pattern a; the nucleotide at position 7 of the antisense strand is replaced with DNA, and the nucleotide at the position of the sense strand that is complementary to position 7 of the antisense strand is also replaced with DNA;
(11) the sense strand is the modified sequence of SEQ ID NO: 186; and the antisense strand is the modified sequence of SEQ ID NO: 379; wherein the antisense strand is modified according to modification pattern A, and the sense strand is modified according to modification pattern a;
(12) the sense strand is the modified sequence of SEQ ID NO: 186; and the antisense strand is the modified sequence of SEQ ID NO: 379; wherein the antisense strand is modified according to modification pattern B, and the sense strand is modified according to modification pattern a;
(13) the sense strand is the modified sequence of SEQ ID NO: 186; and the antisense strand is the modified sequence of SEQ ID NO: 379; wherein the antisense strand is modified according to modification pattern C, and the sense strand is modified according to modification pattern a;
(14) the sense strand is the modified sequence of SEQ ID NO: 186; and the antisense strand is the modified sequence of SEQ ID NO: 379; wherein the antisense strand is modified according to modification pattern C, and the sense strand is modified according to modification pattern b;
(15) the sense strand is the modified sequence of SEQ ID NO: 186; and the antisense strand is the modified sequence of SEQ ID NO: 379; wherein the antisense strand is modified according to modification pattern D, and the sense strand is modified according to modification pattern b; and,
(16) the sense strand is the modified sequence of SEQ ID NO: 186; and the antisense strand is the modified sequence of SEQ ID NO: 379; wherein the antisense strand is modified according to modification pattern F, and the sense strand is modified according to modification pattern b.

7. An siRNA conjugate, wherein the siRNA conjugate comprises the siRNA duplex according to any one of claims 1 to 6 and a conjugate moiety attached thereto.

8. The conjugate according to claim 7, wherein the conjugate moiety is linked to the 3' end or 5' end of the sense strand of the oligonucleotide; and/or, the conjugate moiety is a GalNAc derivative attached using a bivalent or trivalent branched linker arm.

9. The conjugate according to claim 7, wherein the conjugate moiety is: 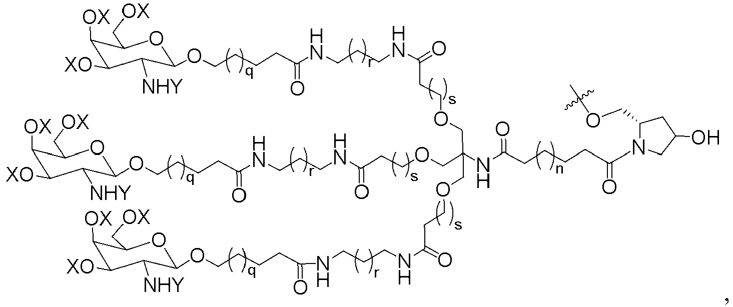
wherein X is a hydroxyl protecting group or H, the hydroxyl protecting group being selected from acetyl, benzoyl, and isobutyryl; Y is an amino protecting group or H, the amino protecting group being selected from formyl, acetyl, propionyl, n-butyryl, and isobutyryl; n is an integer from 0 to 20; q, r, and s are independently integers from 1 to 7; preferably,
the conjugate moiety is:

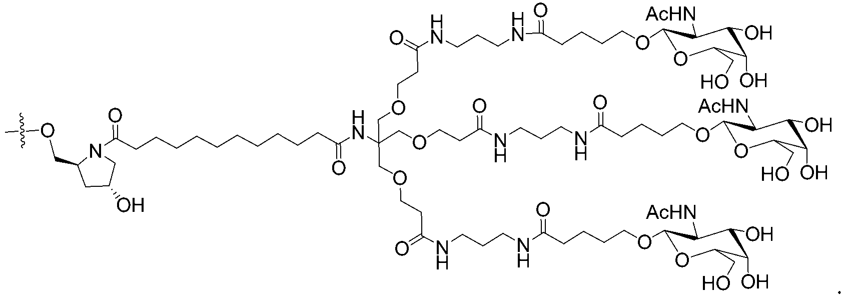

10. The conjugate according to claim 7, wherein the conjugate moiety is: 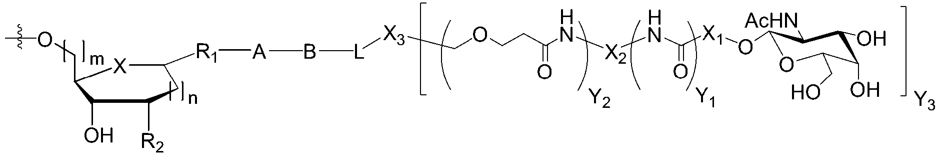
wherein X is oxygen, -N(Y)-, or sulfur;
Y is C₁₋₄ alkyl or C₆₋₁₀ aryl;
R₁ is oxygen or sulfur;
R₂ is hydrogen, -NH₂, C₁₋₄ alkyl, C₆₋₁₀ aryl, C₁₋₄ alkoxy, or halogen;
A is -(CH₂)ₐ-, -(CH₂CH₂O)_{b}-, -((CH₂)_{c}NHCO)_{d}-, or -((CH₂)_{c}CONH)_{d}-, wherein a is an integer from 1 to 15, b is an integer from 1 to 7, c is an integer from 1 to 7, and d is an integer from 1 to 5;
B is -(CH₂)ₑ-, wherein e is an integer from 0 to 7;
L is -CONH- or -NHCO-;
X₁ is -(CH₂)_{f}- or -(CH₂CH₂O)_{f}CH₂-, wherein f is an integer from 1 to 5;
X₂ is -(CH₂)_{g}-, wherein g is an integer from 1 to 6;
Yi is 0 or 1;
Y₂ is 0, 1, or 2;
when Y₃ is 1, X₃ is CH₂; when Y₃ is 2, X₃ is CH; when Y₃ is 3, X₃ is carbon;
m is an integer from 0 to 4;
n is an integer from 0 to 4; preferably,
the conjugate moiety is G4, G5, G6, or G7, the structures of which are as follows:

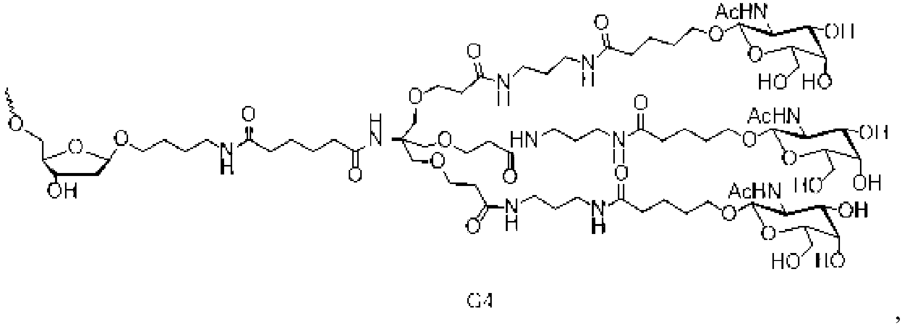

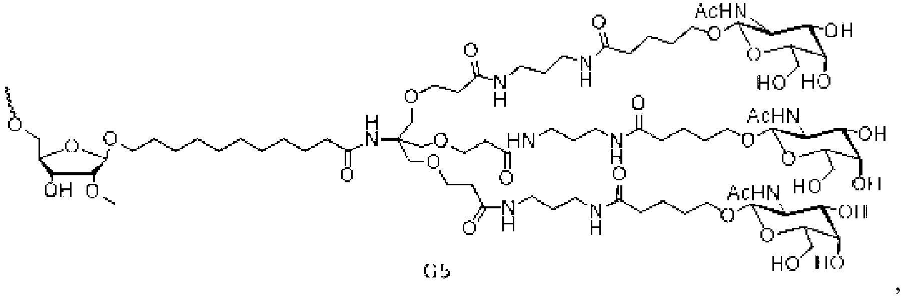

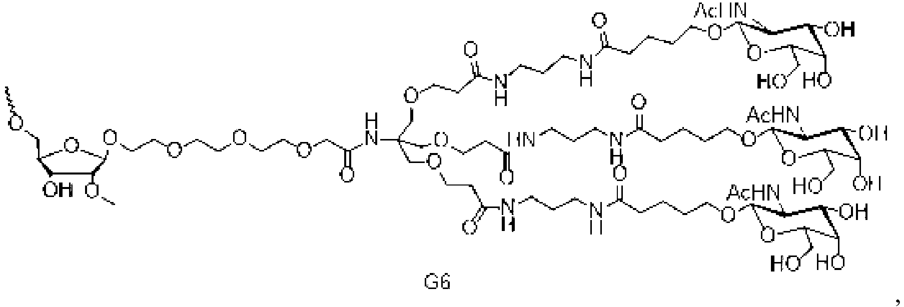

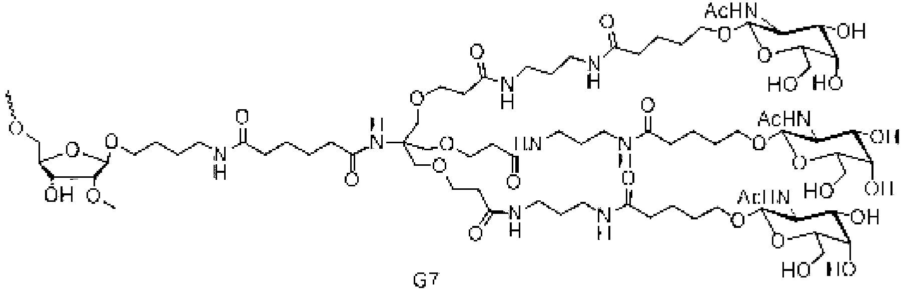

11. The conjugate according to claim 7, wherein the conjugate moiety is as follows: 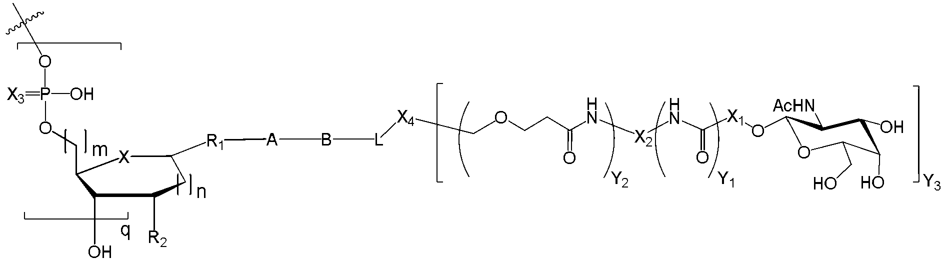
wherein X is oxygen, -N(Y)-, or sulfur;
Y is C₁₋₄ alkyl or C₆₋₁₀ aryl;
R₁ is oxygen or sulfur;
R₂ is hydrogen, -NH₂, C₁₋₄ alkyl, C₆₋₁₀ aryl, C₁₋₄ alkoxy, or halogen;
A is -(CH₂)ₐ-, -(CH₂CH₂O)_{b}-, -((CH₂)_{c}NHCO)_{d}-, or -((CH₂)_{c}CONH)_{d}-, wherein a is an integer from 1 to 15, b is an integer from 1 to 7, c is an integer from 1 to 7, and d is an integer from 1 to 5;
B is -(CH₂)ₑ-, wherein e is an integer from 0 to 7;
L is -CONH- or -NHCO-;
X₁ is -(CH₂)_{f}- or -(CH₂CH₂O)_{f}CH₂-, wherein f is an integer from 1 to 5;
X₂ is -(CH₂)_{g}-, wherein g is an integer from 1 to 6;
X₃ is oxygen or sulfur;
Yi is 0 or 1;
Y₂ is 0, 1, or 2;
when Y₃ is 1, X₄ is CH₂; when Y₃ is 2, X₄ is CH; when Y₃ is 3, X₃ is carbon;
m is an integer from 0 to 4;
n is an integer from 0 to 4;
q is an integer from 0 to 4; preferably,
the conjugate moiety is G101, G102, G103, G105, or G106: 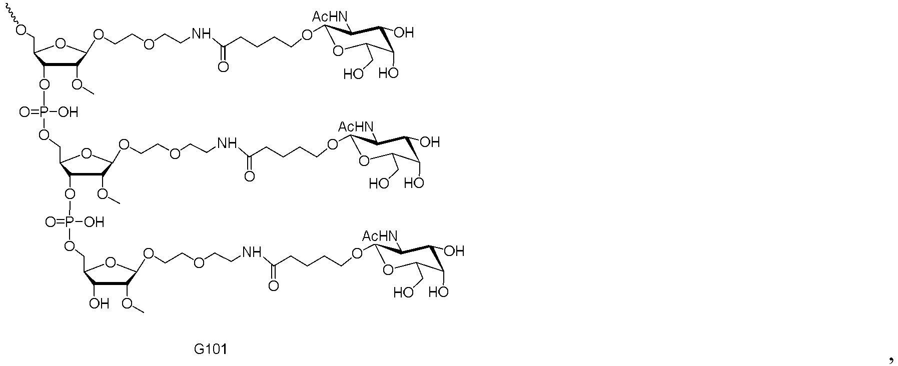 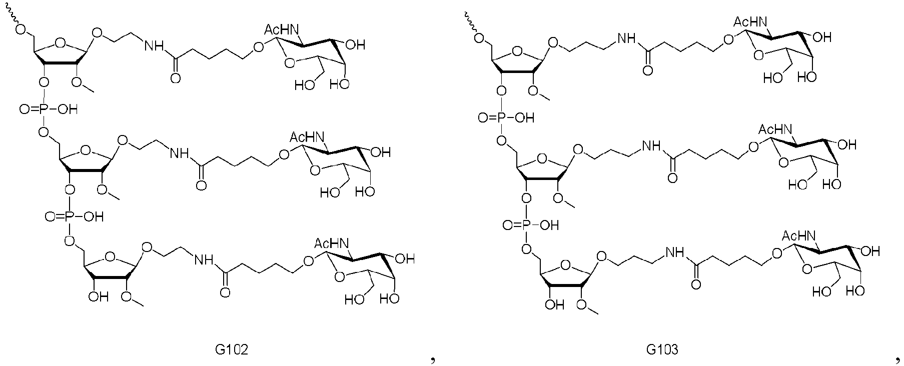 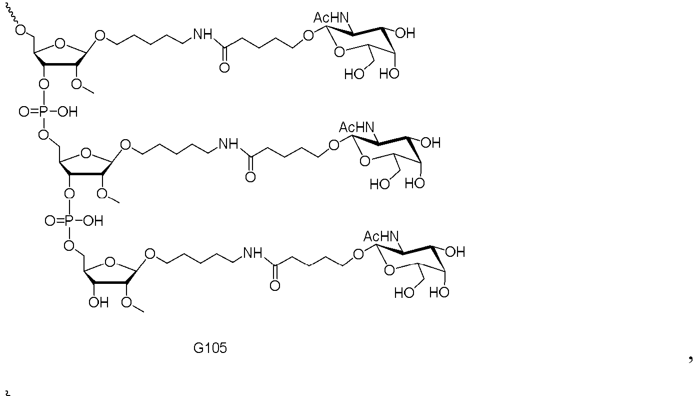 or

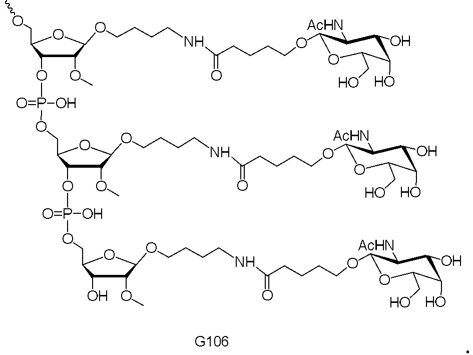

12. The conjugate according to claim 7, wherein the siRNA duplex comprises any one oligonucleotide duplex selected from the following pairings of a sense strand and an antisense strand, or a combination of at least two thereof:
(1) the sense strand is the modified sequence of SEQ ID NO: 21; and the antisense strand is the modified sequence of SEQ ID NO: 214; wherein the antisense strand is modified according to modification pattern B, and the sense strand is modified according to modification pattern a;
(2) the sense strand is the modified sequence of SEQ ID NO: 186; and the antisense strand is the modified sequence of SEQ ID NO: 379; wherein the antisense strand is modified according to modification pattern E, and the sense strand is modified according to modification pattern b;
(3) the sense strand is the modified sequence of SEQ ID NO: 21; and the antisense strand is the modified sequence of SEQ ID NO: 214; wherein the antisense strand is modified according to modification pattern A, and the sense strand is modified according to modification pattern a; nucleotides at positions 5 and 7 of the antisense strand are replaced with DNA, and the nucleotide at the position of the sense strand that is complementary to position 7 of the antisense strand is also replaced with DNA;
(4) the sense strand is the modified sequence of SEQ ID NO: 21; and the antisense strand is the modified sequence of SEQ ID NO: 214; wherein the antisense strand is modified according to modification pattern A, and the sense strand is modified according to modification pattern a;
(5) the sense strand is the modified sequence of SEQ ID NO: 21; and the antisense strand is the modified sequence of SEQ ID NO: 214; wherein the antisense strand is modified according to modification pattern C, and the sense strand is modified according to modification pattern a;
(6) the sense strand is the modified sequence of SEQ ID NO: 21; and the antisense strand is the modified sequence of SEQ ID NO: 214; wherein the antisense strand is modified according to modification pattern C, and the sense strand is modified according to modification pattern b;
(7) the sense strand is the modified sequence of SEQ ID NO: 21; and the antisense strand is the modified sequence of SEQ ID NO: 214; wherein the antisense strand is modified according to modification pattern D, and the sense strand is modified according to modification pattern b;
(8) the sense strand is the modified sequence of SEQ ID NO: 21; and the antisense strand is the modified sequence of SEQ ID NO: 214; wherein the antisense strand is modified according to modification pattern E, and the sense strand is modified according to modification pattern b;
(9) the sense strand is the modified sequence of SEQ ID NO: 21; and the antisense strand is the modified sequence of SEQ ID NO: 214; wherein the antisense strand is modified according to modification pattern B, and the sense strand is modified according to modification pattern a; nucleotides at positions 5 and 7 of the antisense strand are replaced with DNA, and the nucleotide at the position of the sense strand that is complementary to position 7 of the antisense strand is also replaced with DNA;
(10) the sense strand is the modified sequence of SEQ ID NO: 21; and the antisense strand is the modified sequence of SEQ ID NO: 214; wherein the antisense strand is modified according to modification pattern B, and the sense strand is modified according to modification pattern a; the nucleotide at position 7 of the antisense strand is replaced with DNA, and the nucleotide at the position of the sense strand that is complementary to position 7 of the antisense strand is also replaced with DNA;
(11) the sense strand is the modified sequence of SEQ ID NO: 186; and the antisense strand is the modified sequence of SEQ ID NO: 379; wherein the antisense strand is modified according to modification pattern A, and the sense strand is modified according to modification pattern a;
(12) the sense strand is the modified sequence of SEQ ID NO: 186; and the antisense strand is the modified sequence of SEQ ID NO: 379; wherein the antisense strand is modified according to modification pattern B, and the sense strand is modified according to modification pattern a;
(13) the sense strand is the modified sequence of SEQ ID NO: 186; and the antisense strand is the modified sequence of SEQ ID NO: 379; wherein the antisense strand is modified according to modification pattern C, and the sense strand is modified according to modification pattern a;
(14) the sense strand is the modified sequence of SEQ ID NO: 186; and the antisense strand is the modified sequence of SEQ ID NO: 379; wherein the antisense strand is modified according to modification pattern C, and the sense strand is modified according to modification pattern b;
(15) the sense strand is the modified sequence of SEQ ID NO: 186; and the antisense strand is the modified sequence of SEQ ID NO: 379; wherein the antisense strand is modified according to modification pattern D, and the sense strand is modified according to modification pattern b; and,
(16) the sense strand is the modified sequence of SEQ ID NO: 186; and the antisense strand is the modified sequence of SEQ ID NO: 379; wherein the antisense strand is modified according to modification pattern F, and the sense strand is modified according to modification pattern b;
wherein either or both of the sense strand and the antisense strand are linked to the conjugate moiety G4, G5, G6, G7, G101, G102, G103, G105, or G106;
preferably, the conjugate moiety is attached to the 3' end of the sense strand.

13. A nucleic acid-protein complex, wherein the nucleic acid-protein complex comprises a duplex region of the siRNA duplex according to any one of claims 1 to 6, and a nuclease; or, the nucleic acid-protein complex comprises an antisense strand of the duplex region of the siRNA duplex according to any one of claims 1 to 6, and a nuclease;
preferably, the nuclease is an AGO protein.

14. A pharmaceutical composition, wherein the pharmaceutical composition comprises the siRNA duplex according to any one of claims 1 to 6, the conjugate according to any one of claims 7 to 12, or the nucleic acid-protein complex according to claim 13, and a pharmaceutically acceptable carrier.

15. The siRNA duplex according to any one of claims 1 to 6, the conjugate according to any one of claims 7 to 12, the nucleic acid-protein complex according to claim 13, or the pharmaceutical composition according to claim 14, for use in preventing or treating a disease associated with LPA gene expression;
preferably, the disease associated with LPA gene expression is an atherosclerosis or a cardiovascular and cerebrovascular disease caused by overexpression of Apo(a) protein.
